# EUROPEAN PATENT APPLICATION

(11) **EP 2 204 381 A1**
(43) Date of publication of application: **07.07.2010**
(21) Application number: 09180982.2
(22) Date of filing: 03.03.2006
(51) Int. Cl.: C07K 14/47, A61K 38/17, C07K 16/18, G01N 33/68

(54) **Screening method, process for purifying of non-diffusible a-beta oligomers, selective antibodies against said non-diffusible a-beta oligomers and a process for manufacturing of said antibodies**

(30) Priority: 23.03.2005 US 664477 P; 30.11.2005 US 740866 P; 05.03.2005 EP 05004858; 24.03.2005 EP 05006568
(62) Divisional of application: 06707413.8
(71) Applicant: Abbott GmbH & Co. KG, 65205 Wiesbaden (DE)
(72) Inventor: Hillen, Heinz, 67454 Hassloch (DE); Striebinger, Andreas, 67346 Speyer (DE); Keller, Patrick, 64289 Darmstadt (DE); Barghorn, Stefan, 68161 Mannheim (DE); Ebert, Ulrich, 68163 Mannheim (DE)
(74) Representative: Reitstötter - Kinzebach

(57) **Abstract**

Screening method, process for purifying of non-diffusible Aβ oligomers, selective antibodies against said non-diffusible Aβ oligomers and a process for manufacturing of said antibodies

The present invention is relates to non-diffusible globular Aβ(X - 38 .. 43) oligomers ("globulomers") or derivatives thereof, methods for enriching said globulomers or derivatives, compositions comprising said globulomers or derivatives, antibodies and aptamers having specificity for said globulomers or derivatives, methods for preparing such antibodies and aptamers, uses of said globulomers or derivatives, or of said antibodies or aptamers for diagnostic, therapeutic and other purposes, and corresponding methods using said globulomers or derivatives, or said antibodies or aptamers.

## Description

The present invention relates to non-diffusible globular Aβ(X - 38 .. 43) oligomers ("globulomers") or derivatives thereof, methods for enriching said globulomers or derivatives, compositions comprising said globulomers or derivatives, antibodies and aptamers having specificity for said globulomers or derivatives, methods for preparing such antibodies and aptamers, uses of said globulomers or derivatives, or of said antibodies or aptamers for diagnostic, therapeutic and other purposes, and corresponding methods using said globulomers or derivatives, or said antibodies or aptamers.

Alzheimer's disease (AD) is a common neurodegenerative and dementing disorder characterized by a progressive loss of cognitive abilities and by characteristic neuropathological features comprising extracellular amyloid deposits, intracellular neurofibrillary tangles and neuronal loss in several brain regions (Mattson, M.P. Pathways towards and away from Alzheimer's disease. Nature 430, 631 - 639 (2004); Hardy, J. & Selkoe, D.J. The amyloid hypothesis of Alzheimer's disease: progress and problems on the road to therapeutics. Science 297, 353 - 356 (2002)). The principal constituents of the amyloid deposits are amyloid β peptides.

One of the hallmarks of pathology in Alzheimer's disease is excessive formation of amyloid β (Aβ) 1 - 42 that aggregates and is the main constituent of the characteristic plaques. Aβ(1 - 42) is derived from amyloid precursor protein (APP), and abnormal processing of APP, leading to increased production of Aβ(1 - 42), has been a matter of intensive investigation in the past. The amyloid cascade hypothesis by Hardy and Higgins (Hardy, J.A. & Higgins, G.A. Alzheimer's disease: the amyloid cascade hypothesis. Science 256, 184 - 185 (1992)) postulated that increased production of Aβ(1 - 42) leads to its aggregation into fibrils of increasing size (beginning with so-called protofibrils) and ultimately to plaque-like deposits, and that fibrillary Aβ deposits are the reason for the neuropathological symptoms in Alzheimer's disease. This hypothesis was most favoured until recently, although the correlation of dementia and amyloid plaque burden is rather poor in AD patients (Katzman, R. et al. Clinical, pathological, and neurochemical changes in dementia: a subgroup with preserved mental status and numerous neocortical plaques. Ann Neurol 23, 138 - 144 (1988); Naslund, J. et al. Correlation between elevated levels of amyloid beta-peptide in the brain and cognitive decline. JAMA 283, 1571 - 1577 (2000)). It is noteworthy that similar findings have been made in Down's syndrome, suggesting involvement of the same mechanisms.

Oligomers, first described merely as intermediates of the process of fibril and plaque formation, have been recently discussed as important toxic species along AD pathology. The occurrence of soluble, oligomeric Aβ in the brain of AD patients (Kuo, Y.M. et al. Water-soluble Abeta (N - 40, N - 42) oligomers in normal and Alzheimer disease brains. J Biol Chem 271, 4077 - 4081 (1996)) correlates better with AD symptoms than plaque load does (Kuo, Y.M. et al. Water-soluble Abeta (N - 40, N - 42) oligomers in normal and Alzheimer disease brains. J Biol Chem 271, 4077 - 4081 (1996); McLean, C.A. et al. Soluble pool of Abeta amyloid as a determinant of severity of neurodegeneration in Alzheimer's disease. Ann Neurol 46, 860 - 866 (1999)). This has led to a revised amyloid cascade hypothesis (Hardy, J. & Selkoe, D.J. The amyloid hypothesis of Alzheimer's disease: progress and problems on the road to therapeutics. Science 297, 353 - 356 (2002)).

The possibility that soluble assemblies like the Aβ(1 - 42) globulomer, rather than insoluble fibrillary agglomerates, can induce early neuronal alterations in AD has gained much attention since the initial observations of a robust correlation between cortical levels of prefibrillary soluble Aβ and the extent of neuronal loss and severity of cognitive impairment (McLean, C.A. et al. Soluble pool of Abeta amyloid as a determinant of severity of neurodegeneration in Alzheimer's disease. Ann Neurol 46, 860 - 866 (1999); Lue, L.F. et al. Soluble amyloid beta peptide concentration as a predictor of synaptic change in Alzheimer's disease. Am J Pathol. 155, 853 - 862 (1999); Wang, J., Dickson, D.W., Trojanowski, J.Q. & Lee, V.M. The levels of soluble versus insoluble brain Abeta distinguish Alzheimer's disease from normal and pathologic aging. Exp Neurol 158, 328 - 337 (1999)).

Recent work showed the possibility to generate soluble, oligomeric forms of Aβ in vitro (Lambert, M.P. et al. Diffusible, nonfibrillar ligands derived from Abeta 1 - 42 are potent central nervous system neurotoxins. Proc. Natl. Acad. Sci U. S. A 95, 6448 - 6453 (1998); Walsh, D.M. et al. Naturally secreted oligomers of amyloid beta protein potently inhibit hippocampal long-term potentiation in vivo. Nature 416, 535 - 539 (2002)). Some of these soluble, oligomeric forms of Aβ were shown to be detrimental to neurons upon specific binding to synaptic spines (Lacor, P.N. et al. Synaptic targeting by Alzheimer's-related amyloid β oligomers. J Neurosci 24, 10191 - 10200 (2004)). This specific interaction may be responsible for the observed inhibition of hippocampal long-term potentiation (LTP), an experimental paradigm for synaptic plasticity and memory (Lambert, M.P. et al. Diffusible, nonfibrillar ligands derived from Abeta 1 - 42 are potent central nervous system neurotoxins. Proc. Natl. Acad. Sci U. S. A 95, 6448 - 6453 (1998); Walsh, D.M. et al. Naturally secreted oligomers of amyloid beta protein potently inhibit hippocampal long-term potentiation in vivo. Nature 416, 535 - 539 (2002)).

Further evidence for a key role of soluble Aβ forms in AD pathogenesis came from reports that cognitive impairment develops well before deposition of insoluble Aβ in mice transgenic for human APP (Buttini, M. et al. Modulation of Alzheimer-like synaptic and cholinergic deficits in transgenic mice by human apolipoprotein E depends on isoform, aging, and overexpression of amyloid beta peptides but not on plaque formation. J Neurosci 22, 10539 - 10548 (2002); Hsia, A.Y. et al. Plaque-independent disruption of neural circuits in Alzheimer's disease mouse models. Proc. Natl. Acad Sci U. S. A 96, 3228 - 3233 (1999); Mucke, L. et al. High-level neuronal expression of abeta 1 - 42 in wild-type human amyloid protein precursor transgenic mice: synaptotoxicity without plaque formation. J Neurosci 20, 4050 - 4058 (2000)). Since then, many investigators have used synthetic Aβ preparations to model soluble Aβ-mediated neurotoxicity (reviewed in Walsh, D.M. & Selkoe, D.J. Deciphering the molecular basis of memory failure in Alzheimer's disease. Neuron 44, 181 - 193 (2004)).

However, the intrinsic propensity of Aβ peptides (especially Aβ(1 - 42)) to rapidly aggregate in aqueous solutions to a variety of polymerized structures, including soluble oligomers, protofibrils and fibrils (Stine, W.B., Jr., Dahlgren, K.N., Krafft, G.A. & LaDu, M.J. In vitro characterization of conditions for amyloid-beta peptide oligomerization and fibrillogenesis. J Biol Chem 278, 11612 - 11622 (2003)), made it impossible to ascribe the observed neurotoxic effects to one specific form of multimeric Aβ association. Therefore, several groups have recently tried to isolate soluble Aβ(1 - 42) assemblies and test their neurotoxic activity. Lambert and coworkers (Lambert, M.P. et al. Diffusible, nonfibrillar ligands derived from Abeta1 - 42 are potent central nervous system neurotoxins. Proc. Natl. Acad. Sci U. S. A 95, 6448 - 6453 (1998)) described a mixture of small soluble Aβ(1 - 42) aggregates (4-18 kDa on SDS-PAGE), which they called "Aβ-derived diffusible ligands" (ADDLs). They were able to show that concentrations as low as 500 nM of ADDLs caused neuronal death in cell cultures and almost completely blocked LTP (Lambert, M.P. et al. Diffusible, nonfibrillar ligands derived from Abeta 1 - 42 are potent central nervous system neurotoxins. Proc. Natl. Acad. Sci U. S. A 95, 6448 - 6453 (1998); Wang, H.W. et al. Soluble oligomers of beta amyloid 1 - 42 inhibit long-term potentiation but not long-term depression in rat dentate gyrus. Brain Res 924, 133 - 140 (2002)).

In another non-synthetic approach Aβ oligomers were released into the medium by a cell line expressing mutant human APP (Walsh, D.M. et al. Naturally secreted oligomers of amyloid beta protein potently inhibit hippocampal long-term potentiation in vivo. Nature 416, 535 - 539 (2002); Podlisny, M.B. et al. Aggregation of secreted amyloid beta-protein into sodium dodecyl sulfate-stable oligomers in cell culture. J Biol Chem 270, 9564 - 9570 (1995)). Aliquots of this conditioned medium containing uncharacterized Aβ(1 - 42) oligomers blocked LTP in vivo and in vitro at low nanomolar concentrations (Walsh, D.M. et al. Naturally secreted oligomers of amyloid beta protein potently inhibit hippocampal long-term potentiation in vivo. Nature 416, 535 - 539 (2002); Wang, Q., Walsh, D.M., Rowan, M.J., Selkoe, D.J. & Anwyl, R. Block of long-term potentiation by naturally secreted and synthetic amyloid beta-peptide in hippocampal slices is mediated via activation of the kinases c-Jun N-terminal kinase, cyclin-dependent kinase 5, and p38 mitogen-activated protein kinase as well as metabotropic glutamate receptor type 5. J Neurosci 24, 3370 - 3378 (2004)).

WO 98/33815 and WO 01/10900 (G.A. Krafft et al.) describe particular, diffusible amyloid beta1 - 40- or -1 - 42-derived dementing ligands, so-called ADDLs (Amyloid Beta (Aβ)-Derived Diffusible Ligands) as a neurotoxic principle that selectively block long-term potentiation (LTP). According to WO 98/33815 and WO 01/10900 it has been suggested that ADDLs are the neurotoxic species and that the presence of ADDLs in the brain is the causal factor and therefore primary risk factor for occurrence and progression of the main symptoms of Alzheimer's Disease (AD). The relevance of Aβ oligomers for AD pathology has been underlined by their detection in brains of AD patients (Gong, Y. et al. Alzheimer's disease-affected brain: presence of oligomeric Aβ ligands (ADDLs) suggests a molecular basis for reversible memory loss. Proc. Natl. Acad. Sci U. S. A 100, 10417 - 10422 (2003)).

The term "diffusible" as used in Lambert et al. 1998, Walsh et al. 2002, Gong et al. 2003, WO 98/33815 and WO 01/10900 means that a characteristic feature of ADDLs (oligomeric Aβ(1 - 42) or Aβ(1 - 40)) is that they diffuse rapidly when injected into rat hippocampus, in contrast to oligo- or poly-Aβ(1 - 42) or Aβ(1 - 40) fibrils which remain in the injection track (A.M. Manelli et al., Journal of Molecular Neuroscience, 2004, Vol. 23, 235 - 246). Likewise, after intraventricular injection "diffusible" oligomers were observed to spread within the liquor and permeate into sub-surface strata of the CNS.

Despite these reports, the experimental approach to the pathology and function of Aβ oligomers was still difficult because no defined, pure and stable preparation was so far available, which made the identification of the underlying pathological structure impossible.

It was not before WO 2004/067561 (Abbott GmbH & Co. KG) described a new and highly stable Aβ(1 - 42) oligomer species that neuropathological effects could be ascribed to a single distinct oligomeric Aβ species.

Due to the homogeneity and characteristic globular spatial structure of this oligomeric Aβ species it was named "Aβ(1 - 42) globulomer". Aβ(1 - 42) globulomers can be easily and reproducibly generated from synthetic Aβ peptide in vitro. Physicochemical characterization reveals a highly water-soluble globular Aβ(1 - 42) oligomer of approximately 60 kDa size ("Aβ(1 - 42) globulomer") which is a potent antigen in mice and rabbits eliciting generation of Aβ(1 - 42) globulomer-specific antibodies that do not cross-react with amyloid precursor protein.

Surprisingly and unexpectedly, said soluble globular Aβ(1 - 42) oligomer ("globulomers") described in WO 2004/067561 (Abbott GmbH & Co. KG) binds specifically to dendritic processes of neurons but not to glia in hippocampal cell cultures. Binding to tissue is very strong and results in immediate removal after i.c.v. injection in rats. Consequently, Aβ(1 - 42) globulomers were not detected in the cerebrospinal fluid of AD patients within the current detection limits. This means, surprisingly and unexpectedly, that the dementing Aβ(1 - 42) globulomers according to WO 2004/067561 (Abbott GmbH & Co. KG) have non-diffusible properties and that the toxic principle responsible for occurrence and progression of Alzheimer's disease is characterized by the feature "non-diffusible" instead of "diffusible" as postulated by WO 98/33815 and similar publications as discussed above. The memory impairing potency of the non-diffusible Aβ(1 - 42) globulomer is shown by a complete block of long term potentiation (LTP) in rat hippocampal slices. Selective neutralization of Aβ(1 - 42) globulomer is therefore expected to have a high potential for treatment of AD.

The inventors now postulate that this particular, non-diffusible globular oligomer is generated along a new aggregation pathway independent of Aβ fibril formation and represents a novel pathological entity in Alzheimer's disease. Since it is present in the brain of AD patients and APP transgenic mice and also binds specifically to neurons and blocks LTP, the inventors believe that this globular Aβ oligomer characterized by a new structural epitope will provide an unprecedented possibility to elucidate the neuropathology associated with Aβ oligomers and to clinically address deficits of AD patients by specific treatment.

Further, the inventors now were able to show that by using anti Aβ globulomer antibodies like polyclonal antibody 5598 or monoclonal antibody 8F5 in vivo cross-reacting species were not restricted to the Aβ(1-42) sequence but also is extendable to other Aβ(x-y) species, e.g Aβ(1-40) and Aβ (1-38).

The invention thus relates to a non-diffusible globular Aβ(X - 38 .. 43) oligomer ("globulomer") or a derivative thereof, wherein X is selected from the group consisting of the numbers 1 .. 24.

As used herein, the ellipsis A .. B denotes the set comprising all natural numbers from A to B, including both, e.g. "17 .. 20" thus denotes the group of the numbers 17, 18, 19 and 20. The hyphen denotes a contiguous sequence of amino acids, i.e., "X - Y" comprises the sequence from amino acid X to amino acid Y, including both. Thus, "A .. B - C .. D" comprises all possible combinations between members of these two sets, e.g. "17 .. 20 - 40 .. 42" comprises all of the following: 17 - 40, 17 - 41, 17 - 42, 18 - 40, 18 - 41, 18 - 42, 19 - 40, 19 - 41, 19 - 42, 20 - 40, 20 - 41 and 20 - 42. Unless stated otherwise, all numbers refer to the beginning of the mature peptide, 1 indicating the N-terminal amino acid. The term "Aβ(X - A .. B)" is a synonym for and can be interchangeably used with the term "Aβ(X - A/B)".

According to a particular embodiment, the non-diffusible globular oligomer or derivative thereof is selected from the group consisting of non-diffusible globular Aβ(X - 40) oligomers and non-diffusible globular Aβ(X - 42) oligomers or derivatives thereof, wherein X is selected from the group consisting of the numbers 8 .. 24, preferably from the group consisting of the numbers 12 .. 24, more preferably from the group consisting of the numbers 12 .. 20 and most preferably from the group consisting of the numbers 17 .. 20.

According to a further particular embodiment, the non-diffusible globular oligomer or derivative thereof is selected from the group consisting of non-diffusible globular Aβ(X - 38) oligomers, non-diffusible globular Aβ(X - 39) oligomers, and non-diffusible globular Aβ(X - 41) oligomers, or derivatives thereof.

According to still a further particular embodiment, the non-diffusible globular oligomer or derivative thereof is selected from the group consisting of non-diffusible globular Aβ(X - 43) oligomers, or derivatives thereof.

Derivatives of the non-diffusible globular Aβ(X - 38 .. 43) oligomer in particular include cross-linked, preferably chemically cross-linked, more preferably aldehyde cross-linked, most preferably glutardialdehyde cross-linked non-diffusible globular Aβ(X - 38 .. 43) oligomer.

Further derivatives of the non-diffusible globular Aβ(X - 38 .. 43) oligomer include non-diffusible globular Aβ(X - 38 .. 43) oligomers which are labelled by being covalently linked to a group that facilitates detection, preferably a fluorophore, e. g. fluorescein isothiocyanate, phycoerythrin, *Aequorea victoria* fluorescent protein, *Dictyosoma* fluorescent protein or any combination or fluorescence-active derivative thereof; a chromophore; a chemoluminophore, e. g. luciferase, preferably *Photinus pyralis* luciferase, *Vibrio fischeri* luciferase, or any combination or chemoluminescence-active derivative thereof; an enzymatically active group, e. g. peroxidase, e. g. horseradish peroxidase, or any enzymatically active derivative thereof; an electron-dense group, e. g. a heavy metal containing group, e.g. a gold containing group; a hapten, e. g. a phenol derived hapten; a strongly antigenic structure, e. g. peptide sequence predicted to be antigenic, e. g. predicted to be antigenic by the algorithm of Kolaskar and Tongaonkar; an aptamer for another molecule; a chelating group, e. g. hexahistidinyl; a natural or nature-derived protein structure mediating further specific protein-protein interactions, e. g. a member of the fos/jun pair; a magnetic group, e. g. a ferromagnetic group; or a radioactive group, e. g. a group comprising ¹H, ¹⁴C, ³²P, ³⁵S or ¹²⁵I or any combination thereof. Such labelling groups and methods for attaching them to proteins are known in the art.

In another particular embodiment of the invention, the derivative of the non-diffusible globular Aβ(X - 38 .. 43) oligomer is a non-diffusible globular Aβ(X - 38 .. 43) oligomer which comprises at least one monomeric subunit that is flagged by being covalently or by non-covalent high-affinity interaction, preferably covalently linked to a group that facilitates inactivation, sequestration, degradation and/or precipitation, preferably flagged with a group that promotes in vivo degradation, more preferably with ubiquitin. It is particularly preferred if this flagged oligomer is assembled in vivo.

Further derivatives of the non-diffusible globular Aβ(X - 38 .. 43) oligomer include non-diffusible globular Aβ(X - 38 .. 43) oligomers which are cross-linked and labelled or cross-linked and flagged.

In a preferred embodiment of the invention, the oligomer or derivative thereof is soluble.

A method for preparing several Aβ(1 - 42) and Aβ(X - 42) oligomers and derivatives thereof is described in WO 2004/067561. The non-diffusible globular Aβ(X - 38 .. 43) oligomers or derivatives thereof of the invention can be prepared in an analogous manner.

Thus, the invention also relates to a method for preparing non-diffusible globular Aβ(X - 38 .. 43) oligomer or a - in particular cross-linked, labelled and/or flagged - derivative thereof, which method comprises contacting a preparation of a suitable Aβ protein or derivative thereof with a detergent, followed by reduction of the detergent concentration and obtaining the non-diffusible globular Aβ(X - 38 .. 43) oligomer. A detailed description of said method is disclosed in WO 2004/067561, the content of which is incorporated herein by reference.

Derivatization and/or truncation can be performed before or after oligomerization. Accordingly, Aβ protein or derivative thereof suitable for being used in said method includes optionally derivatized Aβ(X - 38 .. 43) monomer, X preferably being selected from the group consisting of the numbers 1 .. 24, more preferably from the group consisting of the numbers 1 .. 22 and most preferably from the group consisting of the numbers 1 .. 20.

Alternatively, and especially usable for in vitro diagnostic methods, the "non-diffusible" globular Aβ(1 - 42) oligomers ("globulomers") of the invention can also be extracted from human or animal nerve cells, including but not limited to APP- or Amyloid β-expressing recombinant or non recombinant derived cell lines and/or liquids, in particular brain or spinal cord tissue and/or liquids, and then be directly detected in the so obtained extract or a processed extract by use of antibodies having specificity against the "non-diffusible" globular Aβ(1 - 38 .. 43) oligomer ("globulomers") or derivatives thereof, like the respective truncated Aβ(X - 38 .. 43) oligomer and/or derivatives thereof.

In a particularly preferred embodiment of the invention, the resulting non-diffusible globular Aβ(X - 38 .. 43) oligomer or derivatives thereof are then subjected to one or more additional purification steps, e. g. precipitation and re-dissolution, by techniques known in the art.

The invention thus also refers to a method for enriching non-diffusible globular Aβ(X - 38 .. 43) oligomer or a derivative thereof in a preparation comprising the oligomer or derivative, which method comprises capturing the oligomer or derivative and obtaining the oligomer or derivative in enriched form ("active" or "positive" enrichment).

In a preferred embodiment of the invention, the method for enriching the oligomer or derivative comprises contacting the preparation with an agent that binds to the oligomer or derivative.

In a particularly preferred embodiment of the invention, said agent is immobilized.

In a particularly preferred embodiment of the invention, said agent is an antibody having specificity for the oligomer or derivative.

In a preferred embodiment of the invention, obtaining the oligomer or derivative in enriched form comprises desorbing the captured oligomer or derivative, preferably in such a way that desorbing the captured oligomer or derivative comprises contacting the captured oligomer or derivative with a high salt buffer or an acidic solution.

The invention also refers to a method for enriching non-diffusible globular Aβ(X - 38 .. 43) oligomer or a derivative thereof in a preparation comprising the oligomer or derivative, which method comprises removing at least one substance other than the oligomer or derivative from the preparation ("passive" or "negative" enrichment). It is preferred if the substance other than the oligomer or derivative is Aβ monomer or Aβ fibrillomer, preferably both, and also if removing the substance comprises contacting the preparation with an agent that specifically binds to the substance.

In a particularly preferred embodiment of the invention, said agent is immobilized.

In a particularly preferred embodiment of the invention, said agent is an antibody that specifically binds to the substance, preferably with high affinity. Antibodies which can be used to remove non-globulomer Aβ species include anti Aβ(33-42) antibodies which are specific for C-terminal Aβ(1-42) species, such as anti Aβ(33-42) polyclonal antibody from Signet Cat.No. 9135, and monoclonal equivalents thereof, e.g. mAb 12F4 from Signet, Cat.No 9142 or anti-Aβ(1-42) mAb, C-terminal clone BDI780; purified, lyophilized; Biodesign Cat.No. Q67780M.

In a more preferred embodiment of the invention, removing the substance comprises contacting the preparation with an antibody that binds to the substance and then subjecting the substance bound to the antibody to affinity chromatography for removal of antibody-antigen complexes, preferably to protein A affinity chromatography.

The present invention thus also relates to a purified non-diffusible globular Aβ(X - 38 .. 43) oligomer or a derivative thereof. According to one embodiment of the present invention, a purified non-diffusible globular Aβ(X - 38 .. 43) oligomer or derivative thereof is one which is obtainable by a method for enriching non-diffusible globular Aβ(X - 38 .. 43) oligomer or derivative thereof, as defined above. In another aspect, a purified non-diffusible globular Aβ(X - 38 .. 43) oligomer or derivative thereof is one which has a purity of more than 99 % by weight, preferably of more than 99.9 % by weight, preferably of more than 99.99 % by weight.

The invention also refers to a composition comprising non-diffusible globular Aβ(X - 38 .. 43) oligomer or derivative thereof, obtainable by a method of the invention, preferably a composition comprising non-diffusible globular Aβ(X - 38 .. 43) oligomer or a derivative thereof, wherein the amount of the oligomer or the derivative is at least 99 % by weight of the composition, preferably at least 99.9 % by weight of the composition and more preferably at least 99.99 % by weight of the composition.

The non-diffusible globular Aβ(X - 38 .. 43) oligomer or derivative thereof of the invention, especially in purified form, has many utilities, some of which are described in the following.

In one aspect, the invention relates to the use of non-diffusible globular Aβ(X - 38 .. 43) oligomer or a derivative thereof for preparing an antibody having specificity for the oligomer or derivative. Accordingly, the invention also relates to a method for preparing an antibody having specificity for non-diffusible globular Aβ(X - 38 .. 43) oligomer or a derivative thereof as defined above, which method comprises
- providing an antigen comprising the oligomer or derivative;
- exposing an antibody repertoire or potential antibody repertoire to said antigen; and
- selecting from said repertoire an antibody which specifically binds to said oligomer or derivative thereof.

Here it is to be understood that a "potential antibody repertoire" refers to any library, collection, assembly or set of amino acid or corresponding nucleic acid sequences or to any generator of such a library, collection, assembly or set of amino acid sequences that can be used for producing an antibody repertoire in vivo or in vitro. In a preferred embodiment of the invention, the generator is the adaptive immune system of an animal, in particular the antigen-producing part of the immune system of a mammal which generates antibody diversity by a recombination process well known to those skilled in the art. In another preferred embodiment of the invention, the generator is a system for the spawning of random nucleic acid sequences which can then, by insertion into a suitable antibody framework, be used to produce an antibody repertoire in vitro.

In a preferred embodiment of the invention, the antibody repertoire or potential antibody repertoire is exposed to the antigen *in vivo* by immunizing an organism with said antigen. In another preferred embodiment of the invention, the potential antibody repertoire is a library of suitable nucleic acids which is exposed to the antibody by in vitro affinity screening as described in the art, e.g. a phage display and panning system.

In another aspect, the invention also relates to an antibody having specificity for non-diffusible globular Aβ(X - 38 .. 43) oligomer or a derivative thereof as defined above.

In a preferred embodiment of the invention, the antibody is obtainable by a method comprising selecting the antibody from a repertoire or potential repertoire as described above.

In a preferred embodiment of the invention, the affinity of the antibody to the oligomer or derivative is at least 2 times, e. g. at least 3 times or at least 5 times, preferably at least 10 times, e. g. at least 20 times, at least 30 times or at least 50 times, more preferably at least 100 times, e. g. at least 200 times, at least 300 times or at least 500 times, and even more preferably at least 1000 times, e. g. at least 2000 times, at least 3000 times or at least 5000 times, even more preferably at least 10000 times, e. g. at least 20000 times, at least 30000 or at least 50000 times, and most preferably at least 100000 times greater than the binding affinity of the antibody to a monomeric Aβ(1 - 42).

In a preferred embodiment of the invention, the affinity of the antibody to the oligomer or derivative is at least 2 times, e. g. at least 3 times or at least 5 times, preferably at least 10 times, e. g. at least 20 times, at least 30 times or at least 50 times, more preferably at least 100 times, e. g. at least 200 times, at least 300 times or at least 500 times, and even more preferably at least 1000 times, e. g. at least 2000 times, at least 3000 times or at least 5000 times, even more preferably at least 10000 times, e. g. at least 20000 times, at least 30000 or at least 50000 times, and most preferably at least 100000 times greater than the binding affinity of the antibody to a monomeric Aβ(1 - 40).

Expediently, the antibody of the present invention binds to one or, more preferably, both monomers with low affinity, most preferably with a K_{D} of 1x10⁻⁸ M or smaller affinity, e. g. with a K_{D} of 3x10⁻⁸ M or smaller affinity, with a K_{D} of 1x10⁻⁷ M or smaller affinity, e. g. with a K_{D} of 3x10⁻⁷ M or smaller affinity, or with a K_{D} of 1x10⁻⁶ M or smaller affinity, e. g. with a K_{D} of 3x10⁻⁵ M or smaller affinity, or with a K_{D} of 1x10⁻⁵ M or smaller affinity.

In a preferred embodiment of the invention, the affinity of the antibody to the oligomer or derivative is at least 2 times, e. g. at least 3 times or at least 5 times, preferably at least 10 times, e. g. at least 20 times, at least 30 times or at least 50 times, more preferably at least 100 times, e. g. at least 200 times, at least 300 times or at least 500 times, and even more preferably at least 1000 times, e. g. at least 2000 times, at least 3000 times or at least 5000 times, even more preferably at least 10000 times, e. g. at least 20000 times, at least 30000 or at least 50000 times, and most preferably at least 100000 times greater than the binding affinity of the antibody to a fibrillomeric Aβ(1 - 42).

In a preferred embodiment of the invention, the affinity of the antibody to the oligomer or derivative is at least 2 times, e. g. at least 3 times or at least 5 times, preferably at least 10 times, e. g. at least 20 times, at least 30 times or at least 50 times, more preferably at least 100 times, e. g. at least 200 times, at least 300 times or at least 500 times, and even more preferably at least 1000 times, e. g. at least 2000 times, at least 3000 times or at least 5000 times, even more preferably at least 10000 times, e. g. at least 20000 times, at least 30000 or at least 50000 times, and most preferably at least 100000 times greater than the binding affinity of the antibody to a fibrillomeric Aβ(1 - 40).

Expediently, the antibody of the present invention binds to one or, more preferably, both fibrils with low affinity, most preferably with a K_{D} of 1x10⁻⁸ M or smaller affinity, e. g. with a K_{D} of 3x10⁻⁸ M or smaller affinity, with a K_{D} of 1x10⁻⁷ M or smaller affinity, e. g. with a K_{D} of 3x10⁻⁷ M or smaller affinity, or with a K_{D} of 1x10⁻⁶ M or smaller affinity, e. g. with a K_{D} of 3x10⁻⁵ M or smaller affinity, or with a K_{D} of 1x10⁻⁵ M or smaller affinity.

According to a particularly preferred embodiment, the present invention relates globulomer-specific antibodies. These include in particular antibodies having a comparatively smaller affinity for both the monomeric and fibrillomeric forms of Aβ than for the Aβ globulomer.

The term "greater affinity" here refers to a degree of interaction where the equilibrium between unbound antibody and unbound globulomer on the one hand and antibody-globulomer complex on the other is further in favour of the antibody-globulomer complex. Likewise, the term "smaller affinity" here refers to a degree of interaction where the equilibrium between unbound antibody and unbound globulomer on the one hand and antibody-globulomer complex on the other is further in favour of the unbound antibody and unbound globulomer.

The invention also relates to the use of non-diffusible globular Aβ(X - 38 .. 43) oligomer or a derivative thereof as defined above for preparing an aptamer having specificity for the oligomer or derivative.

The invention also relates to a method for preparing an aptamer having specificity for non-diffusible globular Aβ(X - 38 .. 43) oligomer or a derivative thereof as defined in claim, which method comprises at least the steps of
- providing a binding target comprising the oligomer or derivative;
- exposing an aptamer repertoire or potential aptamer repertoire to said binding target; and
- selecting from said repertoire an aptamer which specifically binds to said oligomer or derivative thereof.

An "aptamer" herein refers to any small molecule that is capable of specific, non-covalent binding to its target, preferably to a peptide, DNA or RNA sequence, more preferably to a peptide, DNA or RNA sequence of about 3 to 100 monomers, in particular of about 5 to 30 monomers, most preferably to a peptide of about 5 to 30 amino acids, which may at one end or both ends be attached to a larger molecule, preferably a larger molecule mediating biochemical functions, more preferably a larger molecule inducing inactivation and/or degradation, most preferably ubiquitin, or preferably a larger molecule facilitating destruction, more preferably an enzyme or a fluorescent protein.

Here it is to be understood that a "potential aptamer repertoire" refers to any library, collection, assembly or set of amino acid sequences or corresponding nucleic acid sequences or to any generator of such a library, collection, assembly or set of amino acid sequences that can be used for producing an aptamer repertoire in vivo or in vitro.

The invention also relates to an aptamer having specificity for non-diffusible globular Aβ(X - 38 .. 43) oligomer or a derivative thereof as defined above, which aptamer is obtainable by a method as described.

In a preferred embodiment of the invention, the aptamer is labelled by being covalently linked to a group that facilitates detection, preferably a fluorophore, e. g. fluorescein isothiocyanate, phycoerythrin, *Aequorea victoria* fluorescent protein, *Dictyosoma* fluorescent protein or any combination or fluorescence-active derivative thereof; chromophore; chemoluminophore, e. g. luciferase, preferably *Photinus pyralis* luciferase, *Vibrio fischeri* luciferase, or any combination or chemoluminescence-active derivative thereof; enzymatically active group, e. g. peroxidase or any enzymatically active derivative thereof; heavy metal, e.g. gold; hapten, e. g. a phenol derived hapten; antigenic structure, e. g. peptide sequence predicted to be antigenic; other aptamer; natural or nature-derived protein structure mediating further specific protein-protein interactions, e. g. a member of the fos/jun pair; or radioactive group, e. g. a group comprising ¹H, ¹⁴C ³²P, ³⁵S or ¹²⁵I or any combination thereof.

In another preferred embodiment of the invention, the aptamer is flagged by being covalently or by a non-covalent high-affinity bond, preferably covalently linked to a group that facilitates inactivation, sequestration, degradation and/or precipitation when the aptamer is bound to its target, preferably flagged with a group that promotes in vivo degradation, more preferably with ubiquitin.

The invention also relates to the use of non-diffusible globular Aβ(X - 38 .. 43) oligomer or a derivative thereof as defined in claim for preparing a pharmaceutical composition for treating or preventing Alzheimer's disease.

In a preferred embodiment of the invention, the pharmaceutical composition is a vaccine for active immunization.

Accordingly, the invention also relates to a method of treating or preventing Alzheimer's disease in a subject in need thereof, which comprises administering non-diffusible globular Aβ(X - 38 .. 43) oligomer or a derivative thereof as defined to the subject.

In a preferred embodiment of the invention, administering non-diffusible globular Aβ(X - 38 .. 43) oligomer or a derivative thereof is for actively immunizing the subject against Alzheimer's disease.

It particularly preferred if the non-diffusible globular Aβ(X - 38 .. 43) oligomer or its derivative of the composition is not able to enter the patient's CNS in significant amounts.

It is also particularly preferred if the pharmaceutical composition comprising the non-diffusible globular Aβ(X - 38 .. 43) oligomer or its derivative is capable of inducing a strong immune response against Aβ globulomers, preferably a strong immune response directed against Aβ globulomers only, more preferably a strong non-inflammatory antibody-based immune response against Aβ globulomers only. Thus, in a most preferred embodiment of the invention the pharmaceutical composition comprises an immunological adjuvant, preferably an adjuvant and a signalling molecule, e. g. a cytokine, that directs the immune response towards the non-inflammatory, antibody-based type. Such adjuvants and signalling molecules are well known to those skilled in the art.

It is particularly preferred if the pharmaceutical composition for active immunization is administered via a route selected from the group consisting of the intravenous route, the intramuscular route and the subcutaneous route. It is also particularly preferred if the composition is administered by a method selected from injection, bolus infusion and continuous infusion, each of which may be performed once, repeatedly or in regular intervals.

In a particular embodiment of the invention, long-term continuous infusion is achieved by employing an implantable device. In a further particular embodiment of the invention, the composition is applied as an implantable sustained release or controlled release depot formulation. Suitable formulations and devices are known to those skilled in the art. The details of the method to be used for any given route will depend on the stage and severity of the disease and the overall medical parameters of the subject and are preferably decided upon individually at the treating physician's or veterinary's discretion.

In an especially preferred embodiment of the invention, the pharmaceutical composition for active immunization comprises one ore more substances selected from the group consisting of pharmaceutically acceptable preservatives, pharmaceutically acceptable colorants, pharmaceutically acceptable protective colloids, pharmaceutically acceptable pH regulators and pharmaceutically acceptable osmotic pressure regulators. Such substances are described in the art.

The antibodies and aptamers of the invention also have many potential applications, some of which are described in the following. They are especially useful for screening purposes, i.e. detecting and, if desired, also for determining the amount of non-diffusible globular Aβ(X - 38 .. 43) oligomer or a derivative thereof in a preparation suspected or known to comprise such oligomers or derivatives.

Thus, the antibodies of the invention are capable of detecting, both in vitro and in vivo, non-diffusible globular Aβ(X - 38 .. 43) oligomer or a derivative thereof to which they bind. Said antibodies may therefore be used for detecting said oligomer or derivative, for example in a preparation, for instance a sample that is derived from a subject suspect of having an amyloidosis, or in a subject suspect of having an amyloidosis, for instance a human individual or other mammal.

Accordingly, the invention also relates to a method of detecting non-diffusible globular Aβ(X - 38 .. 43) oligomer or a derivative thereof in a preparation, which method comprises allowing an antibody of the invention to act on an oligomer or derivative so as to bind to said oligomer or derivative thereof (and thereby preferably forming a complex comprising the antibody and the oligomer or derivative). Said oligomer may be detected in vitro, for example. For example, the antibody of the invention may be added to a preparation, for instance a sample derived from a subject or a cell culture which contains or is suspected to contain said oligomer or derivative, in order to detect said oligomer or derivative in said preparation. Alternatively, the oligomer or derivative may be detected in an individual in vivo.

The invention also refers to the use of an antibody having specificity for non-diffusible globular Aβ(X - 38 .. 43) oligomer or a derivative thereof as defined above for preparing a pharmaceutical composition for treating or preventing an amyloidosis.

In a preferred embodiment of the invention, the pharmaceutical composition is for passive immunization.

Accordingly, the invention also relates to a method of treating or preventing an amyloidosis in a subject in need thereof, which comprises administering an antibody having specificity for non-diffusible globular Aβ(X - 38 .. 43) oligomer or a derivative thereof as defined in claim to the subject.

In a preferred embodiment of the invention, administering the antibody is for passive immunization.

The term "amyloidosis" here denotes a number of disorders characterized by abnormal folding, clumping, aggregation and/or accumulation of particular proteins (amyloids, fibrous proteins and their precursors) in various tissues of the body. In Alzheimer's disease and Down's syndrome, nerve tissue is affected, and in cerebral amyloid angiopathy (CAA) blood vessels are affected. According to a particular embodiment of the present invention, an amyloidosis is selected from the group consisting of Alzheimer's disease (AD) and the amyloidosis of Down's syndrome.

One result of using the antibodies of the present invention was that diagnosing Alzheimer's disease or having an increased risk of getting Alzheimer's disease cannot easily within detection limits rely on the determination of the non-diffusible soluble globular Aβ(1 - 42) and/or Aβ(1 - 40) oligomer ("globulomers") in a subject. However, surprisingly and unexpectedly, it was found that said diagnosis can be related to the determination of the presence of auto-antibodies which specifically bind to the non-diffusible soluble globular Aβ(1 - 42) oligomers ("globulomers") of the invention.

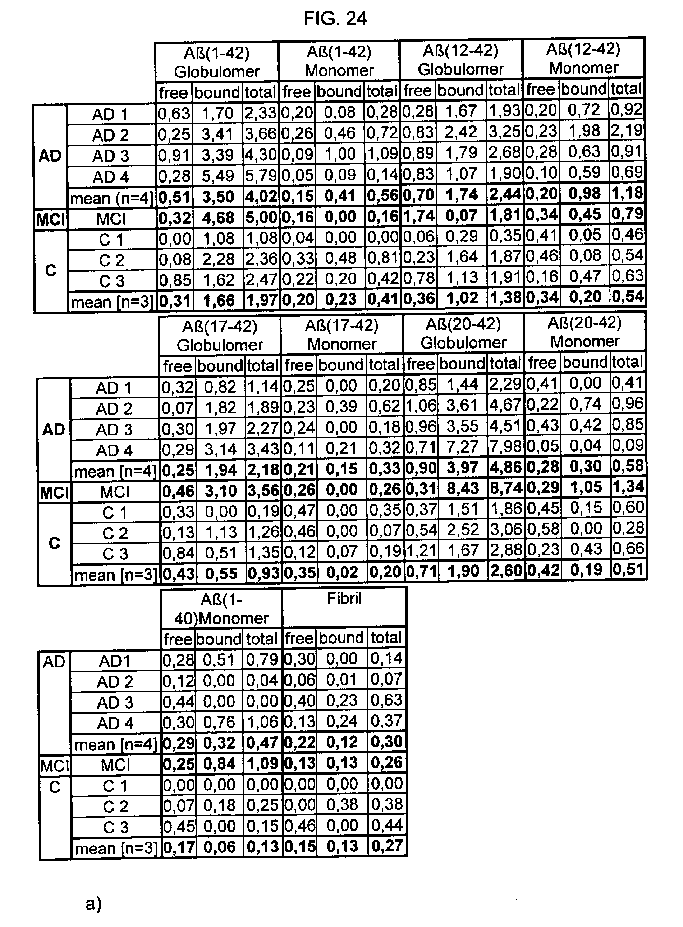

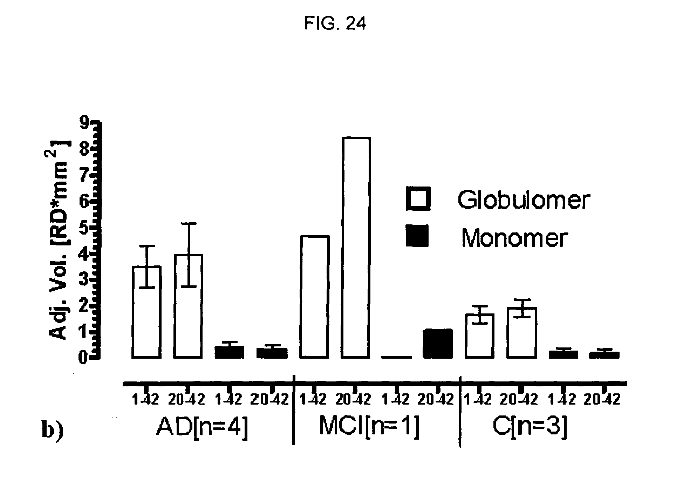

Surprisingly but in agreement with the globulomer hypothesis it was found that the globulomer epitope is an endogenous antigen which gives rise to an endogenous immune response. This endogenous immune response is characterized by at least three independent features:
1. The level of anti-Aβ globulomer auto-antibodies increases with Alzheimer's disease. In particular, it is surprising that these auto-antibodies are elevated in sera of AD patients. So far several investigators have reported autoantibodies in serum of AD patients. According to three papers (S. Brettschneider et al., " Decreased Serum Amyloid 1-42 Autoantibody Levels in Alzheimer's Disease, Determined by a Newly Developed Immuno-Precipitation Assay with Radiolabeled Amyloid 1-42 Peptide, Biol Psychiatry 2005, 57, 813-816, M.E. Weksler, et al., "Patients with Alzheimer disease have lower levels of serum antiamyloid peptide antibodies than healthy elderly individuals" Exp. Gerontology 37, (2002), 943-948 and Y. Du et al., " Reduced levels of amyloid β-peptide antibody in Alzheimer disease", Neurology 57, 801-805 (2001)) levels of anti Aβ antibodies in general were reported to decrease slightly but not significantly in Alzheimer's disease patients.
2. Anti-Aβ globulomer auto-antibodies are predominantly bound in antigen-complexes. The auto-antibodies described here are surprisingly also detected to a substantial amount as part of antigen-antibody-complexes as indicated by pre-treatment of sera with acidic buffers as described in Example 7. This is strong evidence that the immune system is actively trying to eliminate the misfolded globulomer epitope by complexing to endogenous antibodies.
3. Anti-Aβ globulomer auto-antibodies levels are significantly higher than levels of anti-Aβ monomer auto-antibodies. As described here in example 7 and Fig 24, they are about 5 - 10 fold higher compared to the monomer Aβ peptides . Besides the high fraction of antigen-antibody-complexes this is another parameter indicating that globulomers are being actively eliminated by the endogenous immune system.

The invention thus also relates to the use of non-diffusible globular Aβ(X - 38 .. 43) oligomer or a derivative thereof as defined above for preparing a composition for detecting auto-antibodies having specificity for the oligomer or derivative in a subject.

In a preferred embodiment of the invention, the subject is suspected of having any form of amyloidosis, e.g. Alzheimer's disease, and detecting auto-antibodies is for diagnosing the presence or absence of any form of Amyloidosis, e.g. Alzheimer's disease in the subject. In a preferred embodiment of the invention, the antibodies are detected using a labelled derivative of the non-diffusible globular oligomer or cross-linked derivative as described above.

The invention also refers to the use of non-diffusible globular Aβ(X - 38 .. 43) oligomer or a derivative thereof as defined above for detecting auto-antibodies having specificity for the oligomer or derivative in a sample.

In a preferred embodiment of the invention, the sample is derived from a subject suspected of having an amyloidosis, e.g. Alzheimer's disease, and detecting the auto-antibodies is for diagnosing the presence or absence of the amyloidosis, e.g. Alzheimer's disease in the subject. In a preferred embodiment of the invention, the antibodies are detected using a labelled derivative of the non-diffusible globular oligomer or cross-linked derivative as described above.

The invention also refers to a method of detecting auto-antibodies having specificity for non-diffusible globular Aβ(X - 38 .. 43) oligomer or a derivative thereof in a subject, which method comprises administering to the subject non-diffusible globular Aβ(X - 38 .. 43) oligomer or a derivative thereof as defined above and detecting a complex formed by the antibody and the oligomer or derivative, the presence of the complex indicating the presence of the auto-antibodies. In a preferred embodiment of the invention, the method comprises the use of a labelled derivative of the non-diffusible globular oligomer or its cross-linked derivative as described above.

In a preferred embodiment of the invention, the subject is suspected of having an amyloidosis and detecting the auto-antibodies is for diagnosing the presence or absence of the amyloidosis in the subject. In a preferred embodiment of the invention, the antibodies are detected using a labelled derivative of the non-diffusible globular oligomer or cross-linked derivative as described above.

The invention also refers to a method of detecting auto-antibodies having specificity for non-diffusible globular Aβ(X - 38 .. 43) oligomer or a derivative thereof in a sample, which method comprises contacting the sample with non-diffusible globular Aβ(X - 38 .. 43) oligomer or a derivative thereof as defined above and detecting a complex formed by the antibody and the oligomer or derivative, the presence of the complex indicating the presence of the auto-antibodies. In a preferred embodiment of the invention, the method comprises the use of a labelled derivative of the non-diffusible globular oligomer or its cross-linked derivative as described above.

In a preferred embodiment of the invention, the sample is derived from a subject suspected of having an amyloidosis and detecting the auto-antibodies is for diagnosing the presence or absence of the amyloidosis in the subject. In a preferred embodiment of the invention, the antibodies are detected using a labelled derivative of the non-diffusible globular oligomer or cross-linked derivative as described above.

It is particularly preferred if the subject suspected of having an amyloidosis is a subject having the amyloidosis or having an increased risk of getting the amyloidosis.

In one embodiment of the present invention, the samples are biological fluids which may be tested by the aforesaid method. These include plasma, whole blood, dried whole blood, serum, cerebrospinal fluid or aqueous or organo-aqueous extracts of tissues and cells.

According to a particular embodiment of the invention, detecting auto-antibodies as described above further comprises a pre-treatment of the preparation (sample) which causes dissociation of auto-antibody/antigen complexes. A method comprising such a pre-treatment may therefore be used in order to determine the total amount of auto-antibodies present in the preparation (sample) while a method not comprising said pre-treatment may be used in order to determine the amount of auto-antibodies which can still bind to the antigen. Further, both methods will allow to indirectly determine the amount of complexed auto-antibodies.

Conditions suitable for inducing dissociation of auto-antibody/antigen complexes are known to the skilled person. For instance, treating the preparation (sample) with acid, e.g., using a buffer such that the pH of the resulting preparation (sample) is in the range of 1 to 5, preferably in the range of 2 to 4 and in particular in the range of 2 to 3, may be expedient. Suitable buffers include salts in a physiological concentration, e.g. NaCl and acetic acid. The method for separation of antibody/antigen complexes has been described in WO2005/037209, which is incorporated herein in its entirety.

Briefly, dissociating the antibody from the antigen in the antibody/antigen complex comprising the steps of: contacting the sample containing an antibody/antigen complex with a dissociation buffer; incubating the sample; and optionally concentrating the sample.

The dissociation buffer may be a PBS buffer which has a pH in the range as indicated above. For instance a PBS buffer containing about 1.5% BSA and 0.2 M glycine-acetate pH 2.5, or 140 mM NaCl and 0.58% acetic acid is suitable.

Incubation for several minutes, for instance such as 10 to 30, e.g., 20 minutes at a temperature in the range of 20 to 40 °C has proven sufficient.

Concentration can be achieved in a manner known per se, for instance by passing the sample over a Centriprep YM30 (Amincon Inc.).

In one embodiment of the present invention, the Aβ(X - 38 .. 43) globulomer or derivative thereof is coated on a solid phase. The sample (e.g., whole blood, cerebrospinal fluid, serum, etc.) is then contacted with the solid phase. If auto-antibodies are present in the sample, such antibodies bind to the Aβ(X - 38 .. 43) globulomer or derivative thereof on the solid phase and are then detected by either a direct or indirect method. The direct method comprises simply detecting presence of the complex itself and thus presence of the auto-antibodies. In the indirect method, a conjugate is added to the bound auto-antibody. The conjugate comprises a second antibody, which binds to the first bound auto-antibodies, attached to a signal-generating compound or label. Should the second antibody bind to a bound first auto-antibody, the signal-generating compound generates a measurable signal. Such a signal then indicates presence of the first auto-antibodies in the sample.

Examples of solid phases used in diagnostic immunoassays are porous and nonporous materials, latex particles, magnetic particles, microparticles (see U.S. Patent No. 5,705,330), beads, membranes, microtiter wells and plastic tubes. The choice of the solid phase material and the method of labeling the antigen or antibodies present in the conjugate, if desired, are determined based upon desired assay format performance characteristics.

As noted above, the conjugate (or indicator reagent) will comprise an antibody (or perhaps anti-antibodies, depending upon the assay), attached to a signal-generating compound or "label". This signal-generating compound or label is itself detectable or may be reacted with one or more additional compounds to generate a detectable product. Examples of signal-generating compounds are described above and in particular include chromophores, radioisotopes (e.g., ¹²⁵I, ¹³¹I, ³²P, ³H, ³⁵S and ¹⁴C), chemiluminescent compounds (e.g., acridinium), particles (visible or fluorescent), nucleic acids, complexing agents, or catalysts such as enzymes (e.g., alkaline phosphatase, acid phosphatase, horseradish peroxidase, beta-galactosidase and ribonuclease). In the case of enzyme use (e.g., alkaline phosphatase or horseradish peroxidase), addition of a chromo-, fluro-, or lumo-genic substrate results in generation of a detectable signal. Other detection systems such as time-resolved fluorescence, internal-reflection fluorescence, amplification (e.g., polymerase chain reaction) and Raman spectroscopy are also useful.

Kits are also included within the scope of the present invention. More specifically, the present invention includes kits for determining the presence of auto-antibodies in a subject. In particular, a kit for determining the presence of auto-antibodies in a sample comprises a) a Aβ(X - 38 .. 43) globulomer or a derivative thereof, as defined herein; and b) a conjugate comprising an antibody attached to a signal generating compound capable of generating a detectable signal. The kit may also contain a control or calibrator which comprises a reagent which binds to the antigen.

The present invention also includes another type of kit for detecting auto-antibodies in a test sample. The kit may comprise a) an anti-antibody specific for the auto-antibody of interest, and b) Aβ(X - 38 .. 43) globulomer or a derivative thereof as defined above. A control or calibrator comprising a reagent which binds to the Aβ(X - 38 .. 43) globulomer or a derivative thereof may also be included. More specifically, the kit may comprise a) an anti-antibody specific for the auto-antibody and b) a conjugate comprising the Aβ(X - 38 .. 43) globulomer or a derivative thereof, the conjugate being attached to a signal generating compound capable of generating a detectable signal. Again, the kit may also comprise a control or calibrator comprising a reagent which binds to the antigen.

The kit may also comprise one container such as a vial, bottle or strip, with each container with a pre-set solid phase, and other containers containing the respective conjugates. These kits may also contain vials or containers of other reagents needed for performing the assay, such as washing, processing and indicator reagents.

In a preferred embodiment of the invention, such auto-antibodies can also be extracted from commercial immunoglobulin preparations like Octagam® (Octapharma Inc. Vienna, Austria), and further purified for therapeutic use.

Surprisingly and unexpectedly, the screening of preparations other than the preparations described above revealed that such preparations may contain substances that react with antibodies having specificity for the non-diffusible globular Aβ(X - 38 .. 43) oligomers or derivatives of the invention. Such substances which have a certain binding affinity to said antibodies but which cannot be said to correspond to the non-diffusible globular Aβ(X - 38 .. 43) oligomers or derivatives of the invention, are hereinafter referred to as globular Aβ(X - 38 .. 43) oligomer ("globulomer") epitopes or globular Aβ(X - 38 .. 43) oligomer ("globulomer") structure epitopes.

Due to their binding affinity to antibodies having specificity for the non-diffusible globular Aβ(X - 38 .. 43) oligomers or derivatives of the invention, said substances comprising Aβ(X - 38 .. 43) oligomer epitopes can be detected in preparations suspected of containing such epitopes, their amount can be determined in said preparations and they can be enriched. Accordingly, the present invention also relates to a screening method for detecting, for determining the amount of and/or for enriching Aβ(X - 38 .. 43) oligomer epitopes in preparations suspected or know to comprise such epitopes. Once detected and enriched, said substances may have potential applications similar to those described above with respect to the non-diffusible globular Aβ(X - 38 .. 43) oligomers or derivatives of the invention.

In the drawings:

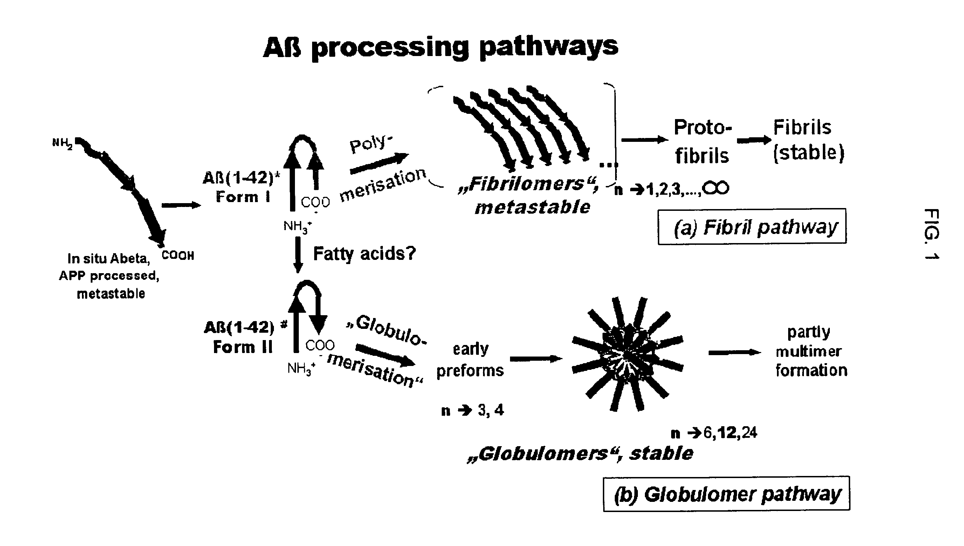

- Fig.1:: shows a schematic view of Aβ processing pathways. Aβ(1 - 42) oligomers exist in two different forms: the fibrillary type oligomer ("fibrillomer") and the globular type oligomer ("globulomer"). This model illustrates the two mutually exclusive pathways leading from monomers to either fibrils or globulomers. The "decision" for the globulomeric instead of the fibrillary pathway is made by a conformational shift at the monomeric level, which is presumed to be aided by fatty acids.

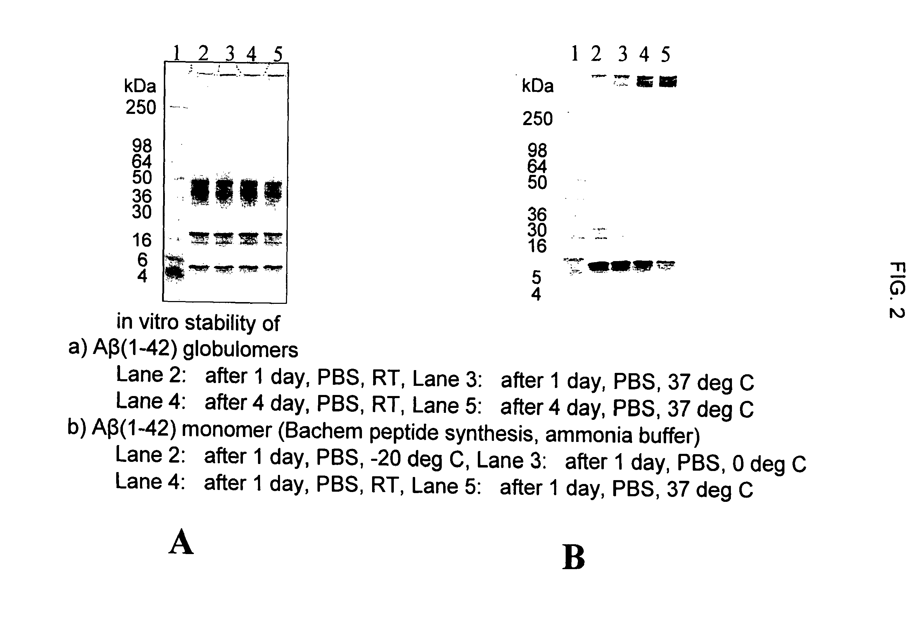

- Fig 2:: shows Western Blots of monomers and globulomers after storage at different temperatures in order to compare in vitro stability of A) Aβ(1 - 42) globulomer (lane 1: standard molecular marker proteins; lane 2: after 1 day, PBS, RT; lane 3: after 1 day, PBS, 37 °C; lane 4: after 4 days, PBS, RT; lane 5: after 4 days, PBS, 37 °C) and B) Aβ(1 - 42) monomer (lane 1: standard molecular marker proteins; lane 2: after 1 day, PBS, -20 °C; lane 3: after 1 day, PBS, -0 °C; lane 4: after 1 day, PBS, RT; lane 5: after 1 day, PBS, 37 °C);

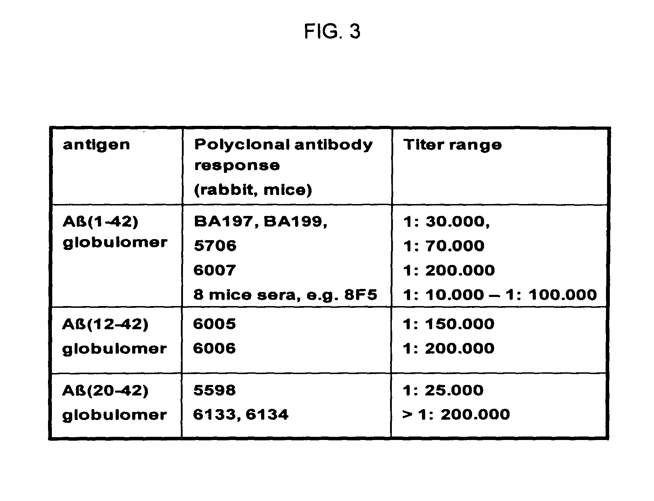

- Fig. 3:: presents antibody titers of rabbits and mice immunized with Aβ(1 - 42) globulomer, Aβ(12 - 42) globulomer and Aβ(20 - 42) globulomer;

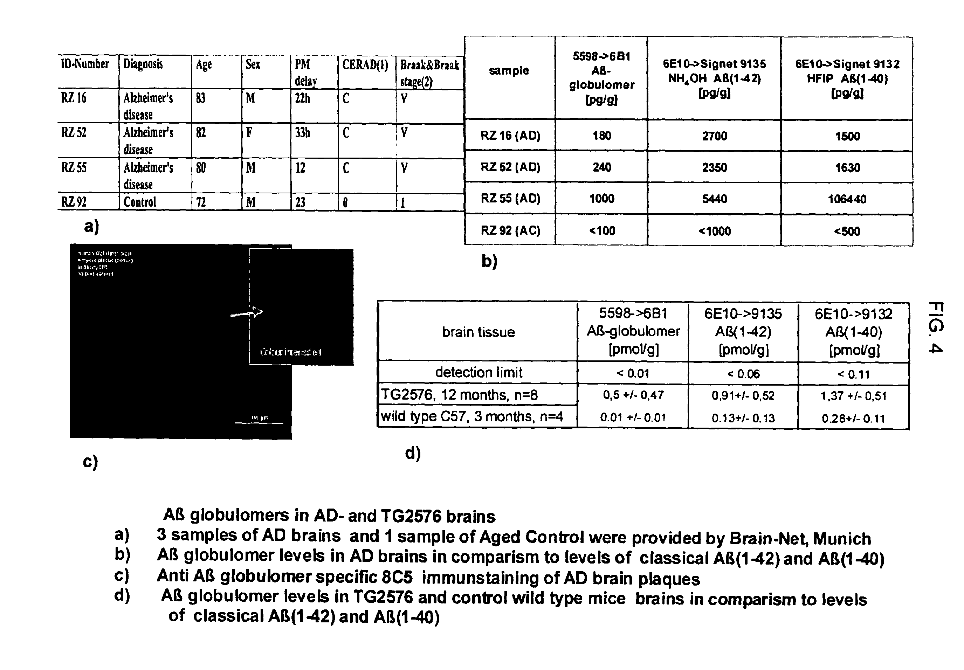

- Fig. 4:: shows the results of detection of Aβ(1 - 42) globulomer epitopes in AD- and TG2576 brains: A) data for 3 samples of AD brains and 1 sample of Aged Control provided by Brain-Net, Munich; B) levels of Aβ(1 - 42) globulomer, Aβ(1 - 42) monomer and Aβ(1 - 40) monomer in AD brains; C) immunostaining of AD brain plaques with Aβ(1 - 42) globulomer specific antibody 8C5; D) levels of Aβ(1 - 42) globulomer, Aβ(1 - 42) monomer and Aβ(1-40) monomer in TG2576 and control wild type mice brains;

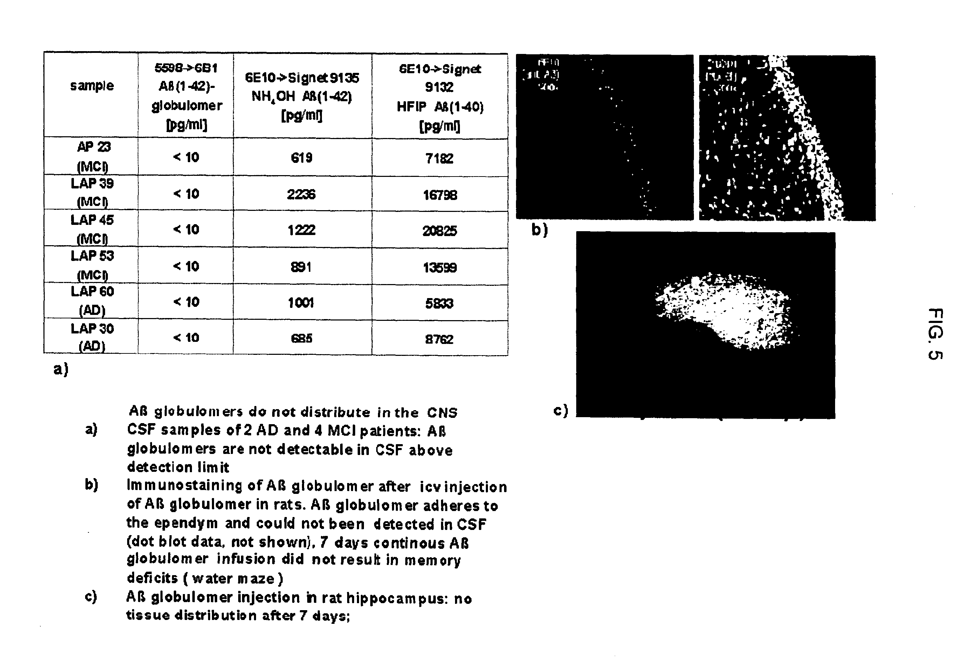

- Fig. 5:: shows the results of detection of Aβ(1 - 42) globulomer epitopes in the CNS: A) reactivity of CSF samples of 2 AD and 4 MCI patients with antiserum 5598 (capture antibody) and monoclonal non-selective anti Aβ(1-42) globulomer antibody 6B1 (primary antibody) in Sandwich-ELISA, B) immunostaining of Aβ(1 - 42) globulomers after icv injection of Aβ(1 - 42) globulomer in rats; C) immunostaining of Aβ(1 - 42) globulomers after injection of Aβ(1-42) globulomer in rat hippocampus;

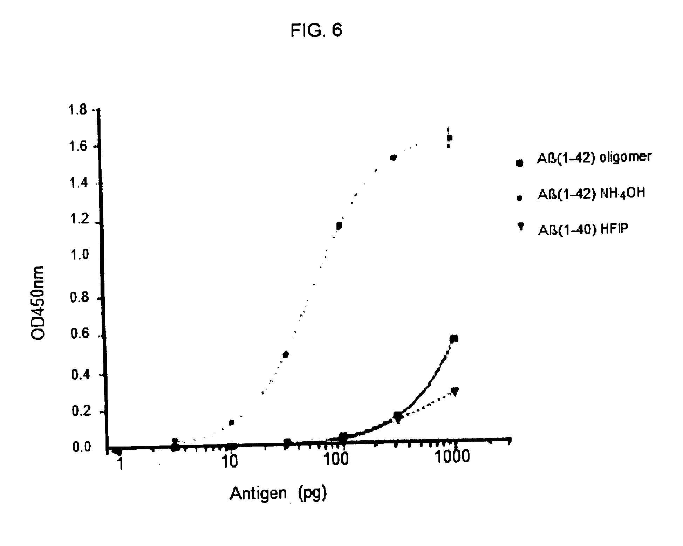

- Fig. 6:: shows reactivity of Aβ(33 - 42) directed PAb (Signet 9135) with various Aβ preparations in order to characterize Aβ(1 - 42) globulomer epitope. Said antibody only reacts with Aβ(1 - 42) monomer, dissolved in ammonia buffer, but does not react with the Aβ globulomer and Aβ(1 - 40) indicating non accessible or changed epitope in the globulomer compared to fibril prone Aβ(1 - 42) monomer, dissolved in ammonia buffer;

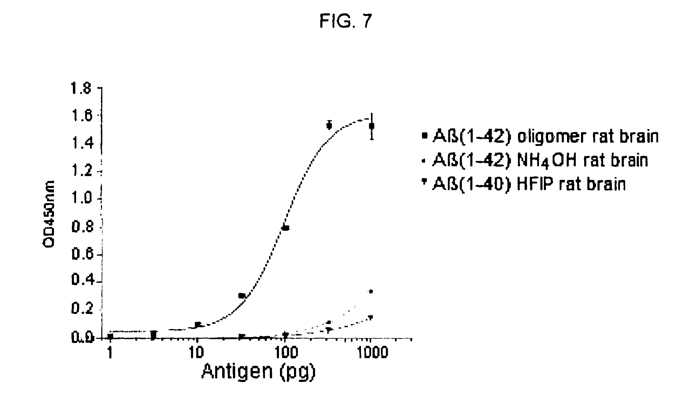

- Fig. 7:: shows reactivity of anti Aβ(20 - 42) globulomer derived antibody 5598 in sandwich ELISA. Rabbit anti Aβ(1 - 42) globulomer affinity-purified PAb 5598 displays selectivity for Aβ(1-42) globulomer versus other Aβ forms;

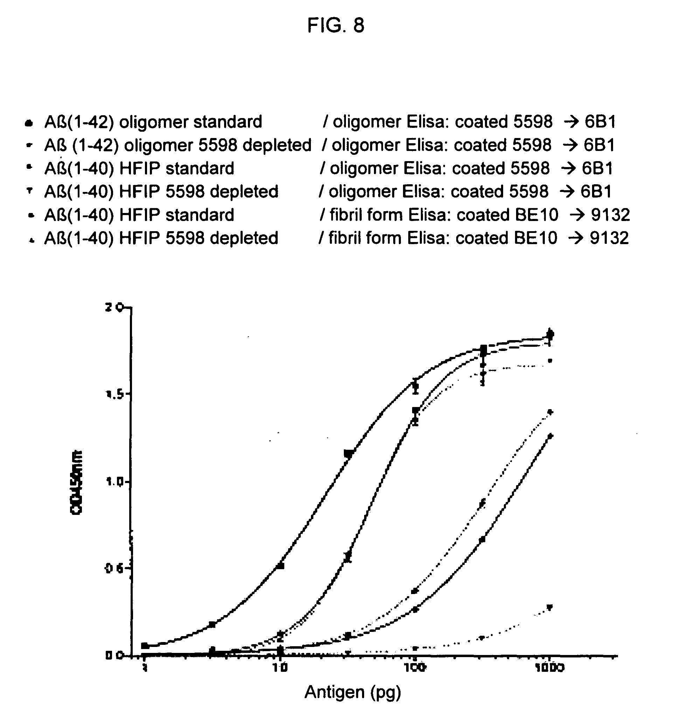

- Fig. 8:: shows the effect of pre-treatment of Aβ(1 - 42) globulomer and Aβ(1 - 40) monomer standard preparations with anti Aβ globulomer specific PAb 5598 measured in the following ELISAs:
- Aβ(1 - 42) globulomer, ± 5598depletion, Aβ globulomer ELISA : >10 fold shift (black to grey);
- Aβ(1 - 40) monomer, ± 5598depletion, Aβ(30 - 40) ELISA: no effect (dark green to light green);
- Aβ(1 - 40) monomer, ± 5598depletion, Aβ globulomer ELISA: 10 fold shift (dark blue to light blue);
indicating that Aβ globulomer epitope is also detectable in Aβ(1 - 40);
- Fig. 9:: shows SDS-PAGE chromatogram of the Aβ(20 - 42) globulomer as generated according to Example 5;
- Fig. 10:: shows SDS-PAGE chromatogram of the Aβ(12 - 42) globulomer as generated according to Example 6;
- Fig. 11:: summarizes data regarding the generation of Aβ(1 - 42) globulomers. Starting from synthetic Aβ(1 - 42) a stable and defined oligomer with a globular structure, 38/48 kDa Aβ(1 - 42) globulomer, can be generated:
(a) As a first step in the generation of 38/48 kDa Aβ(1 - 42) globulomer synthetic Aβ(1 - 42) peptide is dissolved in HFIP, subsequently evaporated and resuspended in DMSO. Incubation in PBS with 0.2% SDS results in intermediate 16/20 kDa Aβ(1 - 42) globulomers. Further dilution in water and incubation results in mature 38/48 Aβ(1 - 42) kDa globulomers which can be dialyzed or precipitated in methanol/acetic acid for buffer exchange;
(b) Several steps of the 38/48 kDa Aβ(1 - 42) globulomer generation are visualized on Coomassie stained 4 20% Tris-Glycine SDS-PAGE gels. Lanes: (1) intermediate 16/20 kDa Aβ(1 - 42) globulomer, (2) glutardialdehyde cross-linked intermediate 16/20 kDa Aβ(1 - 42) globulomer, (3) 38/48 kDa Aβ(1 - 42) globulomer, (4) glutardialdehyde cross-linked 38/48 kDa Aβ(1 - 42) globulomer (5) 38/48 kDa Aβ(1 - 42) globulomer after thermal denaturation in SDS sample buffer at 95 °C for 5 min, (6) glutardialdehyde cross-linked 38/48 kDa Aβ(1 - 42) globulomer after thermal denaturation in SDS sample buffer at 95 °C for 5 min (7) Aβ oligomers prepared according to Lambert, M.P. et al. Diffusible, non-fibrillary ligands derived from Abeta1 - 42 are potent central nervous system neurotoxins. Proc. Natl. Acad. Sci U. S. A 95, 6448 - 6453 (1998). Aβ forms are marked with m = monomer, i = intermediate 16/20 kDa Aβ(1 - 42) globulomer, g = 38/48 kDa Aβ(1 - 42) globulomer and f = fibrillary Aβ(1 - 42);
(c) Temporal stability of the 38/48 kDa Aβ(1 - 42) globulomer compared to the Aβ(1 - 42) monomer by incubation in PBS at different temperatures. Lanes 1 4: 38/48 kDa Aβ(1 - 42) globulomer after incubation for 1 day at RT and 37 °C (1, 2) and 4 days at RT and 37 °C (3, 4). Lanes 5 8: Aβ(1 - 42) NH4OH preparation after 1 day at -20 °C, 0 °C, RT and 37 °C;
(d) Size exclusion chromatography on Superose 12 column: Aβ(1 - 42) globulomer (top panel), glutardialdehyde cross-linked Aβ(1 - 42) globulomer (middle panel) and Aβ(1 - 42) NH₄OH preparation with 5 min incubation at RT after dissolving (lower panel);
(e) Limited proteolysis of the Aβ(1 - 42) globulomer and resulting truncated fragments (marked with t-g) separated by SDS-PAGE and visualized by Coomassie staining. Resulting C-terminal fragments (analyzed by SELDI MS) are indicated in brackets. Lanes: (1) undigested Aβ(1 - 42) globulomer (AA1 42, 4514 Da) (2) Papain (n.d.), (3) Elastase (n.d.), (4) Thermolysine (AA4 42, 4200 Da; AA20 42, 2218 Da; AA24 42, 1756 Da), (5) Chymotrypsin (AA21 42, 2067 Da), (6) Trypsin (AA6 42, 3897 Da; AA17 42, 2578 Da) (7) separated soluble Aβ₁₋₁₉ peptide filtrate after Thermolysine digestion;
(f) Aβ(1 - 42) globulomer (top panel) and glutardialdehyde cross-linked Aβ(1 - 42) globulomer (lower panel) were proteolysed by thermolysine and resulting peptide fragments analyzed by SELDI-MS;

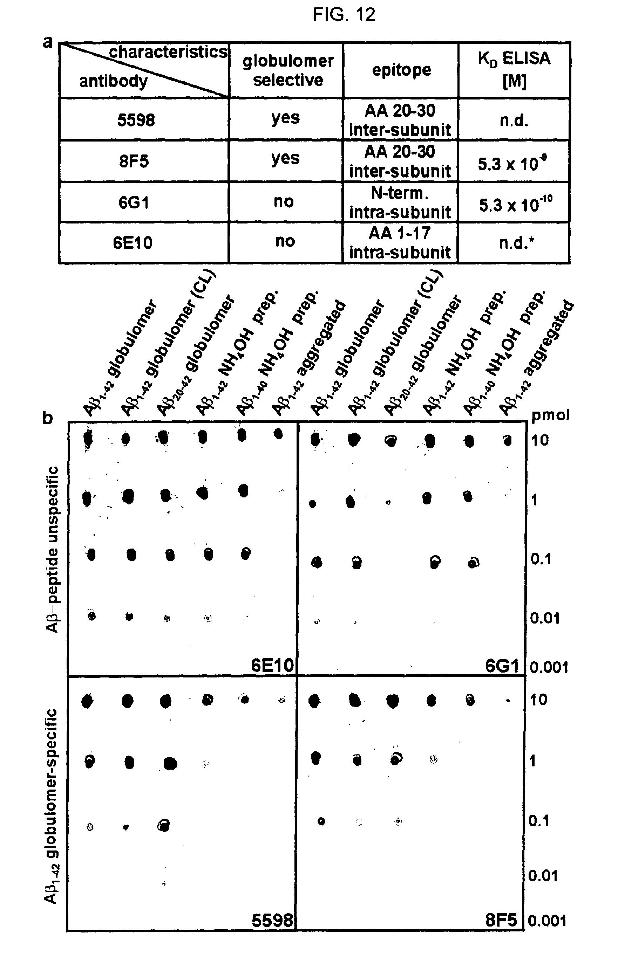

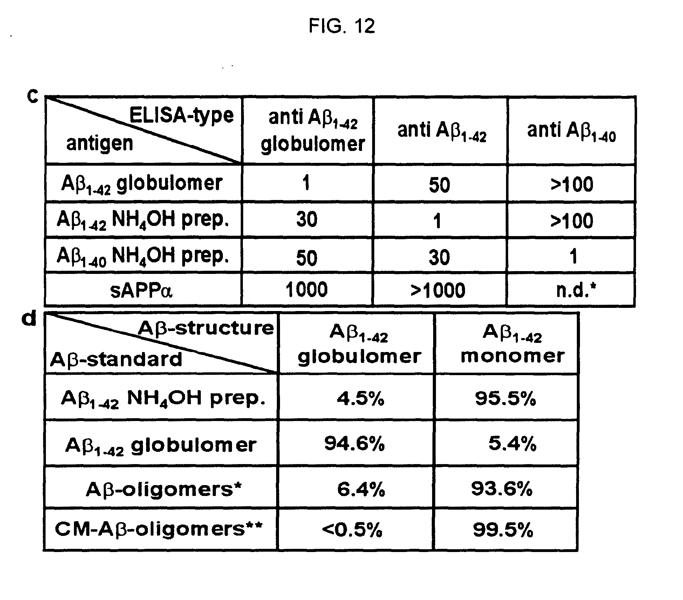

- Fig. 12:: shows reactivity of Aβ(1 - 42) globulomer specific antibodies having a high affinity and specificity in dot-blot and ELISA techniques:
(a) Comparison of anti Aβ antibodies regarding Aβ epitope binding and affinity. Aβ(1 - 42) globulomer specific polyclonal rabbit antibody 5598 and the mouse monoclonal antibody 8F5 recognize Aβ inter-subunit epitope while unspecific anti Aβ mouse monoclonal antibodies 6G1 and 6E10 recognize Aβ intra-subunit epitope. Affinity of 8F5 and 6G1 was determined by Scatchard analysis from ELISA;
(b) Specificity of anti Aβ(1 - 42) globulomer antibodies against diverse Aβ-forms was determined by dot-blot immunoassay. Aβ(1 - 42) globulomer specific antibodies 5598 and 8F5 (lower panel) recognize predominantly Aβ(1 - 42) globulomer forms and not standard preparation of Aβ₁₋₄₀ or Aβ(1 - 42) including aggregated Aβ(1 - 42), in contrast to unspecific antibodies 6G1 and 6E10 (upper panel);
(c) Quantification of discrimination of Aβ-forms in standard preparations by three different sandwich ELISA: quantification of Aβ(1 - 42) globulomer with 5598 as capture antibody and 6G1 as detection antibody, and of Aβ(1 - 40) or Aβ(1 - 42) levels with 6E10 as capture antibody and 9132 (recognizing AA30 - 40) and 9135 (recognizing AA33 - 42) as detection antibody, respectively.;
(d) Standards of specific Aβ-forms are cross contaminated with other Aβ-forms. Values were calculated as ratio between globulomer and non-globulomer forms in % measured by appropriate ELISAs. Previously described in vitro generated Aβ oligomers (* according to Lambert, M.P. et al. Diffusible, nonfibrillar ligands derived from Abeta1 - 42 are potent central nervous system neurotoxins. Proc. Natl. Acad. Sci U. S. A 95, 6448 - 6453 (1998)) and cell culture based Aβ oligomers in conditioned medium (** according to Walsh, D.M. et al. Naturally secreted oligomers of amyloid beta protein potently inhibit hippocampal long-term potentiation in vivo. Nature 416, 535 - 539 (2002)) were included in this experiment;

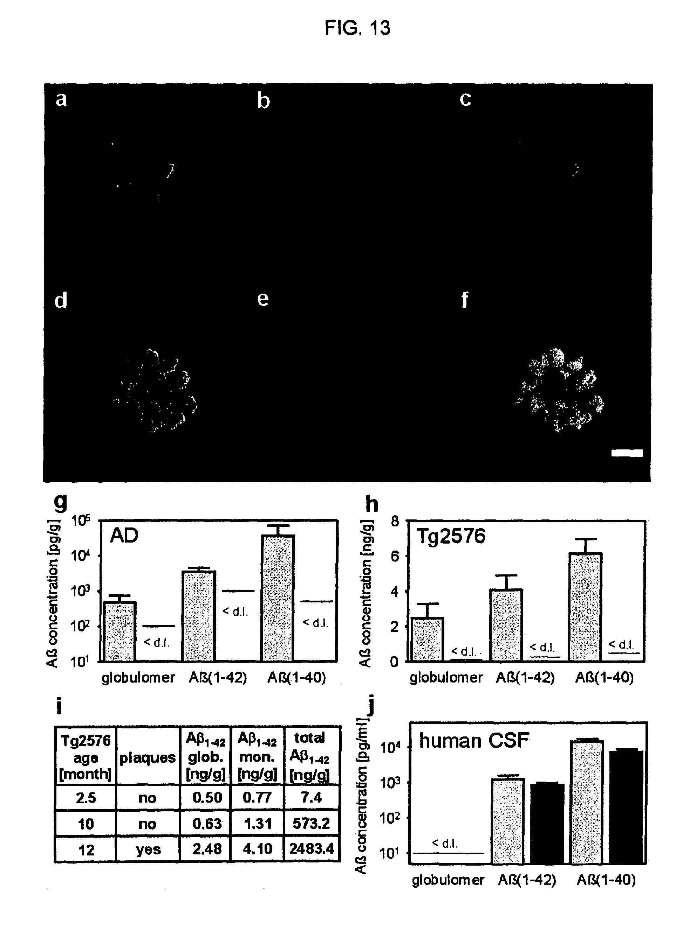

- Fig. 13:: shows that Aβ(1 - 42) globulomers are present in the brains of Alzheimer's patients and APP transgenic mice.
(a-f) Staining of amyloid plaques with thioflavine S (a, d), Aβ(1 - 42) globulomer specific antibody 8F5 (b, e), or both (c, f) in a cortical section of an Alzheimer's disease patient (a-c) and a transverse section of the cortex of a 12 month old Tg2576 mouse (d-f). Scale bar (for a-f): 20 µm.
(g-h) Concentration of Aβ(1 - 42) globulomers or monomeric Aβ species in the soluble fraction of the cortex of Alzheimer's disease patients (n = 3) (g) or 12 month old Tg2576 mice (n = 8) (h). Brains of an aged-matched control person (g) or C57BI6 control mice (n = 4) (h) had no Aβ species concentrations above the detection limit (<d.l.) indicated by the horizontal lines;
(i) Concentration of Aβ(1 - 42) globulomers or monomeric Aβ species in the cerebrospinal fluid of patients with mild cognitive impairment (grey bars; n = 4) or Alzheimer's disease patients (black bars; n = 2). No Aβ globulomers above the detection limit of 10 pg/ ml (horizontal line) were found in either group;

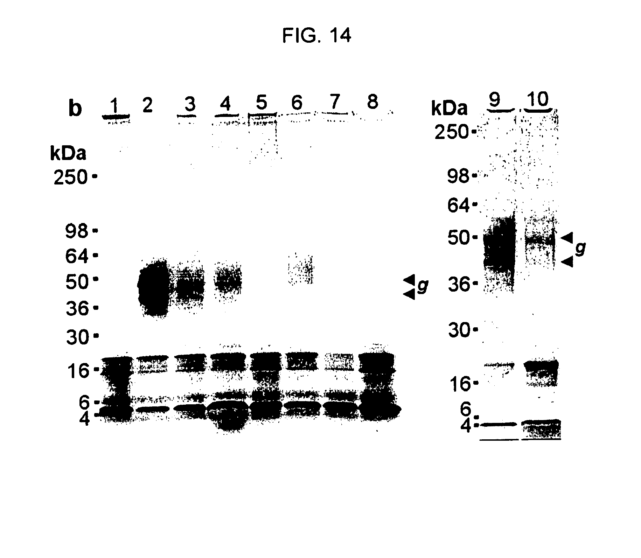

- Fig. 14:: shows that Aβ(1 - 42) globulomer generation can be induced by certain fatty acids. Following the general procedure of Aβ(1 - 42) globulomer generation SDS in the polymerization step was exchanged by various fatty acids at neutral pH. Lanes: (1) control without inducing agent, (2) positive control with 0.2% SDS, (3) 0.5% lauric acid (12:0), (4) 0.5% oleic acid (18:1), (5) linoleic acid (18:3), (6) eicosapentanoic acid (20:5), (7) docosatetranoic acid (22:4), (8) docosahexanoic acid (22:6), (9) positive control with 0.2% SDS, (10) 1 % arachidonic acid. Yields of Aβ(1 - 42) globulomer (g, indicated with arrowheads) differ between various fatty acids;

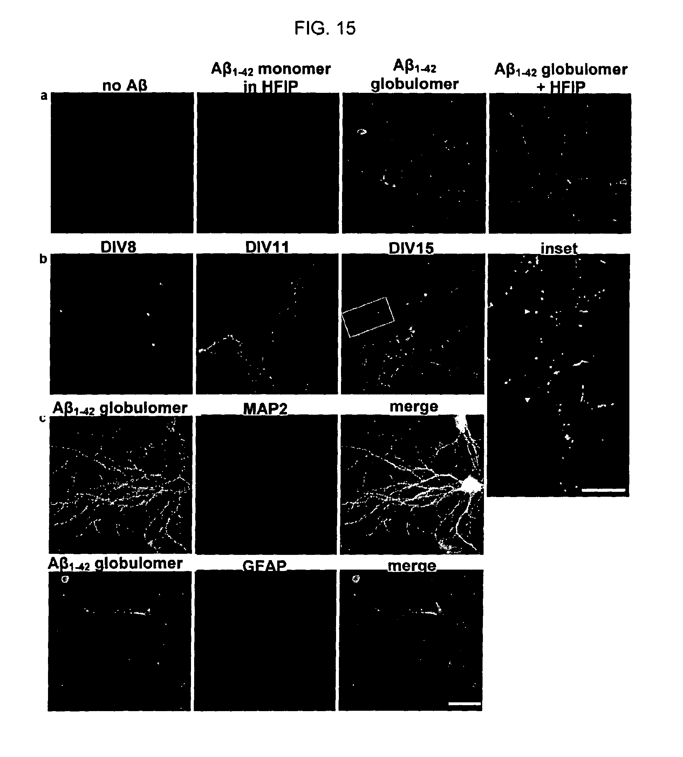

- Fig. 15:: shows that Aβ(1 - 42) globulomers bind specifically to hippocampal neurons depending on the culture age. Cultured hippocampal neurons were incubated with 200 nM Aβ forms (based on monomer concentration = 17 nM Aβ(1 - 42) globulomer) and visualized by immunofluorescence using anti Aβ 6E10 antibody (Aβ unspecific 6E10 was used to allow for quantitative comparison of Aβ(1 - 42) monomer and Aβ(1 - 42) globulomer binding);
(a) Aβ(1 - 42) globulomers showed a punctuated binding on the surface of hippocampal neurons in contrast to control without Aβ and Aβ(1-42) monomer after incubation for 15 min (37 °C, 5% CO₂);
(b) Aβ(1 - 42) globulomer binding to hippocampal cultures was tested at different days in vitro (DIV). Almost no binding (∼1%) of the Aβ(1-42) globulomer was found at DIV8, whereas at DIV11 and DIV15 nearly all (∼90%) of the hippocampal neurons bound Aβ1 42 globulomer. The inset in sample DIV15 shows the punctuated binding along and at some distance of the dendrite (arrowheads);
(c) Hippocampal cultures were double-stained with anti-MAP2 as neuronal marker and anti-GFAP as marker for glial-cells. GFAP positive cells showed no Aβ(1 - 42) globulomer binding in contrast to MAP2 positive cells. Microscope settings for Aβ signal were kept constant in between individual experiments. Scale bar 40 µm (inset = 10 µm);

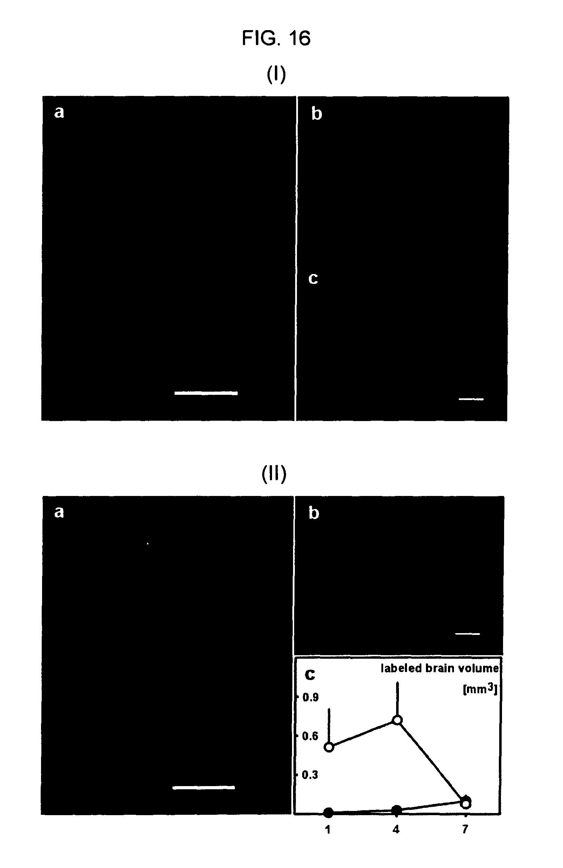

- Fig. 16: (I): shows that Aβ(1 - 42) globulomers have a limited tissue penetration due to high binding:
(a) Staining of Aβ(1 - 42) globulomers with specific antibody 8F5 (Cy 3; red) after i.c.v. infusion in the rat brain. Cell nuclei are counterstained with DAPI (blue). Scale bar: 100 µm;
(b-c) Staining of Aβ(1 - 42) globulomers (red) 1 day (b) or 7 days (c) after intracortical infusion into rat cortex. Cell nuclei are counterstained with DAPI (blue). Scale bar (for b-c): 200 µm;
- Fig. 16: (II): shows that Aβ(1 - 42) globulomers have a limited tissue penetration due to high binding:
(a) Staining of Aβ(1 - 42) globulomers with specific antibody 8F5 (Cy 3) after i.c.v. infusion in the rat brain. Cell nuclei are counterstained with DAPI. Scale bar: 100 µm;
(b) Staining of Aβ(1 - 42) globulomers 7 days after intracortical infusion into rat cortex. Cell nuclei are counterstained with DAPI. Scale bar: 200 µm;
(c) Time course of labeled volume (mean ± S.E.M.) after injection of comparable amounts of Aβ(1 - 42) globulomer (filled circles) into the right and of Aβ(1 - 42) monomer (open circles) into the left cortex of the same rats. The specific antibody 8F5 was used to detect Aβ(1-42) globulomer, and the antibody 6G1 was used to detect Aβ(1 - 42) monomer at 1 (n = 2), 4 (n = 3) and 7 days (n = 2) after injection;

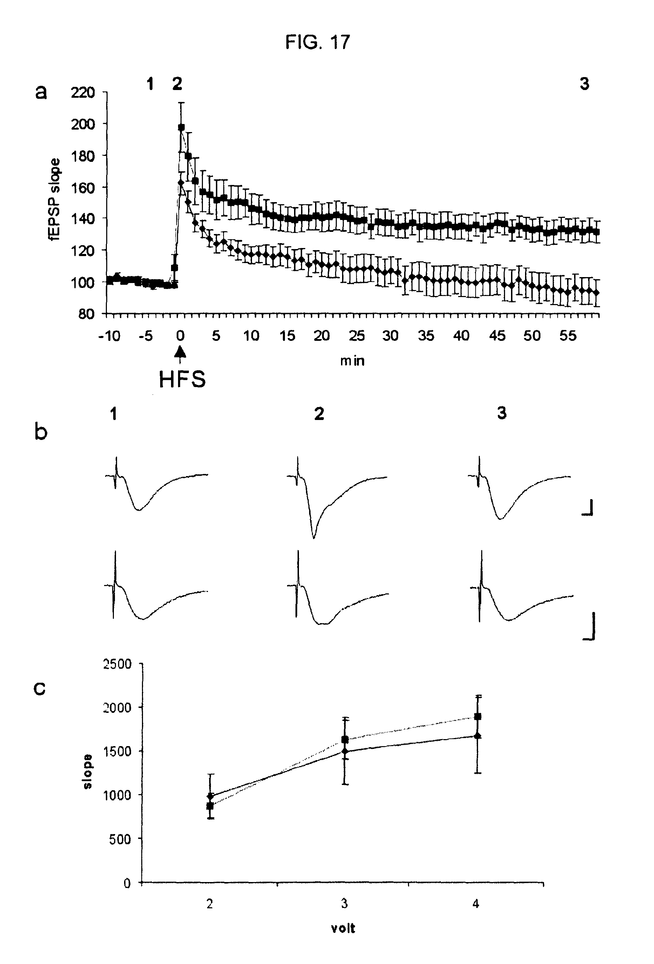

- Fig. 17:: shows that Aβ(1 - 42) globulomer completely blocks long-term potentiation in vitro:
(a) LTP induction in control (squares; n = 9) and 42 nM Aβ(1 - 42) globulomer-treated (diamonds; n = 6) rat brain slices. The field EPSP slope was expressed as percent change relative to baseline value (mean ± S.E.M.);
(b) Representative traces of control (upper row) and Aβ(1 - 42) globulomertreated (lower row) slices taken before (1), directly (2) and 90 min (3) after tetanisation;
(c) Input/output-relation is similar in control (squares; n = 11) and Aβ(1 - 42) globulomer (diamonds; n = 5) group. Horizontal bar: 2 ms; vertical bar: 1 mV;

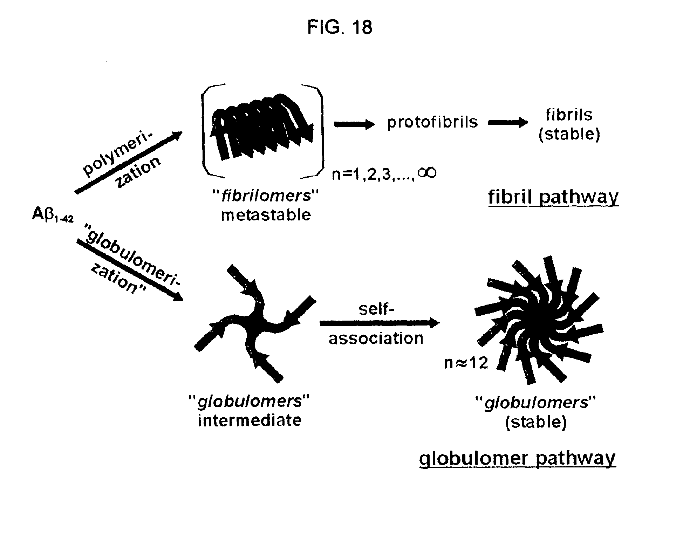

- Fig. 18:: shows a proposed model of a two pathway mechanism for Aβ-peptide multimerization. The initial step is Aβ-monomer generation by β- and γ-secretase cleavage of APP. Presumably depending on intrinsic or environmental factors two independent pathways of A Aβ(1 - 42) peptide multimerization may occur. The well described fibril pathway (Lomakin, A., Chung, D.S., Benedek, G.B., Kirschner, D.A. und Teplow, D.B., On the nucleation and growth of amyloid beta-protein fibrils: detection of nuclei and quantisation of rate constants, Proc. Natl. Acad. Sci. U.S.A., A93, 1125-1129 (1996)) follows classical polymerization kinetics. Aβ(1 - 42) monomers polymerize via metastable nucleation structures (fibrillomers) and further association of monomers to protofibrils and stable Aβ-fibrils composed of β sheet secondary structure. The Aβ(1 - 42) globulomer pathway is characterized by multimerization to structures with distinct Aβ-peptide numbers. As a first step an Aβ(1 - 42) globulomer intermediate is formed, which further self associates into a stable, Aβ(1 - 42) globulomer structure with a distinct number of Aβ-peptides (n = 12). The Aβ(1 - 42) globulomer has no tendency for further polymerization to fibrils. In the Aβ(1 - 42) globulomer the hydrophobic C termini extend to the interior of a globular structure thus avoiding exposure to the water phase. The more hydrophilic N termini are exposed to the outer surface. The Aβ(1 - 42)2 globulomer is stabilized by β sheet secondary structure. The high solubility and the defined and stable characteristics of the Aβ(1 - 42) globulomer can be attributed to this specific globular structure;

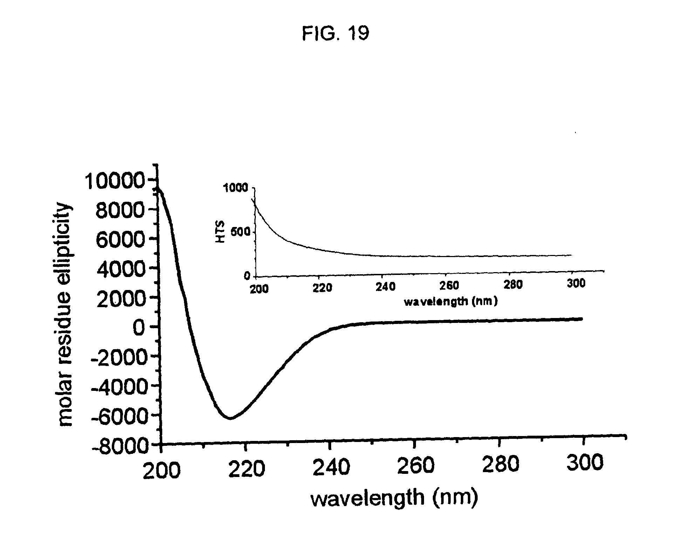

- Fig. 19:: shows a circular dichroism spectra of Aβ(1 - 42) globulomer. Spectra were collected for the Aβ(1 - 42) globulomer at 7.2 mg/ ml with the globulomer freshly buffer-exchanged into PBS as described (see Supplementary methods). The photomultiplier voltage (HTS) as a function of wavelength is shown in the inset. A strong minimum is observed at 216 nm, indicative of high β sheet content. No minima at 208 nm or 222 nm are apparent, suggesting little, if any, α helical content;

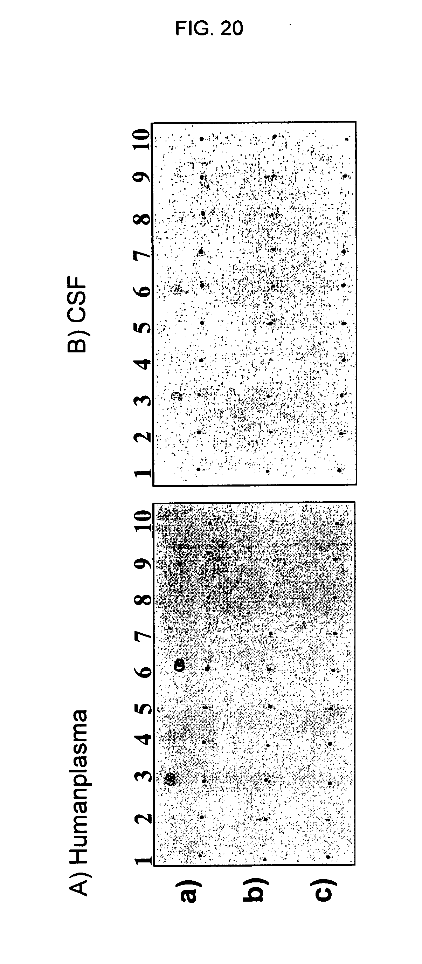

- Fig. 20:: shows dot blots of the reactivity of 100 pmol (row A); 10 pmol (row B); and 1 pmol (row C); of Aβ(1 - 42) globulomer (column 1); of Aβ(1 - 42) monomer in 0.1 % Pluronic F58 (column 2); Aβ(20 - 42) globulomer (column 3); of glutaraldehyde-crosslinked Aβ(1 - 42) globulomer (column 4); of ADDL prepared according to M.P. Lambert et al., J. Neurochem. 79, 595-605 (2001) at 4 °C or room temperature or 37 °C (column 5); Aβ(12 - 42) globulomer (column 6); of Aβ(1 - 40) monomer (column 7); of Aβ(1-42) dissolved in 0.1 % NH₄OH (column 8); of an Aβ(1-42) fibril preparation (column 9); and of PBS-diluted APP from Sigma (column 10) with A) human plasma; and B) CSF;

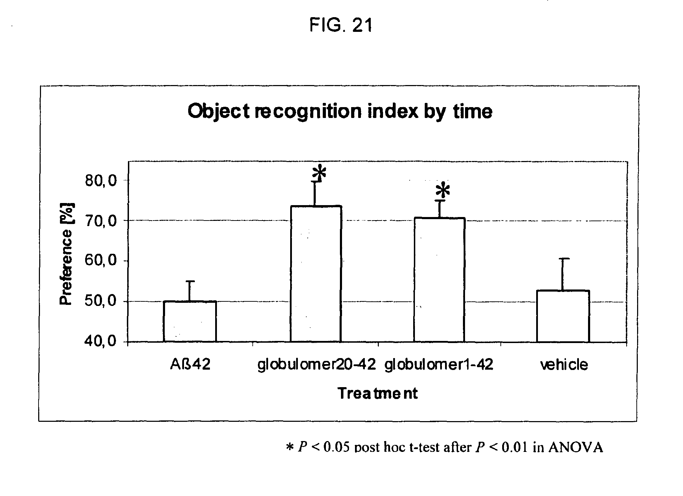

- Fig. 21:: refers to a novel object recognition task in actively immunized APP/Lo mice The preference of investigating a new object over an already known object is shown for groups of mice immunized either with the Aβ(1 - 42) monomer (n = 7), with Aβ(20 - 42) globulomer (n = 9) or with Aβ(1 - 42) globulomer (n = 8), or injected with vehicle (n = 8). Data are means ± S.E.M. After initial immunization mice were boosted every three weeks for three months. Immunization with globulomers led to a significantly increased recognition index, i.e., preference for the new object during the test trial (P<0.01 in ANOVA followed by post-hoc t-test);

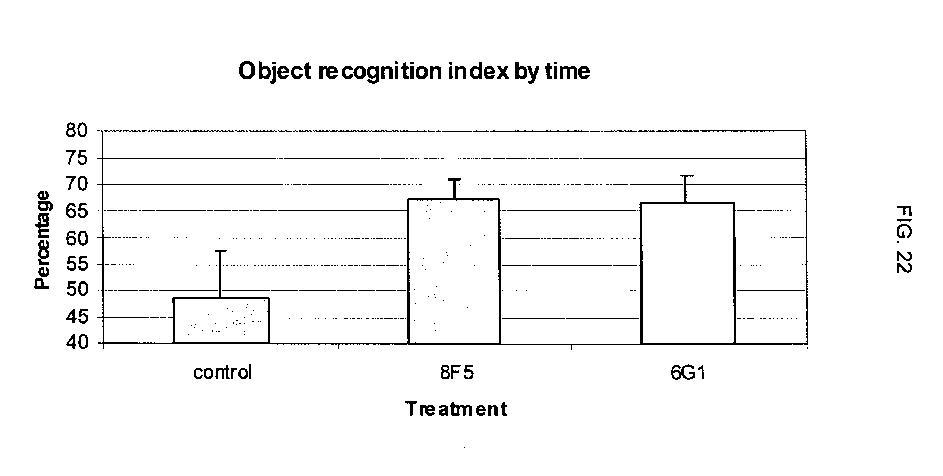

- Fig. 22:: shows a bar diagram with recognition index of mice that were treated with saline (control), antibody 8F5 and antibody 6G1 (data show means + SEM for 8 animals per group of the relative amount of time spent with a novel object compared to an object they introduced with 3 hours ago; exploration was allowed for 10 minutes in each test);

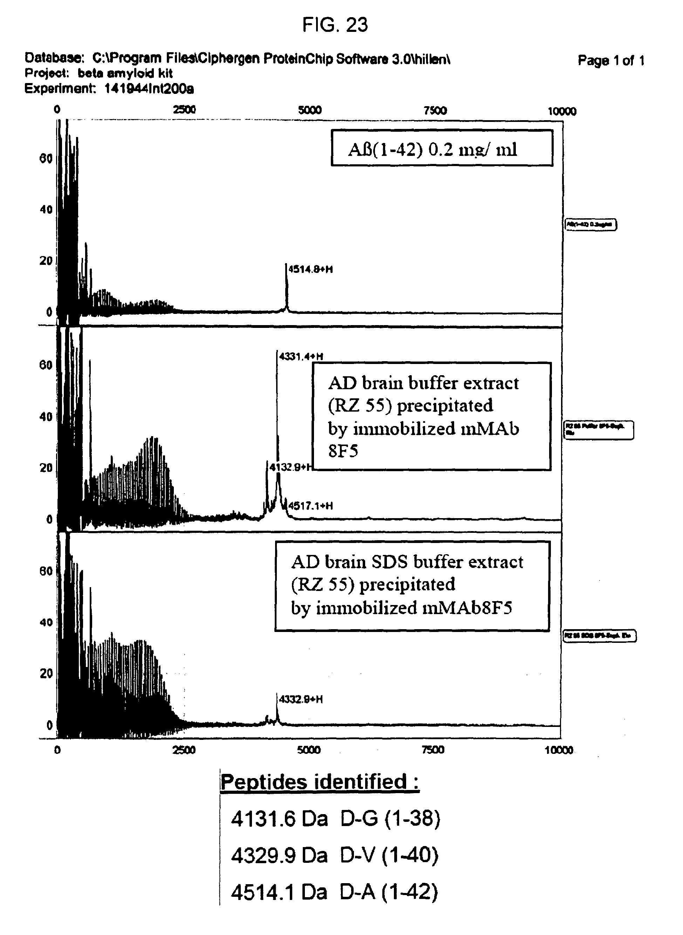

- Fig. 23:: shows SELDI mass spectrometry data from an AD brain extract which was immunoprecipitated by antibody 8F5. It can be seen that this antibody not only recognizes Aβ(1-42) (m/z = 4514) but also Aβ(1-40) species (m/z = 4329.9) as well as Aβ(1-38) species (m/z =4131.6);
- Fig 24:: shows auto-antibody levels derived from human serum/plasma directed against a variety of amyloid beta antigens:
(a) all reflective density values were taken from the 100 pMol antigen concentration;
   - free fraction values were taken from the non-treated sera/plasma
   - total fraction values were taken from the acid treated sera/plasma
   - bound fraction values = total values - free values;
(b) anti Aβ globulomer auto-antibodies levels are higher than anti Aβ monomer autoantibodies levels and are highest in AD patients compared to healthy controls.

### Detailed Description of the Invention:

ABBREVIATIONS as used throughout the present specification, claims and figures: AD, Alzheimer's disease; Aβ, amyloid β peptide; APP, amyloid precursor protein; BSA, bovine serum albumin; CD, circular dichroism spectroscopy; DIV, days in vitro; DMSO, dimethyl sulfoxide; GABA, γ-amino butyric acid; HFIP, 1,1,1,3,3,3-hexafluoro-2-propanol; LTP, long-term potentiation; NaH₂PO₄, Natriumphosphat; PBS, phosphate buffered saline; SDS, sodium dodecyl sulfate; SELDI-MS, surface enhanced laser desorption ionization-mass spectrometry; TBS, Tris buffered saline.

Amyloid β(1 - 42) (Aβ(1 - 42)) oligomers have recently been discussed as intermediate toxic species and putative causative agents and risk factors in Alzheimer's disease (AD) pathology and related conditions such as Down's syndrome, and their involvement with fatty acids, discussed below, suggests that they may also play a role in the dementing effects of certain metabolic disorders. In particular, the invention relates to a highly stable Aβ(1 - 42) oligomer species which can be easily prepared in vitro and is present in the brains of AD patients and Aβ(1 - 42)-overproducing transgenic mice. Physicochemical characterization reveals a pure, highly water-soluble globular oligomer of 60 kDa size which the inventors named "Aβ(1 - 42) globulomer".

The data provided herein indicate that the Aβ(1 - 42) globulomer is a persistent structural entity formed independently of the fibrillary aggregation pathway. It is a potent antigen in mice and rabbits eliciting generation of Aβ(1 - 42) globulomer epitope-specific antibodies which do not significantly cross-react with amyloid precursor protein, Aβ(1 - 40) and Aβ(1 - 42) monomers, and Aβ fibrils. Aβ(1 - 42) globulomer binds specifically to dendritic processes of neurons but not to glia in hippocampal cell cultures and completely blocks long term potentiation in rat hippocampal slices. The data provided herein further suggest that the Aβ(1 - 42) globulomer epitope represents a basic pathogenic structural principle present likely to be present at least in low abundance in previously described oligomer preparations, and that its formation is an early pathological event in AD. Selective neutralization of the Aβ(1 - 42) globulomer structure epitope is expected to have a high potential for treatment of AD.

The ability of Aβ peptides to block LTP has been described in the Art.

A similar blockade of LTP was found with the Aβ(1 - 42) globulomer at concentrations as low as about 40 nM. However, in contrast to previously described soluble oligomers, the preparation according to the invention did not contain a mixture of low molecular Aβ(1 - 42) oligomers but consisted of one homogeneous and stable species only. Therefore the fact that the effective concentration required for the globulomers is lower by an order of magnitude than that reported for previously described preparations may indicate that only a minor part of said preparations does actually exert any detrimental effect.

The previously described Aβ oligomer preparations were primarily characterized by function, i.e., their neurotoxic effects at a given concentration. In addition to these neurotoxic effects the intrinsic high stability and defined chemical nature and steric arrangement of the Aβ(1 - 42) globulomer of the present invention allowed a characterization towards homogeneity, molecular weight, and globular character and structural topology.

In vitro generation of Aβ(1 - 42) globulomers started with an unfolding of synthetic Aβ(1 - 42) as can be induced by agents which break down hydrogen bonds and subsequent refolding into a new conformation. Although this conformational transition was nearly complete, as analyzed by SDS-PAGE, the Aβ(1 - 42) globulomers showed a characteristic double band with an apparent molecular weight of 38/48 kDa. However, cross-linking with glutaraldehyde merged these two bands into one, indicating that Aβ(1 - 42) globulomers do exist only in one conformation. Homogeneity was also shown by size exclusion chromatography in which Aβ(1 - 42) globulomers produced one, although broad peak, which became more defined, i.e., narrower, after cross-linking. Thus, size exclusion chromatography of the cross-linked Aβ(1 - 42) globulomer under native conditions revealed a molecular weight of ∼60 kDa with minimal variance, which was also confirmed by analytical ultracentrifugation. Calculations suggest that the Aβ(1 - 42) globulomer consists of approximately 12 to 14, preferably 12 Aβ subunits, and further experimental data suggest that it may be formed via an unstable tetrameric intermediate.

Several experiments addressed the globular character and structural topology of the Aβ(1 - 42) globulomer. For the mature Aβ(1 - 42) globulomer a molten globular structure which further condenses upon cross-linking to an almost perfectly globular structure was determined.

First, size exclusion chromatography of the Aβ(1 - 42) globulomer with a narrow elution profile, especially of the cross-linked form, suggests a globular character. In addition, the hydrodynamic volume can be calculated from these data and related to the total number of amino acids resulting in a measure for the extent of folding (Tcherkasskaya, O. & Uversky, V.N. Denatured collapsed states in protein folding: example of apomy-oglobin. Proteins 44, 244 -254 (2001); Uversky, V.N. Natively unfolded proteins: a point where biology waits for physics. Protein Sci 11, 739 - 756 (2002)).

Second, digestion of the Aβ(1 - 42) globulomer with a variety of unspecific proteases cleaved off about 20 amino acids from the N-terminal site, while retaining the oligomeric structure of a protease-resistant C-terminal core. This result also indicated a uniform structural topology with each of the 12 single Aβ subunits forming the Aβ(1 - 42) globulomer pointing in the same direction.

Third, mass spectrometry confirmed the uniform globular character and the structural topology, because cross-linking occurred only between amino groups of the N-termini and Lys-16, while sparing Lys-28.

Fourth, and finally, antibodies selective for Aβ(33 - 42) and Aβ(33 - 40) which are used in conventional ELISA for determination of Aβ(1 - 42) and Aβ(1 - 40) concentration do not react with Aβ(1 - 42) globulomers, demonstrating once more that the C-terminus is not accessible.

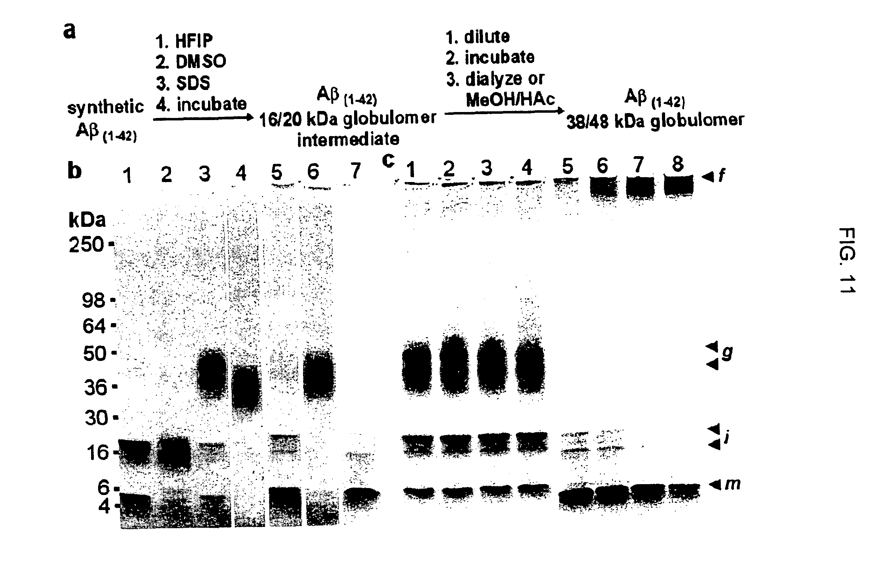

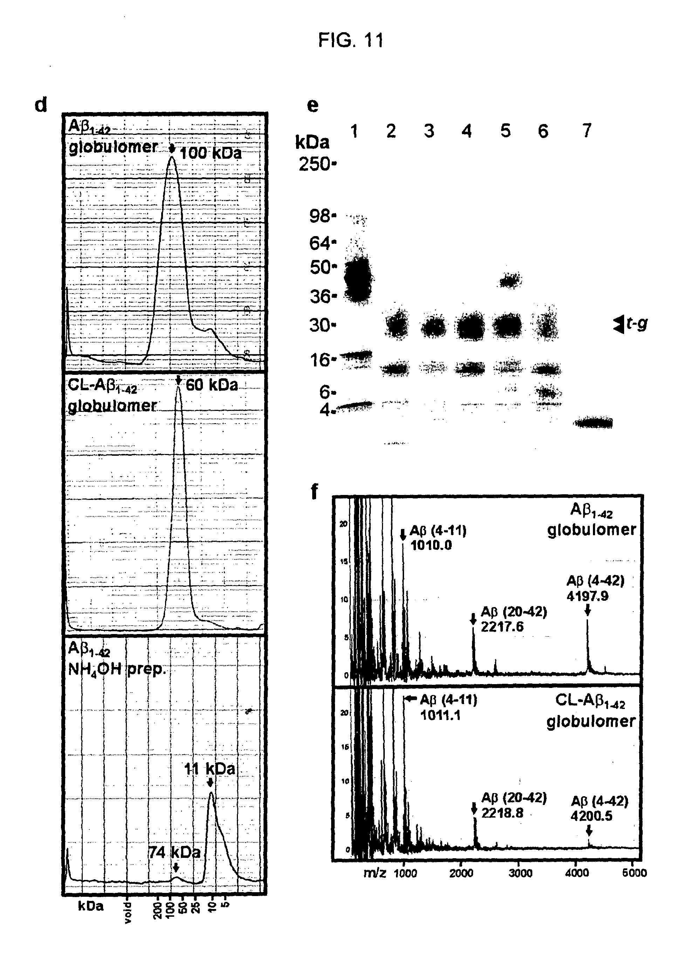

CD spectroscopy suggests that a substantial fraction of the Aβ (1 - 42) globulomer, presumably the C-terminus, is present in β-structure (see Fig. 11), a common characteristic of amyloid proteins in their pathological polymerized form (Rochet, J.C. & Lansbury, P.T., Jr. Amyloid fibrillogenesis: themes and variations. Curr Opin Struct. Biol 10, 60 - 68 (2000)). In the resulting model of the Aβ(1 - 42) globulomer, hydrophobic C-termini (Aβ(24 - 42)) form the inside core, while the hydrophilic N-termini (Aβ(1-19∼23)) are exposed at the outer surface, thereby making the Aβ(1 - 42) globulomer highly water soluble (see Fig. 18).

In summary, the Aβ(1 - 42) globulomer of the invention is a homogenous, defined and stable species with comparable neuropathologic effects on LTP as previously described for soluble Aβ oligomers (Lambert, M.P. et al. Diffusible, nonfibrillar ligands derived from Abeta 1 - 42 are potent central nervous system neurotoxins. Proc. Natl. Acad. Sci U. S. A 95, 6448 - 6453 (1998); Walsh, D.M. et al. Naturally secreted oligomers of amyloid beta protein potently inhibit hippocampal long-term potentiation in vivo. Nature 416, 535 - 539 (2002); Wang, H.W. et al. Soluble oligomers of beta amyloid 1-42 inhibit long-term potentiation but not long-term depression in rat dentate gyrus. Brain Res 924, 133 - 140 (2002) ; Wang, Q., Walsh, D.M., Rowan, M.J., Selkoe, D.J. & Anwyl, R. Block of long-term potentiation by naturally secreted and synthetic amyloid beta-peptide in hippocampal slices is mediated via activation of the kinases c-Jun N-terminal kinase, cyclin-dependent kinase 5, and p38 mitogen-activated protein kinase as well as metabotropic glutamate receptor type 5. J Neurosci 24, 3370 - 3378 (2004)), distinguished by not being diffusible.

For the understanding of the concept of diffusible and non-diffusible ligands it is important to understand that there are two generally independent pathways for processing of APP-derived Aβ(1 - 42) monomers formed in situ:
a) the fibril pathway;
b) the globulomer pathway.

Of these, the fibril pathway has been well characterized for more than twenty years as being the quantitatively dominant pathway for Aβ processing in vitro and in vivo. It is well accepted that Aβ(1 - 40) and especially Aβ(1 - 42) or related derivatives, such as Aβ(1 - 38), Aβ(1 - 39), Aβ(1 - 41) or Aβ(1 - 43), readily polymerize starting from a monomer unit and continuously grow via oligomers (associations of two to twenty-four monomers) and protofibrils (associations of more than twenty-four monomers) finally into insoluble fibrils which are deposited in vitro or in vivo in brain tissue or vessel walls. This fibril formation is so prominent that earlier researchers attributed the dementing effects of Aβ to it. It is important to realize that a special subset of oligomers which the present inventors suggest to name "fibrillomers" (see Fig. 1), different from the globulomers, here occur as intermediates.

The invention here describes a new pathway for stabilisation of in situ formed metastable Aβ which comprises switching its structural conformation on the level of the monomer to an alternative three-dimensional structure, which subsequently results in a different type of association which the inventors have termed "globulomerisation". Characteristically, because of the changed spatial structure of Aβ(1 - 42) (Fig. 1) the processing leads to a terminal globular type oligomer core structure ("globulomers") which is completely different from the non-terminal fibrillary type oligomer core structure ("fibrillomers"). "Terminal" here means that unlike fibrillomers, globulomers do not assemble further to fibrils.

Since the Aβ(1 - 42) globulomer is stable and not an intermediate of higher-molecular aggregates, it is proposed that their formation reflects an alternative pathway to formation of fibrillary aggregates. Assuming that soluble oligomers are toxic to neurons or otherwise detrimental for neuronal function, it may be that those along the fibril pathway are "naturally" inactivated by further polymerization to mature fibrils (Caughey, B. & Lansbury, P.T. Protofibrils, pores, fibrils, and neurodegeneration: separating the responsible protein aggregates from the innocent bystanders. Annu. Rev Neurosci 26, 267 - 298 (2003)), which may even represent a natural attempt at scavenging Aβ by sequestration. In contrast, the Aβ(1 - 42) globulomer is the endpoint of an alternative pathway as a neurotoxic Aβ(1 - 42) multimer with long-term stability under physiological conditions (see Fig. 18).

For the relevance of the Aβ(1 - 42) globulomer it is required that it exists in brains of AD patients. Therefore selective monoclonal antibodies have been raised and Aβ(1 - 42) globulomer epitopes have been detected along with fibrillary Aβ in cerebral plaque deposits of both AD patients and mice transgenic for human APP. Parallel to these findings, ADDLs have also been detected in the brain of AD patients (Lacor, P.N. et al. Synaptic targeting by Alzheimer's-related amyloid beta oligomers. J Neurosci 24, 10191 - 10200 (2004); Gong, Y. et al. Alzheimer's disease-affected brain: presence of oligomeric A beta ligands (ADDLs) suggests a molecular basis for reversible memory loss. Proc. Natl. Acad. Sci U. S. A 100, 10417 - 10422 (2003)). It will be appreciated that, according to the definition given above, ADDLs are essentially different from globulomers in that they were described to be diffusible; however, as discussed above, at least a part of their effect may actually be due to a hitherto undetected amount of substance present in the ADDL preparation that cross-reacts with globulomer-specific antibodies. ADDLs may therefore be said to comprise a low amount Aβ(1 - 42) globulomer epitopes. Using Aβ(1 - 42) globulomer-specific antibodies the amount of said epitopes can be determined to be about 5 %. Such an amount may also account to some extent for the observed neurotoxic effects of said preparation.

Given that Aβ(1 - 42) globulomers naturally occur in AD brains, it remains unclear how the Aβ(1 - 42) globulomer, so far demonstrated in vitro only, may form in vivo. This question has been addressed by replacing SDS, which was used in the original Aβ(1 - 42) globulomer preparation process, with naturally occurring fatty acids. The presence of several of the fatty acids also resulted in aggregation to the Aβ(1 - 42) globulomer.

The same effect, alone or in combination with fatty acids, is also expected to occur with anionic detergents (e.g. SDS) or polyanionic agents (e.g. the glycosaminoglycan heparin). These chemically distinct agents share the capacity to induce amyloid protein aggregation or oligomerization by providing an anionic charged surface for induction and stabilization of conformational changes and for nucleation, suggesting that anionic surfaces, in form of micelles or vesicles, are indeed able to induce Aβ(1 - 42) globulomer formation. Anionic membranes or lipid rafts may serve this function in vivo. This suggests that dementia induced by certain metabolic disorders, in particular by lipid metabolism disorders, may also involve globulomer formation, and that the present invention may therefore be useful in the diagnosis and treatment of this condition as well.

The mode and site of action of Aβ(1 - 42) globulomers in vivo is not known at present. Although soluble by means of their hydrophilic shell they are not detectable in the cerebrospinal fluid, but rather bind fast and tightly to the tissue, in particular when compared to Aβ(1 - 42) monomers. This binding of Aβ(1 - 42) globulomers seems to be of physiological relevance since it specifically targeted hippocampal neurons in primary cultures, suggesting a specific mode of binding.

Several observations exclude the possibility that the interaction of the hydrophilic Aβ(1 - 42) globulomers with hippocampal neurons is unspecific and might be based on a phenomenon like the known insertion of Aβ peptide into lipid bilayers (McLaurin, J. & Chakrabartty, A. Membrane disruption by Alzheimer beta-amyloid peptides mediated through specific binding to either phospholipids or gangliosides. Implications for neurotoxicity. J Biol Chem 271, 26482 - 26489 (1996); Verdier, Y., Zarandi, M. & Penke, B. Amyloid beta-peptide interactions with neuronal and glial cell plasma membrane: binding sites and implications for Alzheimer's disease. J Pept Sci 10, 229 - 248 (2004)).

Most importantly, monomeric Aβ(1 - 42) (known to be innocuous and even implied to have anti-dementing effects) showed no detectable binding to neuronal structures (Plant, L.D. et al.: The production of amyloid beta peptide is a critical requirement for the viability of central neurons. J. Neurosci.23, 5531-5535). Then, Aβ(1 - 42) globulomer binding was strongly dependent on the stage of the cell culture and was restricted to neuronal cells only, leaving glia unlabeled. The punctuated binding pattern of Aβ(1 - 42) globulomers along dendrites resembles that of ADDLs, which have been shown to bind to neurons and colocalize with postsynaptic markers like PSD-95 (Lacor, P.N. et al. Synaptic targeting by Alzheimer's-related amyloid beta oligomers. J Neurosci 24, 10191 - 10200 (2004)). Thus, it appears that bound Aβ(1 - 42) globulomer is specifically localized at postsynaptic processes, and its binding may be restricted to dendritic spines. It is speculated that there it binds to its still uncharacterized receptor, interfering with electrochemical information processing but not with the essential housekeeping biochemistry of the cell. This would explain the specific block of LTP, while leaving basic neurophysiology as well as short-term potentiation unaltered. The suppression of LTP (but not of basic membrane physiology) also indicates that Aβ(1 - 42) globulomer may specifically interfere with learning and memory in vivo, suggesting that the Aβ(1 - 42) globulomer is causal for the memory loss observed in AD patients.

In the present invention, "neurotoxicity" is understood to denote any significant loss of neuronal and/or synaptic function without regard for its influences on the viability of the respective cell.

Although the Aβ(1 - 42) globulomers were found to be stable under physiological conditions in vitro, degradation under in vivo conditions is likely. The experiment with unspecific proteases demonstrated that the hydrophilic N-termini which protrude from the globular structure can be cleaved, leaving a truncated Aβ(1 - 42) globulomer. The existence of a variety of N-terminal truncated Aβ species in AD has been reported recently (Sergeant, N. et al. Truncated beta-amyloid peptide species in pre-clinical Alzheimer's disease as new targets for the vaccination approach. J Neurochem 85, 1581 - 1591 (2003)) suggesting that N-terminal truncation is indeed a possible physiological event in the brain.

Blockade of LTP seems to be a common pathophysiological characteristic of Aβ(1 - 42) globulomer and previously described preparations of Aβ oligomers (Lambert, M.P. et al. Diffusible, nonfibrillar ligands derived from Abeta1 - 42 are potent central nervous system neurotoxins. Proc. Natl. Acad. Sci U. S. A 95, 6448 - 6453 (1998); Walsh, D.M. et al. Naturally secreted oligomers of amyloid beta protein potently inhibit hippocampal long-term potentiation in vivo. Nature 416, 535 - 539 (2002); Wang, H.W. et al. Soluble oligomers of beta amyloid 1 - 42 inhibit long-term potentiation but not long-term depression in rat dentate gyrus. Brain Res 924, 133 - 140 (2002); Wang, Q., Walsh, D.M., Rowan, M.J., Selkoe, D.J. & Anwyl, R. Block of long-term potentiation by naturally secreted and synthetic amyloid beta-peptide in hippocampal slices is mediated via activation of the kinases c-Jun N-terminal kinase, cyclin-dependent kinase 5, and p38 mitogen-activated protein kinase as well as metabotropic glutamate receptor type 5. J Neurosci 24, 3370 - 3378 (2004)).

It may well be that several types of soluble oligomers occur early in AD, targeting the same neuronal function. Specifically, other functionally characterized Aβ(1 - 42) oligomers (Walsh, D.M. et al. Naturally secreted oligomers of amyloid beta protein potently inhibit hippocampal long-term potentiation in vivo. Nature 416, 535 - 539 (2002); Wang, Q., Walsh, D.M., Rowan, M.J., Selkoe, D.J. & Anwyl, R. Block of long-term potentiation by naturally secreted and synthetic amyloid beta-peptide in hippocampal slices is mediated via activation of the kinases c-Jun N-terminal kinase, cyclin-dependent kinase 5, and p38 mitogen-activated protein kinase as well as metabotropic glutamate receptor type 5. J Neurosci 24, 3370 - 3378 (2004)) produced by a cell line expressing mutant human APP are rather of the prefibril type. The inventors found no Aβ(1 - 42) globulomer epitope among the Aβ oligomers released by a similar cell line.

Nevertheless, Aβ(1 - 42) oligomers obtained from this cell line (Walsh, D.M. et al. Naturally secreted oligomers of amyloid beta protein potently inhibit hippocampal long-term potentiation in vivo. Nature 416, 535 - 539 (2002); Wang, Q., Walsh, D.M., Rowan, M.J., Selkoe, D.J. & Anwyl, R. Block of long-term potentiation by naturally secreted and synthetic amyloid beta-peptide in hippocampal slices is mediated via activation of the kinases c-Jun N-terminal kinase, cyclin-dependent kinase 5, and p38 mitogen-activated protein kinase as well as metabotropic glutamate receptor type 5. J Neurosci 24, 3370 - 3378 (2004)) effectively blocked LTP, thereby indicating that different soluble Aβ(1 - 42) oligomer species are neurotoxic (Lambert, M.P. et al. Diffusible, nonfibrillar ligands derived from Abeta 1 - 42 are potent central nervous system neurotoxins. Proc. Natl. Acad. Sci U. S. A 95, 6448 - 6453 (1998); Walsh, D.M. et al. Naturally secreted oligomers of amyloid beta protein potently inhibit hippocampal long-term potentiation in vivo. Nature 416, 535 - 539 (2002); Wang, H.W. et al. Soluble oligomers of beta amyloid 1 - 42 inhibit long-term potentiation but not long-term depression in rat dentate gyrus. Brain Res 924, 133 - 140 (2002); Wang, Q., Walsh, D.M., Rowan, M.J., Selkoe, D.J. & Anwyl, R. Block of long-term potentiation by naturally secreted and synthetic amyloid beta-peptide in hippocampal slices is mediated via activation of the kinases c-Jun N-terminal kinase, cyclin-dependent kinase 5, and p38 mitogen-activated protein kinase as well as metabotropic glutamate receptor type 5. J Neurosci 24, 3370 - 3378 (2004)).

But it is also likely that residual contaminations of Aβ(1 - 42) globulomer epitopes are a small but critical fraction of the ADDLs as the culprits of the pathological nature of the soluble Aβ(1 - 42) oligomers mixture comprising the ADDLs. Regardless of this, working with the Aβ(1 - 42) globulomers has the big advantage to have a defined homogenous population of Aβ(1 - 42) multimers. This is obvious when raising antibodies for scientific or therapeutic purposes.

The present invention also relates to monoclonal and polyclonal antibodies with different specificity against various globulomer epitopes.

The first monoclonal mouse antibody 6G1 was shown to bind to a typical promiscuous N-terminal located linear Aβ epitope shared by all structural types of Aβ(1 - X) species. Thus, 6G1 was found to be very potent but to have an immunotype profile similar to 6E10.

In contrast, two other antibodies, the mouse monoclonal antibody 8F5 and the affinity-purified polyclonal rabbit antibody PAb 5598, were selective for Aβ(1 - 42) globulomers compared to Aβ monomers or fibrils. However, they both cross-react with the truncated Aβ(20 - 42) oligomer, therefore map beyond amino acid 20. Since these antibodies access the globulomer after native but not after denaturating SDS Western blotting (data not shown), it is likely that both antibodies recognize a structural, non-linear epitope in-between subunits in the region of amino acid 20 and 30. Such structural epitopes have been recently discussed to be shared by diverse amyloid proteins with varying primary sequence, therefore eliciting their neurotoxic function by a shared common structure (Kayed, R. et al. Common structure of soluble amyloid oligomers implies common mechanism of pathogenesis. Science 300, 486 - 489 (2003); Glabe, C.G. Conformation-dependent antibodies target diseases of protein misfolding. Trends Biochem Sci 29, 542 - 547 (2004)).

These antibodies provide the tools to search for Aβ(1 - 42) globulomers in the brains of AD patients and human APP transgenic mice. Initially, there was concern about low selectivity versus Aβ(1 - 42) monomer preparations. Later, it was found that the lack of Aβ(1 - 42) globulomers in the presence of abundant Aβ monomers in both CSF samples of AD patients and in conditioned medium of APP overexpressing cells, proofed a much higher selectivity for Aβ(1 - 42) globulomers than initially determined. Indeed, the in vitro standard preparations of Aβ(1 - 42) monomers contained contaminations of Aβ(1 - 42) globulomer epitopes and the Aβ(1 - 42) globulomer selective antibodies were now able to determine the degree of contamination. Thus, Aβ(1 - 42) globulomer epitopes were shown to be the main component in artificial 38/48 kDa globulomer preparations, but a minor component in another previously described Aβ oligomer preparation (Lambert, M.P. et al. Diffusible, nonfibrillar ligands derived from Abeta 1 - 42 are potent central nervous system neurotoxins. Proc. Natl. Acad. Sci U. S. A 95, 6448 - 6453 (1998)).

Antibodies raised against Aβ(1 - 42) globulomers do not only allow for detection of this species in various in vitro preparations and in vivo, but also provide a means to selectively inactivate a defined oligomer species. Besides the possibility to study the role of a specific neurotoxic protein in the genesis of AD, such a specific antibody recognizing a structural epitope is expected to be superior for therapeutic treatment because any side effect associated with an antibody reaction to other common, but less toxic Aβ(1 - 42) species can be avoided. In this respect, it is interesting to note that Aβ(1 - 42) globulomers mounted an excellent immune response resulting in high antibody titers, providing the possibility fort active and passive immunization against Aβ(1 - 42) globulomers.

Thus, Aβ(1 - 42) globulomers as well as specific antibodies raised against them, including derivatives of the latter such as recombinant, humanized and/or chimeric antibodies, are expected to be useful in the diagnosis as well as the treatment of, and/or the manufacturing of substances useful in the diagnosis and/or treatment of Alzheimer's disease and other conditions involving Aβ globulomers. It is conceivable that these conditions may include Down's syndrome and dementia brought about by metabolic disorders such as certain forms of hyperlipidemia.

The term "Aβ(X-Y)" here refers to the amino acid sequence from amino acid position X to amino acid position Y of the human amyloid β protein including both X and Y, in particular to the amino acid sequence from amino acid position X to amino acid position Y of the amino acid sequence DAEFRHDSGY EVHHQKLVFF AEDVGSNKGA IIGLMVGGVV IAT (corresponding to amino acid positions 1 to 43) or any of its naturally occurring variants, in particular those with at least one mutation selected from the group consisting of A2T, H6R, D7N, A21G ("Flemish"), E22G ("Arctic"), E22Q ("Dutch"), E22K ("Italian"), D23N ("Iowa"), A42T and A42V wherein the numbers are relative to the start of the β peptide, including both position X and position Y or a sequence with up to three additional amino acid substitutions none of which may prevent globulomer formation, preferably with no additional amino acid substitutions in the portion from amino acid 12 or X, whichever number is higher, to amino acid 42 or Y, whichever number is lower, more preferably with no additional amino acid substitutions in the portion from amino acid 20 or X, whichever number is higher, to amino acid 42 or Y, whichever number is lower, and most preferably with no additional amino acid substitutions in the portion from amino acid 20 or X, whichever number is higher, to amino acid 40 or Y, whichever number is lower, an "additional" amino acid substation herein being any deviation from the canonical sequence that is not found in nature.

More specifically, the term "Aβ(1-42)" here refers to the amino acid sequence from amino acid position 1 to amino acid position 42 of the human amyloid β protein including both 1 and 42, in particular to the amino acid sequence DAEFRHDSGY EVHHQKLVFF AEDVGSNKGA IIGLMVGGVV IA or any of its naturally occurring variants, in particular those with at least one mutation selected from the group consisting of A2T, H6R, D7N, A21G ("Flemish"), E22G ("Arctic"), E22Q ("Dutch"), E22K ("Italian"), D23N ("Iowa"), A42T and A42V wherein the numbers are relative to the start of the Aβ peptide, including both 1 and 42 or a sequence with up to three additional amino acid substitutions none of which may prevent globulomer formation, preferably with no additional amino acid substitutions in the portion from amino acid 20 to amino acid 42. Likewise, the term "Aβ(1-40)" here refers to the amino acid sequence from amino acid position 1 to amino acid position 40 of the human amyloid β protein including both 1 and 40, in particular to the amino acid sequence DAEFRHDSGY EVHHQKLVFF AEDVGSNKGA IIGLMVGGVV or any of its naturally occurring variants, in particular those with at least one mutation selected from the group consisting of A2T, H6R, D7N, A21G ("Flemish"), E22G ("Arctic"), E22Q ("Dutch"), E22K ("Italian"), and D23N ("Iowa") wherein the numbers are relative to the start of the Aβ peptide, including both 1 and 40 or a sequence with up to three additional amino acid substitutions none of which may prevent globulomer formation, preferably with no additional amino acid substitutions in the portion from amino acid 20 to amino acid 40.

More specifically, the term "Aβ(12-42)" here refers to the amino acid sequence from amino acid position 12 to amino acid position 42 of the human amyloid β protein including both 12 and 42, in particular to the amino acid sequence VHHQKLVFF AEDVGSNKGA IIGLMVGGVV IA or any of its naturally occurring variants, in particular those with at least one mutation selected from the group consisting of A21G ("Flemish"), E22G ("Arctic"), E22Q ("Dutch"), E22K ("Italian"), D23N ("Iowa"), A42T and A42V wherein the numbers are relative to the start of the Aβ peptide, including both 12 and 42 or a sequence with up to three additional amino acid substitutions none of which may prevent globulomer formation, preferably with no additional amino acid substitutions in the portion from amino acid 20 to amino acid 42.

More specifically, the term "Aβ(20-42)" here refers to the amino acid sequence from amino acid position 20 to amino acid position 42 of the human amyloid β protein including both 20 and 42, in particular to the amino acid sequence F AEDVGSNKGA IIGLMVGGVV IA or any of its naturally occurring variants, in particular those with at least one mutation selected from the group consisting of A21G ("Flemish"), E22G ("Arctic"), E22Q ("Dutch"), E22K ("Italian"), D23N ("Iowa"), A42T and A42V wherein the numbers are relative to the start of the Aβ peptide, including both 20 and 42 or a sequence with up to three additional amino acid substitutions none of which may prevent globulomer formation, preferably without any additional amino acid substitutions.

The term "Aβ(X-Y) globulomer" (Aβ(X-Y) globular oligomer) here refers to a soluble, globular, non-covalent association of Aβ(X- Y) peptides as defined above, possessing homogeneity and distinct physical characteristics. According to one aspect, Aβ(X-Y) globulomers are stable, non-fibrillar, oligomeric assemblies of Aβ(X-Y) peptides which are obtainable by incubation with anionic detergents. In contrast to monomer and fibrils, these globulomers are characterized by defined assembly numbers of subunits (e.g. early assembly forms, n=4-6, "oligomers A", and late assembly forms, n=12-14, "oligomers B", as described in WO2004/067561). The globulomers have a 3-dimensional globular type structure ("molten globule", see Barghorn et al., 2005, J Neurochem, 95, 834-847). They may be further characterized by one or more of the following features:
- cleavability of N-terminal amino acids X-23 with promiscuous proteases (such as thermolysin or endoproteinase GluC) yielding truncated forms of globulomers;
- non-accessibility of C-terminal amino acids 24-Y with promiscuous proteases and antibodies;
- truncated forms of these globulomers maintain the 3-dimensional core structure of said globulomers with a better accessibility of the core epitope Aβ(20-Y) in its globulomer conformation.

According to the invention and in particular for the purpose of assessing the binding affinities of the antibodies of the present invention, the term "Aβ(X-Y) globulomer" here refers to a product which is obtainable by a process as described in WO 2004/067561, which is incorporated herein by reference.

Said process comprises unfolding a natural, recombinant or synthetic Aβ(X-Y) peptide or a derivative thereof; exposing the at least partially unfolded Aβ(X-Y) peptide or derivative thereof to a detergent, reducing the detergent action and continuing incubation.

For the purpose of unfolding the peptide, hydrogen bond-breaking agents such as, for example, hexafluoroisopropanol (HFIP) may be allowed to act on the protein. Times of action of a few minutes, for example about 10 to 60 minutes, are sufficient when the temperature of action is from about 20 to 50°C and in particular about 35 to 40°C. Subsequent dissolution of the residue evaporated to dryness, preferably in concentrated form, in suitable organic solvents miscible with aqueous buffers, such as, for example, dimethyl sulfoxide (DMSO), results in a suspension of the at least partially unfolded peptide or derivative thereof, which can be used subsequently. If required, the stock suspension may be stored at low temperature, for example at about -20°C, for an interim period.

Alternatively, the peptide or the derivative thereof may be taken up in slightly acidic, preferably aqueous, solution, for example an about 10 mM aqueous HCl solution. After an incubation time of usually a few minutes, insoluble components are removed by centrifugation. A few minutes at 10000 g is expedient. These method steps are preferably carried out at room temperature, i.e. a temperature in the range from 20 to 30°C. The supernatant obtained after centrifugation contains the Aβ(X-Y) peptide or the derivative thereof and may be stored at low temperature, for example at about -20°C, for an interim period.

The following exposure to a detergent relates to the oligomerization of the peptide or the derivative thereof to give an intermediate type of oligomers (in WO 2004/067561 referred to as oligomers A). For this purpose, a detergent is allowed to act on the at least partially unfolded peptide or derivative thereof until sufficient intermediate oligomer has been produced.

Preference is given to using ionic detergents, in particular anionic detergents.

According to a particular embodiment, a detergent of the formula (I):

R-X,

is used, in which
the radical R is unbranched or branched alkyl having from 6 to 20 and preferably 10 to 14 carbon atoms or unbranched or branched alkenyl having from 6 to 20 and preferably 10 to 14 carbon atoms,
the radical X is an acidic group or salt thereof, with X being preferably selected from among
-COO⁻M⁺, -SO₃⁻M⁺, and especially
-OSO₃⁻M⁺ and M⁺ is a hydrogen cation or an inorganic or organic cation preferably selected from alkali metal and alkaline earth metal cations and ammonium cations.

Advantageous are detergents of the formula (I), in which R is unbranched alkyl of which alk-1-yl radicals must be mentioned in particular. Particular preference is given to sodium dodecyl sulfate (SDS). Lauric acid and oleic acid can also be used advantageously. The sodium salt of the detergent lauroylsarcosin (also known as sarkosyl NL-30 or Gardol^{®}) is also particularly advantageous.

The time of detergent action in particular depends on whether - and if yes, to what extent - the peptide or the derivative thereof subjected to oligomerization has unfolded. If, according to the unfolding step, the peptide or derivative thereof has been treated beforehand with a hydrogen bond-breaking agent, i.e. in particular with hexafluoroisopropanol, times of action in the range of a few hours, advantageously from about 1 to 20 and in particular from about 2 to 10 hours, are sufficient when the temperature of action is about 20 to 50°C and in particular about 35 to 40°C. If a less unfolded or an essentially not unfolded peptide or derivative thereof is the starting point, correspondingly longer times of action are expedient. If the peptide or the derivative thereof has been pretreated, for example, according to the procedure indicated above as an alternative to the HFIP treatment or said peptide or derivative thereof is directly subjected to oligomerization, times of action in the range from about 5 to 30 hours and in particular from about 10 to 20 hours are sufficient when the temperature of action is about 20 to 50°C and in particular about 35 to 40°C. After incubation, insoluble components are advantageously removed by centrifugation. A few minutes at 10000 g is expedient.

The detergent concentration to be chosen depends on the detergent used. If SDS is used, a concentration in the range from 0.01 to 1 % by weight, preferably from 0.05 to 0.5% by weight, for example of about 0.2% by weight, proves expedient. If lauric acid or oleic acid are used, somewhat higher concentrations are expedient, for example in a range from 0.05 to 2% by weight, preferably from 0.1 to 0.5% by weight, for example of about 0.5% by weight.

The detergent action should take place at a salt concentration approximately in the physiological range. Thus, in particular NaCl concentrations in the range from 50 to 500 mM, preferably from 100 to 200 mM and particularly at about 140 mM are expedient.

The subsequent reduction of the detergent action and continuation of incubation relates to a further oligomerization to give the Aβ(X-Y) globulomer of the invention (in WO 2004/067561 referred to as oligomers B). Since the composition obtained from the preceding step regularly contains detergent and a salt concentration in the physiological range it is then expedient to reduce detergent action and, preferably, also the salt concentration. This may be carried out by reducing the concentration of detergent and salt, for example, by diluting, expediently with water or a buffer of lower salt concentration, for example Tris-HCI, pH 7.3. Dilution factors in the range from about 2 to 10, advantageously in the range from about 3 to 8 and in particular of about 4, have proved suitable. The reduction in detergent action may also be achieved by adding substances which can neutralize said detergent action. Examples of these include substances capable of complexing the detergents, like substances capable of stabilizing cells in the course of purification and extraction measures, for example particular EO/PO block copolymers, in particular the block copolymer under the trade name Pluronic® F 68. Alkoxylated and, in particular, ethoxylated alkyl phenols such as the ethoxylated t-octylphenols of the Triton® X series, in particular Triton® X100, 3-(3-cholamidopropyldimethylammonio)-1-propanesulfonate (CHAPS®) or alkoxylated and, in particular, ethoxylated sorbitan fatty esters such as those of the Tween® series, in particular Tween® 20, in concentration ranges around or above the particular critical micelle concentration, may be equally used.

Subsequently, the solution is incubated until sufficient Aβ(X-Y) globulomer of the invention has been produced. Times of action in the range of several hours, preferably in the range from about 10 to 30 hours and in particular in the range from about 15 to 25 hours, are sufficient when the temperature of action is about 20 to 50°C and in particular about 35 to 40°C. The solution may then be concentrated and possible residues may be removed by centrifugation. Here too, a few minutes at 10000 g proves expedient. The supernatant obtained after centrifugation contains an Aβ(X-Y) globulomer of the invention.

An Aβ(X-Y) globulomer of the invention can be finally recovered in a manner known per se, e. g. by ultrafiltration, dialysis, precipitation or centrifugation.

It is further preferred if electrophoretic separation of the Aβ(X-Y) globulomers under denaturing conditions, e. g. by SDS-PAGE, produces a double band (e. g. with an apparent molecular weight of 38 / 48 kDa for Aβ(1-42)), and especially preferred if upon glutardialdehyde treatment of the globulomers before separation these two bands are merged into one. It is also preferred if size exclusion chromatography of the globulomers results in a single peak (e. g. corresponding to a molecular weight of approximately 60 kDa for Aβ(1-42)).

Starting out from Aβ(1-42) peptide, Aβ(12-42) peptide, and Aβ(20-42) peptide said processes are in particular suitable for obtaining Aβ(1-42) globulomers, Aβ(12-42) globulomers, and Aβ(20-42) globulomers.

In a particular embodiment of the invention, Aβ(X-Y) globulomers wherein X is selected from the group consisting of the numbers 2 .. 24 and Y is as defined above, are those which are obtainable by truncating Aβ(1-Y) globulomers into shorter forms wherein X is selected from the group consisting of the numbers 2 .. 24, with X preferably being 20 or 12, and Y is as defined above, which can be achieved by treatment with appropriate proteases. For instance, an Aβ(20-42) globulomer can be obtained by subjecting an Aβ(1-42) globulomer to thermolysin proteolysis, and an Aβ(12-42) globulomer can be obtained by subjecting an Aβ(1-42) globulomer to endoproteinase GluC proteolysis. When the desired degree of proteolysis is reached, the protease is inactivated in a generally known manner. The resulting globulomers may then be isolated following the procedures already described herein and, if required, processed further by further work-up and purification steps. A detailed description of said processes is disclosed in WO 2004/067561, which is incorporated herein by reference.

The term "Aβ(X-Y) monomer" here refers to the isolated form of the Aβ(X-Y) peptide, preferably a form of the Aβ(X-Y) peptide which is not engaged in essentially non-covalent interactions with other Aβ peptides. Practically, the Aβ(X-Y) monomer is usually provided in the form of an aqueous solution. In a particularly preferred embodiment of the invention, the aqueous monomer solution contains 0.05% to 0.2%, more preferably about 0.1 % NH₄OH. In another particularly preferred embodiment of the invention, the aqueous monomer solution contains 0.05% to 0.2%, more preferably about 0.1 % NaOH. When used (for instance for determining the binding affinities of the antibodies of the present invention), it may be expedient to dilute said solution in an appropriate manner. Further, it is usually expedient to use said solution within 2 hours, in particular within 1 hour, and especially within 30 minutes after its preparation.

The term "fibril" here refers to a molecular structure that comprises assemblies of non-covalently associated, individual Aβ(X-Y) peptides, which show fibrillary structure in the electron microscope, which bind Congo red and then exhibit birefringence under polarized light and whose X-ray diffraction pattern is a cross-β structure.

In another aspect of the invention, a fibril is a molecular structure obtainable by a process that comprises the self-induced polymeric aggregation of a suitable Aβ peptide in the absence of detergents, e. g. in 0.1 M HCl, leading to the formation of aggregates of more than 24, preferably more than 100 units. This process is well known in the art. Expediently, Aβ(X-Y) fibrils are used in the form of an aqueous solution. In a particularly preferred embodiment of the invention, the aqueous fibril solution is made by dissolving the Aβ peptide in 0.1 % NH₄OH, diluting it 1 : 4 with 20 mM NaH₂PO₄, 140 mM NaCl, pH 7.4, followed by readjusting the pH to 7.4, incubating the solution at 37 °C for 20 h, followed by centrifugation at 10000 g for 10 min and resuspension in 20 mM NaH₂PO₄, 140 mM NaCl, pH 7.4.

The present invention further relates to the following teaching.

The results obtained for the soluble globular Aβ(1 - 42) oligomers are also valid and applicable for the respective soluble globular Aβ(1 - 40) oligomers as well as the respective truncated Aβ(X - 38), Aβ(X - 39), Aβ(X - 40), Aβ(X - 41), Aβ(X - 42) or Aβ(X - 43), preferably Aβ(X - 42) or Aβ(X - 40) oligomers, and/or the respective cross-linked oligomers thereof. Thus according to the present invention, the term "soluble globular Aβ(1 - 42) oligomer ("globulomers")" is understood to comprise for example also "soluble globular Aβ(1 - 40) oligomers" as well as "soluble globular Aβ(X - 42) oligomers" and "soluble globular Aβ(X - 40) oligomers" and/or a cross-linked derivative thereof with "x being independently of its occurrence selected from the group consisting of the numbers 2 .. 24, preferably 8 .. 24, more preferably 12 .. 24, more preferably 12 .. 20" and most preferably 17 .. 20" where the meaning of the ellipsis is as described above (i.e. 1 .. 24 stands for 1, 2, 3, 4, 5, 6, 7, 8, 9, 10, 11, 12, 13, 14, 15, 16, 17, 18, 19, 20, 21, 22, 23 and 24, and so forth).

Further, it was found that the results according to the present invention with regard to the non-diffusible water-soluble globular Aβ(1 - 42) oligomer ("globulomer") are also valid and applicable with regard to the respective Aβ(X - 42)- and/or Aβ(X - 40) oligomers, with X being independently of its occurrence selected from the group consisting of the numbers 1 .. 24, preferably 8 .. 24, more preferably 12 .. 24, more preferably 12 .. 20 and most preferably 17 .. 20, as well as a cross-linked derivative thereof.

In the course of further investigating the above screening method, the inventors also investigated chemical preparations as described in the literature. Surprisingly, the inventors of the present invention found that the oligomers according to WO 98/33815 (and similar publications) are not a homogenous identifiable substance or homogeneous mixture of identifiable substances. Unexpectedly, the inventors found that the oligomers according to WO 98/33815 (and similar publications) comprise in fact a mixture of about 5 % of a non-diffusible soluble globular Aβ(1 - 42) oligomer epitopes ("globulomer epitopes") and of about 95 % fibril prone monomer including diffusible oligomers as protofibril precursors ("fibrillomers") on the pathway to fibrils.

Further, surprisingly, it has also been found that other known Aβ(1 - 42) oligomer preparations also comprise an amount of about 5 % of a non-diffusible soluble globular Aβ(1 - 42) oligomer epitopes ("globulomer epitopes").

Calculations suggest that the Aβ(1 - 42) globulomer consists of 12 or about 12 Aβ subunits but it is also probable and suggested that assemblies exist having the same characteristic change in its epitope structure but with a lower number of Aβ chains (e.g. with only 4 subunits). Typically this is the case with "oligomers A" as described in WO 2004/067561 (Abbott GmbH & Co. KG) (see Fig. 18).

According to one aspect of the present invention, a method for qualitatively (= positively or negatively) or quantitatively in vitro or in vivo diagnosing in a human or animal patient for the presence or absence of Alzheimer's disease (AD) or the patient's risk of getting AD, characterized by the step of determining in one or more representative samples taken from the patient the presence (= positive diagnosis) or absence (= negative diagnosis) or the quantitative amount of auto-antibodies having specificity against the non-diffusible globular Aβ(X - 38/43) oligomer, preferably Aβ(X - 42) and/or Aβ(X - 40), and/or a cross-linked derivative thereof with X being independently of its occurrence selected from the group consisting of the numbers 1 .. 24, preferably 8 .. 24, more preferably 12 .. 24, more preferably 12 .. 20 and most preferably 17 .. 20, is provided.

According to a preferred embodiment of the invention, the method as described above is **characterized in that** the sample is selected from the group consisting of a cell, a cell assembly, a body tissue (cell) sample, a body liquid sample and a body tissue (cell)/liquid sample, each said sample being from an animal body, preferably a human, non-human animal or non-human transgenic animal body.

According to another preferred embodiment of the invention, the method as described above is **characterized in that** the mono- or polyclonal antibody is selective for a globulomer structure type specific epitope located between the amino acid positions of from about 20 to about 30 of the non-diffusible soluble globular Aβ(X - 42) and/or Aβ(X - 40) oligomer ("globulomer") and/or a cross-linked derivative thereof, with X being independently of its occurrence selected from the group consisting of the numbers 1 .. 24, preferably 8 .. 24, more preferably 12 .. 24, more preferably 12 .. 20 and most preferably 17 .. 20.

The word "about" when used above or elsewhere in the present specification and claims is used to signify the fact that the exact position may vary within normal experimental and manufacturing limits of from 0 to 1, 2, or 3 amino acid positions.

The term "amino acid positions of from about 20 to about 30" is to be understood in the present invention and wherever used in the present specification to include without being limited thereto at least the terms "amino acid positions of from about 20 to about 30", "amino acid positions of from about 21 to about 30", "amino acid positions of from about 22 to about 30", "amino acid positions of from about 23 to about 30", and "amino acid positions of from about 24 to about 30". The term "about 30" shall independently of the meaning of the term "about 20" be understood to include without being limited thereto at least the numbers selected from the group consisting of 26 .. 34. The term "about 20" shall independently of the meaning of the term "about 30" be understood to include without being limited thereto at least the numbers selected from the group consisting of 16 .. 24.

According to a preferred embodiment, the method as described above is **characterized in that** the sample is selected from the group consisting of a cell, a cell assembly, a body tissue (cell) sample, a body liquid sample and a body tissue (cell)/liquid sample, each said sample being from an animal body, preferably a human, non-human animal or non-human transgenic animal body.

According to a preferred embodiment, the method as described above is **characterized in that** the mono- or polyclonal antibody is selective for a globulomer structure type specific epitope located between the amino acid positions of from about 20 to about 30 of the non-diffusible globular Aβ(X - 38/43) oligomer, preferably Aβ(X - 42)- and/or Aβ(X - 40) oligomer ("globulomer"), and/or a cross-linked derivative thereof, with X being independently of its occurrence selected from the group consisting of the numbers 1 .. 24, preferably 8 .. 24, more preferably 12 .. 24, more preferably 12 .. 20 and most preferably 17 .. 20.

According to a preferred embodiment, the method as described above is **characterized in that** the non-diffusible globular Aβ(X - 38/43) oligomer, preferably Aβ(X - 42)- and/or Aβ(X - 40) oligomer ("globulomer") is soluble.

According to a further aspect of the present invention, an enriching process, characterized by comprising the step of selectively and reversibly capturing the non-diffusible globular Aβ(X - 38/43) oligomer, preferably Aβ(X - 42) and/or Aβ(X - 40) oligomer ("globulomers"), with X being independently of its occurrence selected from the group consisting of the numbers 1 .. 24, preferably 8 .. 24, more preferably 12 .. 24, more preferably 12 .. 20 and most preferably 17 .. 20, and/or the cross-linked derivative thereof from the preparation by using one or more per se known peptide purification process steps, is provided.

According to a further aspect of the present invention, a process for determining the amount of at least one non-diffusible globular Aβ(X - 38/43) oligomer, preferably Aβ(X - 42) and/or Aβ(X - 40) oligomer ("globulomer"), and/or a cross-linked derivative thereof, with X being independently of its occurrence selected from the group consisting of the numbers 1 .. 24, preferably 8 .. 24, more preferably 12 .. 24, more preferably 12 .. 20 and most preferably 17 .. 20, and/or a cross-linked derivative thereof in an artificial or natural preparation, characterized by the step of contacting the preparation, or a representative part thereof, with a specific mono- or polyclonal antibody having specificity against at least one non-diffusible globular Aβ(X - 38/43) oligomer, preferably Aβ(X - 42) and/or Aβ(X - 40) oligomer, and/or a cross-linked derivative thereof and then determining the amount of the resulting antibody-globulomer binding products, is provided.

According to a preferred embodiment of the present invention, said process is a dot blot and ELISA anti-Aβ immunoassay as described below or in WO 2004/067561.

According to a preferred embodiment of the present invention, the process as described above is **characterized in that** the mono- or polyclonal antibody is specific for a globulomer structure type specific epitope located at or between the amino acid positions of from about 20 to about 30 of the non-diffusible soluble globular Aβ(X - 38/43) oligomer, preferably Aβ(X - 42) and/or Aβ(X - 40) oligomer ("globulomer") and/or a cross-linked derivative thereof.

According to a further preferred embodiment of the present invention, the process as described above is characterized by enriching the amount of non-diffusible globular Aβ(X - 38/43) oligomer, preferably Aβ(X - 42)- and/or Aβ(X - 40) oligomers, with X being independently of its occurrence selected from the group consisting of the numbers 1 .. 24, preferably 8 .. 24, more preferably 12 .. 24, more preferably 12 .. 20 and most preferably 17 .. 20, and/or a cross-linked derivative thereof as contained in the preparation by using one or more per se known peptide purification process steps.

According to a further preferred embodiment of the present invention, the enriching process as described above is characterized by an enrichment of the amount of non-diffusible globular Aβ(X - 38/43) oligomer, preferably Aβ(X - 42) and/or Aβ(X - 40) oligomers, with X being independently of its occurrence selected from the group consisting of the numbers 1 .. 24, preferably 8 .. 24, more preferably 12 .. 24, more preferably 12 .. 20 and most preferably 17 .. 20, and/or a cross-linked derivative thereof of equal to or higher than 50 % by weight, preferably of equal to or higher than 60 % by weight, more preferably of equal to or higher than 70 % by weight, more preferably of equal to or higher than 80 % by weight, more preferably of equal to or higher than 90 % by weight, more preferably of equal to or higher than 95 % by weight, more preferably of equal to or higher than 99 % by weight., more preferably of equal to or higher than 99,9 % by weight, more preferably of equal to or higher than 99,99 % by weight.

According to a further preferred embodiment of the present invention, the enriching process as described above is characterized by comprising the step of selectively and reversibly capturing the non-diffusible globular Aβ(X - 38/43) oligomer, preferably Aβ(X - 42) and/or Aβ(X - 40) oligomer ("globulomers"), with X being independently of its occurrence selected from the group consisting of the numbers 1 .. 24, preferably 8 .. 24, more preferably 12 .. 24, more preferably 12 .. 20 and most preferably 17 .. 20, and/or the cross-linked derivative thereof from the preparation by using one or more per se known peptide purification process steps.

According to a further preferred embodiment of the present invention, the enriching process as described above is characterized by comprising the step of reversibly or irreversibly removing those substances being other than non-diffusible globular Aβ(X - 38/43) oligomer, preferably Aβ(X - 42) and/or Aβ(X - 40) oligomer ("globulomers") and/or the cross-linked derivative thereof from the preparation by using one or more per se known peptide purification process steps.

According to a further preferred embodiment of the present invention, the enriching process as described above is **characterized in that** the step of determining the amount of non-diffusible globular Aβ(X - 38/43) oligomer, preferably Aβ(X - 42) and/or Aβ(X - 40) oligomer ("globulomers") and/or the cross-linked derivative thereof is only performed after having performed one or more of the enrichment steps.

According to a further preferred embodiment of the present invention, the determining and/or enriching process as described above is **characterized in that** the obtained non-diffusible globular Aβ(X - 38/43) oligomer, preferably Aβ(X - 42)- and/or Aβ(X - 40) oligomer ("globulomers") is cross-linked by using one or more per se known cross-linking steps which can be performed either before, during and/or after the enriching and/or determining steps.

The cross-linking can be performed, for example, by treatment with glutardialdehyde. Suitable cross-linking methods are described in WO 2004/067561. The cross-linking step can be performed before, during and/or after the enriching procedure.

According to a further preferred embodiment of the present invention, the determining and/or enriching process as described above is **characterized in that** the non-diffusible globular Aβ(X - 38/43) oligomer, preferably Aβ(X - 42)- and/or Aβ(X - 40) oligomer ("globulomer") is soluble.

According to a further aspect of the present invention, a purified or essentially purified or highly purified non-diffusible globular Aβ(X - 38/43) oligomer, preferably Aβ(X - 42) and/or Aβ(X - 40) oligomer ("globulomer"), with X being independently of its occurrence selected from the group consisting of the numbers 1 .. 24, preferably 8 .. 24, more preferably 12 .. 24, more preferably 12 .. 20 and most preferably 17 .. 20, and/or a cross-linked derivative thereof, as described above, obtained or obtainable by a method as described above, is provided.

According to a further aspect of the present invention, a purified or essentially purified or highly purified non-diffusible globular Aβ(X - 38/43) oligomer, preferably Aβ(X - 42) and/or Aβ(X - 40) oligomer ("globulomer"), with X being independently of its occurrence selected from the group consisting of the numbers 1 .. 24, preferably 8 .. 24, more preferably 12 .. 24, more preferably 12 .. 20 and most preferably 17 .. 20, and/or a cross-linked derivative thereof, as described above, obtained or obtainable by a method as described above, is provided.

According to a further preferred embodiment of the present invention, the purified or essentially purified or highly purified non-diffusible globular Aβ(X - 38/43) oligomer, preferably Aβ(X - 42) and/or Aβ(X - 40) oligomer ("globulomer") as described above is **characterized in that** the non-diffusible globular Aβ(X - 38/43) oligomer, preferably Aβ(X - 42)- and/or Aβ(X - 40) oligomer ("globulomer") is soluble.

According to a further aspect of the present invention, a composition comprising a purified or essentially purified or highly purified non-diffusible globular Aβ(X - 38/43) oligomer, preferably Aβ(X - 42) or Aβ(X - 40) oligomer ("globulomer"), with X being independently of its occurrence selected from the group consisting of the numbers 1 .. 24, preferably 8 .. 24, more preferably 12 .. 24, more preferably 12 .. 20 and most preferably 17 .. 20, and/or a cross-linked derivative thereof as described above, obtained or obtainable by a method as described above, is provided.

According to a further preferred embodiment of the present invention, the composition as described above is **characterized in that** the non-diffusible globular Aβ(X - 38/43) oligomer, preferably Aβ(X - 42)- and/or Aβ(X - 40) oligomer ("globulomer") is soluble.

According to a further aspect of the present invention, the use of a purified or essentially purified or highly purified non-diffusible globular Aβ(X - 38/43) oligomer, preferably Aβ(X - 42) and/or Aβ(X - 40) oligomer ("globulomers"), with X being independently of its occurrence selected from the group consisting of the numbers 1 .. 24, preferably 8 .. 24, more preferably 12 .. 24, more preferably 12 .. 20 and most preferably 17 .. 20, and/or a cross-linked derivative thereof as obtained or obtainable by a method as described above use as a vaccine for the active immunization against Alzheimer's Disease (AD) of a patient in need thereof, is provided.

According to a further aspect of the present invention, the use of a purified or essentially purified or highly purified non-diffusible globular Aβ(X - 38/43) oligomer, preferably Aβ(X - 42) and/or Aβ(X - 40) oligomer ("globulomers"), with X being independently of its occurrence selected from the group consisting of the numbers 1 .. 24, preferably 8 .. 24, more preferably 12 .. 24, more preferably 12 .. 20 and most preferably 17 .. 20, and/or a cross-linked derivative thereof as obtained or obtainable by a method as described above for the preparation of a specific aptamer against Alzheimer's Disease (AD) of a patient in need thereof, is provided.

Methods for preparation of suitable specific aptamers against the above-identified purified or essentially purified or highly purified non-diffusible globular Aβ(X - 38/43) oligomer, preferably Aβ(X - 42) and/or Aβ(X - 40) oligomer ("globulomers") are known to the skilled person in the art. See for example the published German Patent Application No. DE 199 16 417 A1.

The term "active" or "passive" immunization shall mean in terms of patient's disease treatment that the occurrence or development or progress of Alzheimer's disease (AD) is prevented or prolonged or the AD pathogenesis related to the extent of neuronal loss and severity of cognitive impairment is stopped or slowed down.

According to a further aspect of the present invention, the use of a purified or essentially purified or highly purified non-diffusible globular Aβ(X - 38/43) oligomer, preferably Aβ(X - 42) and/or Aβ(X - 40) oligomer ("globulomer"), with X being independently of its occurrence selected from the group consisting of the numbers 1 .. 24, preferably 8 .. 24, more preferably 12 .. 24, more preferably 12 .. 20 and most preferably 17 .. 20, and/or a cross-linked derivative thereof as obtained or obtainable by a method as described above for the preparation of a medicament for the passive immunization against Alzheimer's Disease (AD) of a patient in need thereof, is provided.

According to a further aspect of the present invention, the use of a purified or essentially purified or highly purified non-diffusible globular Aβ(X - 38/43) oligomer, preferably Aβ(X - 42) and/or Aβ(X - 40) oligomer ("globulomers"), with X being independently of its occurrence selected from the group consisting of the numbers 1 .. 24, preferably 8 .. 24, more preferably 12 .. 24, more preferably 12 .. 20 and most preferably 17 .. 20, and/or a cross-linked derivative thereof as obtained or obtainable by a method as described above for the preparation of a monoclonal or polyclonal, native or recombinant human or animal or chimeric antibody having specificity against the non-diffusible globular Aβ(X - 42) and/or Aβ(X - 40) oligomer ("globulomers") and/or the cross-linked derivative thereof, being useful for the passive immunization against Alzheimer's disease in a patient in need thereof, is provided.

According to a further preferred embodiment of the present invention, any of the uses as described above is **characterized in that** the non-diffusible globular Aβ(X - 38/43) oligomer, preferably Aβ(X - 42)- and/or Aβ(X - 40) oligomer ("globulomer") is soluble.

According to a further aspect of the present invention, a method for the preparation of a monoclonal or polyclonal, native or recombinant human, humanized, animal or chimeric antibody having specificity against the non-diffusible globular Aβ(X - 38/43) oligomer, preferably Aβ(X - 42) and/or Aβ(X - 40) oligomers ("globulomers"), with X being independently of its occurrence selected from the group consisting of the numbers 1 .. 24, preferably 8 .. 24, more preferably 12 .. 24, more preferably 12 .. 20 and most preferably 17 .. 20, and/or a cross-linked derivative thereof characterized by the step of bringing an organism having an immune system into contact with a purified or essentially purified or highly purified non-diffusible globular Aβ(X - 42) and/or Aβ(X - 40) oligomer ("globulomers") and/or a cross-linked derivative thereof as obtained or obtainable by a method as described above and isolating the antibody having specificity against the non-diffusible globular Aβ(X - 42) and/or Aβ(X - 40) oligomer ("globulomers") and/or a cross-linked derivative thereof or a cell producing an antibody having specificity against the non-diffusible globular Aβ(X - 42) and/or Aβ(X - 40) oligomer ("globulomers") and/or a cross-linked derivative from the organism and optionally determining the chemical sequence and/or structure of the obtained antibody, is provided.

According to a further preferred embodiment of the present invention, the method as described above is **characterized in that** the non-diffusible globular Aβ(X - 38/43) oligomer, preferably Aβ(X - 42)- and/or Aβ(X - 40) oligomer ("globulomer") is soluble.

According to a further aspect of the present invention, a monoclonal or polyclonal, native or recombinant, humanized, human, animal or chimeric antibody having specificity against the non-diffusible globular Aβ(X - 38/43) oligomer, preferably Aβ(X - 42) and/or Aβ(X - 40) oligomers ("globulomers"), with X being independently of its occurrence selected from the group consisting of the numbers 1 .. 24, preferably 8 .. 24, more preferably 12 .. 24, more preferably 12 .. 20 and most preferably 17 .. 20, and/or a cross-linked derivative thereof as obtained or obtainable according to the method described above for active or passive immunization against Alzheimer's disease (AD) in a patient in need thereof, is provided.

According to a further preferred embodiment of the present invention, the monoclonal or polyclonal, native or recombinant, humanized, human, animal or chimeric antibody as described above is **characterized in that** the non-diffusible globular Aβ(X - 38/43) oligomer, preferably Aβ(X - 42)- and/or Aβ(X - 40) oligomer ("globulomer") is soluble.

According to a further aspect of the present invention, a composition comprising a monoclonal or polyclonal, native or recombinant, human, humanized, animal or chimeric antibody having specificity against the non-diffusible globular Aβ(X - 38/43) oligomer, preferably Aβ(X - 42) and/or Aβ(X - 40) oligomers ("globulomers"), with X being independently of its occurrence selected from the group consisting of the numbers 1 .. 24, preferably 8 .. 24, more preferably 12 .. 24, more preferably 12 .. 20 and most preferably 17 .. 20, and/or a cross-linked derivative thereof as obtained or obtainable according to the method described above for active or passive immunization against Alzheimer's disease (AD) in a patient in need thereof, is provided.

According to a further preferred embodiment of the present invention, the aforesaid composition, **characterized in that** the non-diffusible globular Aβ(X - 38/43) oligomer, preferably Aβ(X - 42)- and/or Aβ(X - 40) oligomer ("globulomer") is soluble.

According to a further aspect of the present invention, a method for positively or negatively diagnosing a patient for having Alzheimer's Disease (AD) or having an increased risk for getting Alzheimer's Disease, characterized by the step of determining in one or more representative samples taken from the patient the presence (positive diagnosis) or absence (negative diagnosis) of auto-antibodies against non-diffusible globular Aβ(X - 38/43) oligomer, preferably Aβ(X - 42) and/or Aβ(X - 40) oligomers ("globulomers"), with X being independently of its occurrence selected from the group consisting of the numbers 1 .. 24, preferably 8 .. 24, more preferably 12 .. 24, more preferably 12 .. 20 and most preferably 17 .. 20, and/or a cross-linked derivative thereof, is provided.

According to a preferred embodiment of the present invention, the method as described above is **characterized in that** the sample is selected from the group consisting of a cell, a cell assembly, a body tissue (cell) sample, a body liquid sample and a body tissue (cell)/liquid sample, each said sample being from a human, animal or transgenic animal body.

According to a preferred embodiment of the present invention, the method as described above is **characterized in that** the non-diffusible globular Aβ(X - 38/43) oligomer, preferably Aβ(X - 42)- and/or Aβ(X - 40) oligomer ("globulomer"), is soluble.

According to a further aspect of the present invention, the use of a humanized, human, animal or chimeric antibodies against a non-diffusible globular Aβ(X - 38/43) oligomer, preferably Aβ(X - 42) and/or Aβ(X - 40) oligomer ("globulomers"), with X being independently of its occurrence selected from the group consisting of the numbers 1 .. 24, preferably 8 .. 24, more preferably 12 .. 24, more preferably 12 .. 20 and most preferably 17 .. 20, and/or an cross-linked derivative thereof as a diagnostic marker for determining Alzheimer's disease (AD) or an increased risk of getting Alzheimer's disease in a patient in need thereof, is provided.

According to a preferred embodiment of the present invention, the use of a humanized, human, animal or chimeric antibodies against a non-diffusible globular Aβ(X - 38/43) oligomer, preferably Aβ(X - 42) and/or Aβ(X - 40) oligomer ("globulomers") is characterized in that the non-diffusible globular Aβ(X - 38/43) oligomer, preferably Aβ(X - 42)-and/or Aβ(X - 40) oligomer ("globulomer") is soluble.

According to a further aspect of the present invention, the use of one or more drugs that lower the fatty acid level in the brain of a patient for the preparation of a medicament for the treatment or prevention of Alzheimer's disease or related diseases like Down's syndrome in a patient in need thereof, is provided.

According to a further aspect of the present invention, a non-diffusible globular Aβ(X-Y) oligomer, with X being independently of its occurrence selected from the group consisting of the numbers 1 .. 24, preferably 8 .. 24, more preferably 12 .. 24, more preferably 12 .. 20 and most preferably 17 .. 20, and Y being independently of its occurrence selected from the group consisting of the numbers 38, 39, 41 and 43, and/or an cross-linked derivative thereof useful as a diagnostic marker for determining Alzheimer's disease (AD) or an increased risk of getting Alzheimer's disease in a patient in need thereof, is provided.

With regard to the present invention including the specification, claims and figures, the term "Aβ(1 - 42) oligomer" is understood to include Aβ(X - 42) oligomers, Aβ(X - 40) oligomers and/or the cross-linked derivatives thereof, with X being independently of its occurrence selected from the group consisting of the numbers 1 .. 24, preferably 8 .. 24, more preferably 12 .. 24, more preferably 12 .. 20 and most preferably 17 .. 20.

In particular throughout the present specification, any claims and any figures, the following definitions are applicable:

According to the present invention, the term "soluble" is defined with regard to the Aβ(1 - Y) globulomers as having a high or significant water-solubility (hydrophilicity) of up to about 15 mg/ ml or more" and with regard to the truncated Aβ(X - Y) globulomers as having low, moderate or high solubility in aqueous physiological buffer solutions in the presence of proteins (e.g. HAS) or detergents (e.g. pluriol, Pluronic F68), with X being independently of its occurrence selected from the group consisting of the numbers 1 .. 24, preferably 8 .. 24, more preferably 12 .. 24, more preferably 12 .. 20 and most preferably 17 .. 20, and Y being independently of its occurrence selected from the group consisting of the numbers 38 .. 43.

According to the present invention, the term "Aβ(X - 38/43) oligomer" is defined as comprising at least one Aβ oligomer selected from the group consisting of "Aβ(X - 38) oligomer", "Aβ(X - 39) oligomer", "Aβ(X - 40) oligomer", "Aβ(X - 41) oligomer", "Aβ(X - 42) oligomer" and "Aβ(X - 43) oligomer" and/or a cross-linked derivative thereof, with X being independently of its occurrence selected from the group consisting of the numbers 1 .. 24, preferably 8 .. 24, more preferably 12 .. 24, more preferably 12 .. 20 and most preferably 17 .. 20.

According to the present invention, the term "Aβ(X - 38) oligomer" is defined as comprising at least one Aβ(X - 38) oligomer and/or a cross-linked derivative thereof, with X being selected from the group consisting of the numbers 1 .. 24, preferably 8 .. 24, more preferably 12 .. 24, more preferably 12 .. 20 and most preferably 17 .. 20.

According to the present invention, the term "Aβ(X - 39) oligomer" is defined as comprising at least one Aβ(X - 39) oligomer and/or a cross-linked derivative thereof, with X being selected from the group consisting of the numbers 1 .. 24, preferably 8 .. 24, more preferably 12 .. 24, more preferably 12 .. 20 and most preferably 17 .. 20.

According to the present invention, the term "Aβ(X - 40) oligomer" is defined as comprising at least one Aβ(X - 40) oligomer and/or a cross-linked derivative thereof, with X being selected from the group consisting of the numbers 1 .. 24, preferably 8 .. 24, more preferably 12 .. 24, more preferably 12 .. 20 and most preferably 17 .. 20.

According to the present invention, the term "Aβ(X - 41) oligomer" is defined as comprising at least one Aβ(X - 41) oligomer and/or a cross-linked derivative thereof, with X being selected from the group consisting of the numbers 1 .. 24, preferably 8 .. 24, more preferably 12 .. 24, more preferably 12 .. 20 and most preferably 17 .. 20.

According to the present invention, the term "Aβ(X - 42) oligomer" is defined as comprising at least one Aβ(X - 42) oligomer and/or a cross-linked derivative thereof, with X being selected from the group consisting of the numbers 1 .. 24, preferably 8 .. 24, more preferably 12 .. 24, more preferably 12 .. 20 and most preferably 17 .. 20.

According to the present invention, the term "Aβ(X - 43) oligomer" is defined as comprising at least one Aβ(X - 43) oligomer and/or a cross-linked derivative thereof, with X being selected from the group consisting of the numbers 1 .. 24, preferably 8 .. 24, more preferably 12 .. 24, more preferably 12 .. 20 and most preferably 17 .. 20.

According to an additional aspect of the present invention, a method for qualitatively (= positively or negatively) or quantitatively in vitro or in vivo diagnosing in a human or non-human animal subject for the presence or absence of Alzheimer's disease (AD) or the risk of getting AD, characterized by the step of determining in one or more representative samples taken from said subject the presence (= positive diagnosis) or absence (= negative diagnosis) or the quantitative amount of auto-antibodies having specificity against at least one non-diffusible globular Aβ(X - 38/43) oligomer structure epitope, preferably at least one non-diffusible globular Aβ(X - 42) and/or Aβ(X - 40) oligomer structure epitope, and/or a cross-linked derivative thereof with X being independently of its occurrence selected from the group consisting of the numbers 1 .. 24, preferably 8 .. 24, more preferably 12 .. 24, more preferably 12 .. 20 and most preferably 17 .. 20, is provided.

According to a preferred embodiment of the present invention, the method as described above is **characterized in that** the sample is selected from the group consisting of a cell, a cell assembly, a body tissue (cell) sample, a body liquid sample and a body tissue (cell)/liquid sample, each said sample being derived from a human, non-human or transgenic animal body.

According to a preferred embodiment of the present invention, the method as described above is **characterized in that** the mono- or polyclonal antibody is selective for at least one globulomer structure type specific epitope located between the amino acid positions of from about 20 to about 30 of the non-diffusible globular Aβ(X - 38/43) oligomer, preferably of the non-diffusible globular Aβ(X - 42)- and/or Aβ(X - 40) oligomer ("globulomer"), and/or a cross-linked derivative thereof, with X being independently of its occurrence selected from the group consisting of the numbers 1 .. 24, preferably 8 .. 24, more preferably 12 .. 24, more preferably 12 .. 20 and most preferably 17 .. 20.

According to a preferred embodiment of the present invention, the method as described above is **characterized in that** the non-diffusible globular Aβ(X - 38/43) oligomer, preferably non-diffusible globular Aβ(X - 42)- and/or Aβ(X - 40) oligomer ("globulomer"), is soluble.

According to a further aspect of the present invention, a process for determining the amount of at least one non-diffusible globular Aβ(X - 38/43) oligomer, preferably Aβ(X - 42) and/or Aβ(X - 40) oligomer ("globulomer"), and/or a cross-linked derivative thereof, with X being independently of its occurrence selected from the group consisting of the numbers 1 .. 24, preferably 8 .. 24, more preferably 12 .. 24, more preferably 12 .. 20 and most preferably 17 .. 20, and/or a cross-linked derivative thereof in an artificial or natural preparation, characterized by the step of contacting the preparation, or a representative part thereof, with a specific mono- or polyclonal antibody having specificity against at least one non-diffusible globular Aβ(X - 38/43) oligomer structure epitope, preferably at least one non-diffusible globular Aβ(X - 42) and/or Aβ(X - 40) oligomer structure epitope, and/or a cross-linked derivative thereof and then determining the amount of the resulting antibody-globulomer binding products, is provided.

In particular, the process according to the last aspect, **characterized in that** the mono- or polyclonal antibody is specific for a globulomer structure type specific epitope located at or between the amino acid positions of from about 20 to about 30 of the non-diffusible globular Aβ(X - 38/43) oligomer, preferably non-diffusible globular Aβ(X - 42) and/or Aβ(X - 40) oligomer ("globulomer") and/or a cross-linked derivative thereof, is provided.

According to a preferred embodiment of the present invention, the process as described above is characterized by enriching the amount of non-diffusible globular Aβ(X - 38/43) oligomer, preferably non-diffusible globular Aβ(X - 42) and/or Aβ(X - 40) oligomer, with X being independently of its occurrence selected from the group consisting of the numbers 1 .. 24, preferably 8 .. 24, more preferably 12 .. 24, more preferably 12 .. 20 and most preferably 17 .. 20, and/or a cross-linked derivative thereof as contained in the preparation by using one or more per se known peptide purification process steps.

According to a preferred embodiment of the present invention, the enriching process as described above is characterized by an enrichment of the amount of non-diffusible globular Aβ(X - 38/43) oligomer, preferably non-diffusible globular Aβ(X - 42) and/or Aβ(X - 40) oligomer, with X being independently of its occurrence selected from the group consisting of the numbers 1 .. 24, preferably 8 .. 24, more preferably 12 .. 24, more preferably 12 .. 20 and most preferably 17 .. 20, and/or a cross-linked derivative thereof of equal to or higher than 50 % by weight, preferably of equal to or higher than 60 % by weight, more preferably of equal to or higher than 70 % by weight, more preferably of equal to or higher than 80 % by weight, more preferably of equal to or higher than 90 % by weight, more preferably of equal to or higher than 95 % by weight, more preferably of equal to or higher than 99 % by weight., more preferably of equal to or higher than 99,9 % by weight, more preferably of equal to or higher than 99,99 % by weight.

According to a preferred embodiment of the present invention, the enriching process as described above is characterized by its comprising the step of selectively and reversibly capturing the non-diffusible globular Aβ(X - 38/43) oligomer, preferably Aβ(X - 42) and/or Aβ(X - 40) oligomer ("globulomers"), with X being independently of its occurrence selected from the group consisting of the numbers 1 .. 24, preferably 8 .. 24, more preferably 12 .. 24, more preferably 12 .. 20 and most preferably 17 .. 20, and/or the cross-linked derivative thereof from the preparation by using one or more per se known peptide purification process steps.

According to a preferred embodiment of the present invention, the enriching process as described above is characterized by comprising the step of reversibly or irreversibly removing those substances being other than non-diffusible globular Aβ(X - 38/43) oligomer, preferably non-diffusible globular Aβ(X - 42) and/or Aβ(X - 40) oligomer ("globulomers") and/or the cross-linked derivative thereof from the preparation by using one or more per se known peptide purification process steps.

According to a preferred embodiment of the present invention, the enriching process as described above is **characterized in that** the step of determining the amount of non-diffusible globular Aβ(X - 38/43) oligomer, preferably non-diffusible globular Aβ(X - 42) and/or Aβ(X - 40) oligomer ("globulomers") and/or the cross-linked derivative thereof is only performed after having performed one or more of the enrichment steps.

According to a preferred embodiment of the present invention, the determining and/or enriching process as described above is **characterized in that** the obtained non-diffusible globular Aβ(X - 38/43) oligomer, preferably non-diffusible globular Aβ(X - 42)- and/or Aβ(X - 40) oligomer ("globulomers") is cross-linked by using one or more per se known cross-linking steps which can be performed either before, during and/or after the enriching and/or determining steps.

According to a preferred embodiment of the present invention, the determining and/or enriching process as described above is **characterized in that** the non-diffusible globular Aβ(X - 38/43) oligomer, preferably the non-diffusible globular Aβ(X - 42)- and/or Aβ(X - 40) oligomer ("globulomer"), is soluble.

According to a further aspect of the present invention, a purified or essentially purified or highly purified non-diffusible globular Aβ(X - 38/43) oligomer, preferably non-diffusible globular Aβ(X - 42) and/or Aβ(X - 40) oligomer ("globulomer"), with X being independently of its occurrence selected from the group consisting of the numbers 1 .. 24, preferably 8 .. 24, more preferably 12 .. 24, more preferably 12 .. 20 and most preferably 17 .. 20, and/or a cross-linked derivative thereof, according to as described above, obtained or obtainable by a method as described above, is provided.

According to a preferred embodiment of the present invention, the purified or essentially purified or highly purified non-diffusible globular Aβ(X - 38/43) oligomer, preferably non-diffusible globular Aβ(X - 42) and/or Aβ(X - 40) oligomer ("globulomer") as described above is **characterized in that** the non-diffusible globular Aβ(X - 38/43) oligomer, preferably non-diffusible globular Aβ(X - 42)- and/or Aβ(X - 40) oligomer ("globulomer"), is soluble.

According to a further aspect of the present invention, a composition comprising a purified or essentially purified or highly purified non-diffusible globular Aβ(X - 38/43) oligomer, preferably non-diffusible globular Aβ(X - 42) or Aβ(X - 40) oligomer ("globulomer"), with X being independently of its occurrence selected from the group consisting of the numbers 1 .. 24, preferably 8 .. 24, more preferably 12 .. 24, more preferably 12 .. 20 and most preferably 17 .. 20, and/or a cross-linked derivative thereof according to as described above, obtained or obtainable by a method as described above, is provided.

According to a preferred embodiment of the present invention, the composition as described above is **characterized in that** the non-diffusible globular Aβ(X - 38/43) oligomer, preferably the non-diffusible globular Aβ(X - 42)- and/or Aβ(X - 40) oligomer ("globulomer"), is soluble.

According to a further aspect of the present invention, the use of a purified or essentially purified or highly purified non-diffusible globular Av(X - 38/43) oligomer, preferably non-diffusible globular Aβ(X - 42) and/or Aβ(X - 40) oligomer ("globulomers"), with X being independently of its occurrence selected from the group consisting of the numbers 1 .. 24, preferably 8 .. 24, more preferably 12 .. 24, more preferably 12 .. 20 and most preferably 17 .. 20, and/or a cross-linked derivative thereof as obtained or obtainable by a method as described above for use as a vaccine for the active immunization against Alzheimer's Disease (AD) of a patient in need thereof, is provided.

According to a further aspect of the present invention, the use of a purified or essentially purified or highly purified non-diffusible globular Aβ(X - 38/43) oligomer, preferably non-diffusible globular Aβ(X - 42) and/or Aβ(X - 40) oligomer ("globulomers"), with X being independently of its occurrence selected from the group consisting of the numbers 1 .. 24, preferably 8 .. 24, more preferably 12 .. 24, more preferably 12 .. 20 and most preferably 17 .. 20, and/or a cross-linked derivative thereof as obtained or obtainable by a method as described above for the preparation of a specific aptamer against Alzheimer's Disease (AD) of a patient in need thereof, is provided.

According to a further aspect of the present invention, the use of a purified or essentially purified or highly purified non-diffusible globular Aβ(X - 38/43) oligomer, preferably non-diffusible globular Aβ(X - 42) and/or Aβ(X - 40) oligomer ("globulomer"), with X being independently of its occurrence selected from the group consisting of the numbers 1 .. 24, preferably 8 .. 24, more preferably 12 .. 24, more preferably 12 .. 20 and most preferably 17 .. 20, and/or a cross-linked derivative thereof as obtained or obtainable by a method as described above for the preparation of a medicament for the passive immunization against Alzheimer's Disease (AD) of a patient in need thereof, is provided.

According to a further aspect of the present invention, the use of a purified or essentially purified or highly purified non-diffusible globular Aβ(X - 38/43) oligomer, preferably non-diffusible globular Aβ(X - 42) and/or Aβ(X - 40) oligomer ("globulomers"), with X being independently of its occurrence selected from the group consisting of the numbers 1 .. 24, preferably 8 .. 24, more preferably 12 .. 24, more preferably 12 .. 20 and most preferably 17 .. 20, and/or a cross-linked derivative thereof as obtained or obtainable by a method as described above for the preparation of a monoclonal or polyclonal, native or recombinant human or animal or chimeric antibody having specificity against at least one non-diffusible globular Aβ(X - 38/43) oligomer structure epitope, preferably at least one non-diffusible globular Aβ(X - 42) and/or Aβ(X - 40) oligomer structure epitope ("globulomers") and/or the cross-linked derivative thereof, is provided.

According to a preferred embodiment of the present invention, the use as described above is **characterized in that** the non-diffusible globular Aβ(X - 38/43) oligomer, preferably non-diffusible globular Aβ(X - 42)- and/or Aβ(X - 40) oligomer ("globulomer"), is soluble.

According to a further aspect of the present invention, a method for preparation of a monoclonal or polyclonal, native or recombinant human, humanized, animal or chimeric antibody having specificity against at least one non-diffusible globular Aβ(X - 38/43) oligomer structure epitope, preferably at least one non-diffusible globular Aβ(X - 42) and/or Aβ(X - 40) oligomer structure epitope ("globulomers"), with X being independently of its occurrence selected from the group consisting of the numbers 1 .. 24, preferably 8 .. 24, more preferably 12 .. 24, more preferably 12 .. 20 and most preferably 17 .. 20, and/or a cross-linked derivative thereof characterized by the steps of (i) bringing an organism having an immune system into contact with a purified or essentially purified or highly purified non-diffusible globular Aβ(X - 38/43) oligomer, preferably non-diffusible globular Aβ(X - 42) and/or Aβ(X - 40) oligomer ("globulomers") and/or a cross-linked derivative thereof as obtained or obtainable by a method described above and (ii) isolating the antibody having specificity against at least one non-diffusible globular Aβ(X - 38/43) oligomer structure epitope, preferably against at least one non-diffusible globular Aβ(X - 42) and/or Aβ(X - 40) oligomer structure epitope ("globulomers") and/or a cross-linked derivative thereof from the organism and (iii) optionally determining the chemical sequence and/or structure of the obtained antibody or, alternatively, characterized by the step of isolating the antibody having specificity against at least one non-diffusible globular Aβ(X - 38/43) oligomer structure epitope, preferably against at least one non-diffusible globular Aβ(X - 42) and/or Aβ(X - 40) oligomer structure epitope ("globulomers") and/or a cross-linked derivative thereof from a cell or cell line producing an antibody having specificity against at least one non-diffusible globular Aβ(X - 38/43) oligomer structure epitope, preferably against at least one non-diffusible globular Aβ(X - 42) and/or Aβ(X - 40) oligomer structure epitope ("globulomers") and/or a cross-linked derivative thereof, is provided.

According to a preferred embodiment of the present invention, the method as described above is **characterized in that** the non-diffusible globular Aβ(X - 38/43) oligomer, preferably non-diffusible globular Aβ(X - 42)- and/or Aβ(X - 40) oligomer ("globulomer"), is soluble.

According to a further aspect of the present invention, a monoclonal or polyclonal, native or recombinant, humanized, human, animal or chimeric antibody having specificity against at least one non-diffusible globular Aβ(X - 38/43) oligomer structure epitope, preferably at least one non-diffusible globular Aβ(X - 42) and/or Aβ(X - 40) oligomer structure epitope ("globulomers"), with X being independently of its occurrence selected from the group consisting of the numbers 1 .. 24, preferably 8 .. 24, more preferably 12 .. 24, more preferably 12 .. 20 and most preferably 17 .. 20, and/or a cross-linked derivative thereof as obtained or obtainable according to the method as described above for active or passive immunization against Alzheimer's disease (AD) in a patient in need thereof, is provided.

According to a preferred embodiment of the present invention, the monoclonal or polyclonal, native or recombinant, humanized, human, animal or chimeric antibody as described above is **characterized in that** the non-diffusible globular Aβ(X - 38/43) oligomer, non-diffusible globular preferably Aβ(X - 42)- and/or Aβ(X - 40) oligomer ("globulomer"), is soluble.

According to a further aspect of the present invention, a composition comprising a monoclonal or polyclonal, native or recombinant, human, humanized, animal or chimeric antibody having specificity against at least one non-diffusible globular Aβ(X - 38/43) oligomer structure epitope, preferably at least one non-diffusible globular Aβ(X - 42) and/or Aβ(X - 40) oligomer structure epitope ("globulomers"), with X being independently of its occurrence selected from the group consisting of the numbers 1 .. 24, preferably 8 .. 24, more preferably 12 .. 24, more preferably 12 .. 20 and most preferably 17 .. 20, and/or a cross-linked derivative thereof as obtained or obtainable as described above for active or passive immunization against Alzheimer's disease (AD) in a patient in need thereof.

According to a preferred embodiment of the present invention, the composition as described above is **characterized in that** the non-diffusible globular Aβ(X - 38/43) oligomer, preferably non-diffusible globular Aβ(X - 42)- and/or Aβ(X - 40) oligomer ("globulomer"), is soluble.

According to a further aspect of the present invention, a method for positively or negatively diagnosing a human or non-human animal subject for having Alzheimer's Disease (AD) or having an increased risk for getting Alzheimer's Disease, characterized by the step of determining in one or more representative samples taken from the patient the presence (positive diagnosis) or absence (negative diagnosis) of auto-antibodies having specificity against at least one non-diffusible globular Aβ(X - 38/43) oligomer structure epitope, preferably at least one non-diffusible globular Aβ(X - 42) and/or Aβ(X - 40) oligomer structure epitope ("globulomers"), with X being independently of its occurrence selected from the group consisting of the numbers 1 .. 24, preferably 8 .. 24, more preferably 12 .. 24, more preferably 12 .. 20 and most preferably 17 .. 20, and/or a cross-linked derivative thereof, is provided.

According to a preferred embodiment of the present invention, the method as described above is **characterized in that** the sample is selected from the group consisting of a cell, a cell assembly, a body tissue (cell) sample, a body liquid sample and a body tissue (cell)/liquid sample, each said sample being derived from a human, non-human or transgenic animal body.

According to a preferred embodiment of the present invention, the method as described above is **characterized in that** the non-diffusible globular Aβ(X - 38/43) oligomer, preferably non-diffusible globular Aβ(X - 42)- and/or Aβ(X - 40) oligomer ("globulomer"), is soluble.

According to a further aspect of the present invention, a use of a humanized, human, animal or chimeric antibody having specificity against at least non-diffusible globular Aβ(X - 38/43) oligomer structure epitope, preferably at least one non-diffusible globular Aβ(X - 42) and/or Aβ(X - 40) oligomer structure epitope ("globulomers"), with X being independently of its occurrence selected from the group consisting of the numbers 1 .. 24, preferably 8 .. 24, more preferably 12 .. 24, more preferably 12 .. 20 and most preferably 17 .. 20, and/or an cross-linked derivative thereof as a diagnostic marker for determining Alzheimer's disease (AD) or an increased risk of getting Alzheimer's disease in a patient in need thereof, is provided.

According to a preferred embodiment of the present invention, the use of a humanized, human, animal or chimeric antibody having specificity against at least one non-diffusible globular Aβ(X - 38/43) oligomer structure epitope, preferably at least one non-diffusible globular Aβ(X - 42) and/or Aβ(X - 40) oligomer structure epitope("globulomers") as described above is **characterized in that** the non-diffusible globular Aβ(X - 38/43) oligomer, preferably non-diffusible globular Aβ(X - 42)- and/or Aβ(X - 40) oligomer ("globulomer"), is soluble.

According to a further aspect of the present invention, the use of one or more drugs that lower the fatty acid level in the brain of a patient for the preparation of a medicament for the treatment or prevention of Alzheimer's disease or related diseases like Down's Syndrome in a patient in need thereof, is provided.

According to a further aspect of the present invention, a non-diffusible globular Aβ_{X - Y} oligomer, with X being independently of its occurrence selected from the group consisting of the numbers 1 .. 24, preferably 8 .. 24, more preferably 12 .. 24, more preferably 12 .. 20 and most preferably 17 .. 20, and Y being independently of its occurrence selected from the group consisting of the numbers 38 .. 43, and/or an cross-linked derivative thereof useful as a diagnostic marker for determining Alzheimer's disease (AD) or an increased risk of getting Alzheimer's disease in a patient in need thereof, is provided.

In connection with diagnostics uses, the present invention includes a method of diagnosing Alzheimer's disease in a patient suspected of having this disease. This method comprises the steps of: a) isolating a biological sample from the patient; b) contacting the biological sample with one of the antibodies described above, for a time and under conditions sufficient for formation of globulomer/antibody complexes; and c) detecting the presence of the globulomer/antibody complexes in the sample, presence of the complexes indicating a diagnosis of Alzheimer's disease in said patient.

An additional method of the present invention includes a method of diagnosing Alzheimer's disease in a patient suspected of having this disease comprising the steps of: a) isolating a biological sample from the patient; b) contacting the biological sample with a globulomer for a time and under conditions sufficient for the formation of antibody/globulomer complexes; c) adding a conjugate to the resulting antibody/globulomer complexes for a time and under conditions sufficient to allow the conjugate to bind to the bound antibody, wherein the conjugate comprises an antibody attached to a signal generating compound capable of generating a detectable signal; and d) detecting the presence of antibodies which may be present in the biological sample by detecting a signal generated by the signal generating compound, the signal indicating a diagnosis of Alzheimer's disease in the patient.

The present invention includes a further method of diagnosing Alzheimer's disease in a patient suspected of having Alzheimer's disease comprising the steps of: a) isolating a biological sample from the patient; b) contacting the biological sample with the globulomer described above for a time and under conditions sufficient for formation of antibody/purified globulomer complexes; and c) detecting presence of the antibody/purified globulomer complexes in the sample, presence of the complexes indicating a diagnosis of Alzheimer's disease in the patient.

Further, the present invention includes another method of diagnosing Alzheimer's disease in a patient suspected of having Alzheimer's disease comprising the steps of: a) isolating a biological sample from the patient;
contacting the biological sample with anti-antibody specific for antibodies in the sample for a time and under conditions sufficient to allow for formation of anti-antibody/antibody complexes; b) adding a conjugate to resulting anti-antibody/antibody complexes for a time and under conditions sufficient to allow the conjugate to bind to bound antibody, wherein the conjugate comprises a globulomer attached to a signal generating compound capable of generating a detectable signal; and c) detecting a signal generated by the signal generating compound, the signal indicating a diagnosis of Alzheimer's disease in the patient.

Additionally, the present invention includes a method of identifying compounds for treatment or prevention of Alzheimer's disease. This method comprises the steps of: a) exposing one or more compounds of interest to the purified globulomer described above for a time and under conditions sufficient for the one or more compounds to bind to or neutralize the purified globulomer; and b) identifying those compounds which bind to or neutralize the purified globulomer, the identified compounds to be used in the treatment or prevention of Alzheimer's disease.

Moreover, the present invention includes a method of designing a small molecule useful in the treatment or prevention of Alzheimer's disease in a patient. This method comprises the steps of: a) analyzing the three-dimensional structure of a protein such as the purified globulomer described above, the isolated beta-amyloid protein composition described above, or an assembly of the isolated beta-amyloid protein compositions; b) identifying one or more epitopes on the surface of the selected protein of step a); and c) designing a small molecule which will bind to the identified epitope or epitopes of step b), the small molecule to be used in the treatment or prevention of Alzheimer's disease.

The present invention also includes a method of identifying a monoclonal antibody to be used in the treatment or prevention of Alzheimer's disease. This method comprises the steps of: a) exposing the purified globulomer described above to a library of monoclonal antibodies for a time and under conditions sufficient for binding of one or more of the monoclonal antibodies to the globulomer and formation of globulomer/antibody complexes; b) identifying presence of the globulomer/antibody complexes; and c) determining the identity of one or more
antibodies within the complexes, the one or more antibodies to be used in the treatment or prevention of Alzheimer's disease.

Additionally, it should be noted that the globulomers of the present invention (as well as antibodies directed thereto), may be used in a variety of diagnostic assays.

In one embodiment of the present invention, the globulomer, or a portion thereof, is coated on a solid phase (or is present in a liquid phase). The test or biological sample (e.g., whole blood, cerebrospinal fluid, serum, etc.) is then contacted with the solid phase. If antibodies are present in the sample, such antibodies bind to the antigen on the solid phase and are then detected by either a direct or indirect method. The direct method comprises simply detecting presence of the complex itself and thus presence of the antibodies. In the indirect method, a conjugate is added to the bound antibody. The conjugate comprises a second antibody, which binds to the first bound antibody, attached to a signal-generating compound or label. Should the second antibody bind to a bound first antibody, the signal-generating compound generates a measurable signal. Such signal then indicates presence of the first antibody in the test sample.

Examples of solid phases used in diagnostic immunoassays are porous and nonporous materials, latex particles, magnetic particles, microparticles (see U.S. Patent No. 5,705,330), beads, membranes, microtiter wells and plastic tubes. The choice of solid phase material and method of labeling the antigen or antibody present in the conjugate, if desired, are determined based upon desired assay format performance characteristics.

As noted above, the conjugate (or indicator reagent) will comprise an antibody (or perhaps anti-antibody, depending upon the assay), attached to a signal-generating compound or label. This signal-generating compound or "label" is itself detectable or may be reacted with one or more additional compounds to generate a detectable product. Examples of signal-generating compounds include chromogens, radioisotopes (e.g., 125I, 131I, 32P, 3H, 35S and 14C), chemiluminescent compounds (e.g., acridinium), particles (visible or fluorescent), nucleic acids, complexing agents, or catalysts such as enzymes (e.g., alkaline phosphatase, acid phosphatase, horseradish peroxidase, beta-galactosidase and ribonuclease). In the case of enzyme use (e.g., alkaline phosphatase or horseradish peroxidase), addition of a chromo-, fluro-, or lumo-genic substrate results in generation of a detectable signal. Other detection systems such as time-resolved fluorescence, internal-reflection fluorescence, amplification (e.g., polymerase chain reaction) and Raman spectroscopy are also useful.

Examples of biological fluids which may be tested by the above immunoassays include plasma, whole blood, dried whole blood, serum, cerebrospinal fluid or aqueous or organo-aqueous extracts of tissues and cells.

In another embodiment of the present invention, the test sample may be exposed to a solid phase (or liquid phase) coated with specific antibodies of the present invention (e.g., human or humanized monoclonal antibodies, polyclonal antibodies, etc.). Globulomers as described above and, if present in the sample, bind to the solid phase and may then be detected by a direct or indirect method as described above. More specifically, the indirect method involves the addition of a conjugate comprising a second antibody (which binds to the bound antigen) attached to a label or signal-generating compound. When the second antibody binds to the bound antigen, a detectable signal is then generated indicating presence of an Alzheimer's protein such as a globulomer or portion thereof, in the test sample.

The present invention also encompasses a third method for detecting the presence of antibodies in a test sample. This method comprises the steps of: (a) contacting the test sample suspected of containing the antibodies with anti-antibody specific for the antigen (e.g., globulomer or a portion thereof), under time and conditions sufficient to allow the formation of anti-antibody/antigen complexes; (b) adding antigen to the resulting anti-antibody/antigen complexes for a time and under conditions sufficient to allow the antigen to bind to the bound antibody, the antigen comprising a globulomer or portion thereof, as defined herein; and (c) adding a conjugate to the resulting anti-antibody/antibody/antigen complexes, the conjugate comprising a composition comprising monoclonal or polyclonal antibody attached to a signal generating compound capable of detecting a detectable signal, the monoclonal or polyclonal antibody being directed against the antigen; and (d) detecting the presence of the antibodies which may be present in the test sample by detecting the signal generated by the signal generating compound. A control or calibrator may be used which comprises antibody to the anti-antibody. The antigen mixture may further comprise P30, if desired.

Kits are also included within the scope of the present invention. More specifically, the present invention includes kits for determining the presence of antibodies in a patient. In particular, a kit for determining the presence of antibodies in a test sample comprises a) an antigen as defined herein (e.g., globulomer or portion thereof; and b) a conjugate comprising an antibody attached to a signal generating compound capable of generating a detectable signal. The kit may also contain a control or calibrator which comprises a reagent which binds to the antigen.

The present invention also includes another type of kit for detecting antibodies in a test sample. The kit may comprise a) an anti-antibody specific for the antibody of interest, and b) an antigen or portion thereof as defined above. A control or calibrator comprising a reagent which binds to the antigen may also be included. More specifically, the kit may comprise a) an anti-antibody specific for the antibody and b) a conjugate comprising the antigen, the conjugate being attached to a signal generating compound capable of generating a detectable signal. Again, the kit may also comprise a control of calibrator comprising a reagent which binds to the antigen.

The kit may also comprise one container such as vial, bottles or strip, with each container with a pre-set solid phase, and other containers containing the respective conjugates. These kits may also contain vials or containers of other reagents needed for performing the assay, such as washing, processing and indicator reagents.

The invention will now be further illustrated by the subsequent examples, which are not to be construed as limiting in any respect.

### Examples

### Example 1: Preparation of fibril-prone monomers

1 mg Aβ(1 - 42) solid powder, chemically synthesized by Bachem (Bachem, Weil am Rhein, Germany), cat. no. H-1368, was dissolved in 0.5 ml 0.1 % NH₄OH in water (freshly prepared) and shaken for 30 seconds at room temperature to obtain a clear solution with a concentration of 2 mg/ ml.

The resulting Aβ(1 - 42) monomer preparation was diluted with 1.5 ml 20 mM NaH₂PO₄, 140 mM NaCl, pH 7.4 to a final protein concentration of 0.5 mg/ ml. The pH of this Aβ(1 - 42) fibril prone monomer preparation was adjusted with 5% hydrochloric acid to pH 7.4, and then the preparation was put on ice bath for immediate use.

All steps were performed quickly to avoid starting of polymerization of Aβ(1 - 42) during preparation.

Maximum stability of the monomers was found to be 1 h at 4 °C or 15 min at room temperature. Alternatively samples could be frozen immediately at -80 °C and stored at -80 °C for a maximum of 1 - 2 weeks. Even at this extremely low temperature, the Aβ protofibril and fibril polymerization process started, rendering the preparation unusable if stored for more than two weeks.

### Example 2: Stability of Aβ(X - 42) globulomers and fibril-prone monomers

A solution of Aβ(1 - 42) globulomers was prepared as described in example 5a of WO2004/067561. Briefly, Aβ(1 - 42) synthetic peptide (Cat. No. H-1368, Bachem, Switzerland) was suspended in 1,1,1,3,3,3-hexafluoro-2-propanol (HFIP) at - 6 mg/ ml and incubated for complete solubilization under shaking at 37 °C for 1.5 h. HFIP was removed by evaporation in a SpeedVac and Aβ(1 - 42) resuspended at a concentration of 5 mM in dimethyl sulfoxide (DMSO) and 20 sec sonicated. The HFIP pretreated Aβ(1 - 42) was diluted in phosphate buffered solution (PBS) (20 mM NaH₂PO₄, 140 mM NaCl, pH 7.4) to 400 µM and 1/10 volume 2% SDS (in H₂O) added (final concentration of 0.2% sodium dodecyl sulfate (SDS)). An incubation for 6 h at 37 °C resulted in the 16/20 kDa Aβ(1 - 42) globulomer intermediate. The 38/48 kDa Aβ(1 - 42) globulomer was generated by a further dilution with three volumes H₂O and incubation for 18 h at 37 °C. After centrifugation at 3,000 xg for 20 min the sample was concentrated by ultrafiltration (30kDa cut-off), dialyzed against 5 mM NaH₂PO₄, 35 mM NaCl, pH 7.4, centrifuged at 10,000 xg for 10 min and the supernatant containing the 38/48 kDa Aβ(1 - 42) globulomer withdrawn. For fatty acid induced 38/48 kDa Aβ(1 - 42) globulomer the SDS was replaced by the addition of 1/10 volume of 0.5% fatty acid. For cross-linking the 38/48 kDa Aβ(1 - 42) globulomer was treated before concentration and dialysis with 1 mM glutardialdehyde for 2 h at RT followed by ethanolamine (5 mM) treatment for 30 min at room temperature (RT). Limited proteolysis of the 38/48 kDa Aβ(1 - 42) globulomer was performed using 1/50 (mg/mg) of the respective protease and incubation for 20 h at RT, yielding the corresponding truncated form.

Concentration was assessed as follows: On the basis of its experimentally determined molecular weight, the Aβ(1 - 42) globulomer was approximated to consist of ∼12 monomers. The indicated concentrations stated are therefore approximations as well. For instance, an indicated Aβ(1 - 42) globulomer concentration of 42 nM would be equivalent to 500 nM Aβ(1 - 42) globulomer concentration based on the monomer.

The sample at 0.5 mg/ ml in 20 mM NaH₂PO₄; 140 mM NaCl; pH 7.4 was incubated at room temperature and 37 °C for 1 day and 4 days, respectively. An aliquot of 10 µl of the samples was analyzed by SDS-PAGE.

Aβ(1 - 42) (Bachem, peptide synthesis) was dissolved at 2 mg/ ml in 0.1 % NH₄OH and diluted 1:4 in water (0.5 mg/ ml), as described in Example 1. This Aβ(1 - 42) fibril prone preparation was incubated for 24 hours at each of the following temperatures: -20 °C; 0 °C; room temperature (corresponding to approx. 20 - 22 °C) and 37 °C. An aliquot of 10 µl of each of the samples was analyzed by SDS-PAGE.

Results are shown in Fig 2.

It was found that the Aβ(1 - 42) globulomers are stable for at least 4 days in PBS both at ambient temperature and at 37 °C, whereas Aβ(1 - 42) monomers exhibit marked polymerization to mega-Dalton aggregates even after 1 day at 0 °C, extensive polymerization after 1 day at ambient temperature and virtually complete polymerization after 1 day at 37 °C in PBS.

Example 3: Demonstration of the existence of Aβ(1 - 42) globulomer epitopes in vivo Using the specific antibodies of the invention, it was possible to detect Aβ(1 - 42) globulomer epitopes in amyloid plaques in the brains of Alzheimer's disease patients (see Fig. 13a-c) and of APP transgenic Tg2576 mice (see Fig. 13d-f).

Most of the immunofluorescence was associated with thioflavine S positive plaques (see Fig. 13a, d) where the Aβ(1 - 42) globulomer epitopes formed a dense rim around the plaques (see Fig. 13c, f). Quantification of the relative amount of Aβ(1 - 42) globulomer epitopes was possible for the PBS soluble fraction of brain extracts. In both, Alzheimer's disease patients (see Fig. 13g) and Tg2576 mice (see Fig. 13h), only small amounts of total Aβ turned out to be of Aβ(1 - 42) globulomer epitope structure. Aβ(1 - 42) globulomer epitopes were detected not only in plaques, but already in the brains of young, 2.5 month old Tg2576 mice which still have a low total Aβ(1 - 42) concentration. This is well before the development of amyloid plaques and strongly increased total Aβ(1 - 42) levels starting at an age of 11 months (see Fig. 13i), indicating that globulomer epitope formation is frequent even at comparatively low Aβ(1 - 42) concentrations. Surprisingly, no Aβ(1 - 42) globulomer epitopes were detected in the cerebrospinal fluid of patients with mild cognitive impairment (see Fig. 13j, gray bars) or with Alzheimer's disease (see Fig. 13j, black bars). However, this negative finding may be due to concentrations of Aβ(1 - 42) globulomer epitopes below the detection limit, as it is a distinguishing characteristic of globulomers that they bind to neural structures swiftly and easily and are thus expected to be quantitatively cleared from the cerebrospinal fluid thereby.

The detection was made using the following materials and methods.
3.1 Extraction and detection of Aβ globulomer epitopes in post mortem AD human brain tissue
3.1.A. Reagent List:
   - Extraction-buffer: 5 mM NaH₂PO₄ ; 35 mM NaCl; pH 7.4
   - Complete Protease Inhibitor Cocktail Tablets ; Roche Diagnostics GmbH Cat.No.: 697498 ; store at 4 °C.
   - Extraction-buffer + complete inhibitor cocktail:
      Dissolve 1 tablet complete inhibitor cocktail in 1 ml water.
      Add 1/100 complete inhibitor cocktail solution to the extraction-buffer.
3.1.B. Procedure for one-step extraction from human AD brain -80 °C frozen post mortem human AD and aged match control brain tissue samples were provided by Brain-Net, Munich. 9 ml extraction buffer and complete inhibitor cocktail were added to 1 g of frozen brain tissue material in a 50 ml falcon tube. The sample was homogenized on ice by an UltraTurrax homogenizer for 2 min and the homogenized sample filled in an ultracentrifugation tube and centrifuged at 8 °C at 150'000 g for 1 hour. The supernatant was carefully collected and stored in a 15 ml Falcon tube at -80 °C for further ELISA testing as described below.
3.1.C. Sandwich ELISA: as described in 3.2.B.
3.2 Extraction and detection of Aβ globulomer epitopes in brain tissue of Aβ overproducing Aβ transgenic mice TG 2576
3.2.A. Extraction procedure (reagents required as described in 3.1.A): Several 3 months old female Tg2576 mice which carry and overexpress the human APP gene with the Swedish double mutation (K670N; M671 L) of familial Alzheimer's disease were purchased from Taconics (USA) and kept in our animal care facility until an age of 12 months. 100 mg frozen cortex from 12 months old APP overexpressing TG2576 mice (Taconics Inc.) or 3 months old C57 wild type mice were rinsed with 2.5 ml PBST + 0.5% BSA-buffer + complete inhibitor cocktail (10 mg / 0.25 ml wet weight). Samples were pre-homogenized in a glass potter and sonicated in an ice bath at 0 °C for 5 times of 1-2 sec with an ultrasonic cell disruptor. 2 ml aliquots of samples were incubated in an ultra centrifugation tube for 16 hours at 37 °C overnight and centrifuged at 8 °C for 1 hour at 100'000 g. The supernatant was removed carefully and stored at -20 °C for ELISA analysis.
3.2.B. Sandwich-Elisa:
3.2.B.1. Reagent List:
1. F96 Cert. Maxisorp NUNC-Immuno Plate Cat.No.:439454
2. Capture antibody Anti-Aβ pAb rabbit 5598 affinity purified (purification procedure Labj.:1286/155) ; solution in PBS ; conc.: 0.24 mg/ ml ; stored at -20 °C
3. Coating buffer 100 mM sodium hydrogen carbonate; pH 9.6
4. Blocking Reagent for Elisa ; Roche Diagnostics GmbH Cat.No.: 1112589
5. PBST buffer 20 mM NaH₂PO₄ ; 140 mM NaCl ; 0.05% Tween 20 ; pH 7.4
6. Albumin bovine fraction V, protease-free ; Serva Cat.No.: 11926.03 ; stored at 4 °C.
7. PBST + 0.5% BSA buffer: 20 mM NaH₂PO₄ ; 140 mM NaCl ; 0.05% Tween 20 ; pH 7.4 + 0.5% BSA
8. Aβ(1 - 42) oligomer Standard Stock ; solution in 5 mM NaH₂PO₄; 35 mM NaCl ; pH 7.4 ; conc.: 10.77 mg/ ml ; stored at -20 °C
9. anti-Aβ mouse mAb clone 6B1; solution in PBS ; conc.: 1.2mg/ ml ; stored at -80 °C
10. anti-mouse-POD conjugate ; Fa.Jackson ImmunoResearch Cat.No.: 715-035-150 ;
11. Development:
   TMB ; Roche Diagnostics GmbH Cat.No.: 92817060 ; 42 mM in DMSO 3% H₂O₂ in water
   100 mM sodium acetate, pH 4.9
12. Stop Solution: 2 M Sulfonic Acid
3.2.B.2. Preparation of reagents:
1. Capture antibody:
Rabbit polyclonal anti Aβ(20 - 42) globulomer antibody 5598 was obtained by immunizing rabbits according to procedure described in Example 25 of W02004/067561.
5598 was immunopurified from this rabbit antiserum by affinity chromatography on Sepharose immobilized Aβ(1 - 42) globulomer.
- Preparation of Aβ(1 - 42) globulomer Sepharose:
Preparation of Aβ(1 - 42) globulomer: 9 mg Aβ(1 - 42) Fa. Bachem were dissolved in 1.5 ml HFIP (1,1,1,3,3,3-hexafluoro-2-propanol) and incubated for 1, 5 h at 37 °C. The solution was evaporated in a SpeedVac and suspended in 396 µl DMSO (5 mM Aβ stock solution). The sample was sonified for 20 seconds in a sonic water bath, shaken for 10 minutes and stored over night at -20 °C.
The sample was diluted with 4554 µl PBS (20 mM NaH₂PO₄ ; 140 mM NaCl ; pH 7.4) and 495 µl 2 % aqueous SDS-solution were added (0.2% SDS content).
The mixture was incubated for 7 h at 37 °C, diluted with 16335 µl H₂O and further incubated for 16 hours at 37 °C. After that the Aβ(1 - 42) globulomer solution was centrifuged for 20 min at 3000 g. The final Aβ(1 - 42) globulomer supernatant was discarded which was used for immobilisation.
Preparation of NHS-activated Sepharose: 2.0 g NHS-activated Sepharose Fast Flow, Amersham Biosciences, Cat. No.: 17-0906-01 were washed subsequently with
2 x 25 ml isopropanol 30% in 1 mM HCl (ice cooled);
2 x 25 ml 1 mM HCl (ice cooled); and finally
2 x 25 ml 50 mM NaHCO₃ pH 7.5 (ice cooled).
Immobilisation: 10 ml of Aβ(1 - 42) globulomer-solution were mixed with 2.0 g NHS-activated Sepharose and incubated for 2 h at room temperature.
The reaction mixture was centrifuged at 4000 g and the supernatant discarded.
Blockade: The resin was incubated after adding of 10 ml 50 mM NaHCO_{3 ;} 250 mM ethanolamine ; 0.05 % SDS, pH 7.5 for 1 hour at RT. The Sepharose material was collected by filtration and washed 4 x with 25 ml 5 mM NaH₂PO₄ ; 35 mM NaCl ; 0.05% SDS ; pH 7.4.
Cross linking: Sepharose material was resuspended with 10 ml 5 mM NaH₂PO₄; 35 mM NaCl ; 0.05% SDS ; pH 7.4 and mixed with 1 ml 40 mM glutaraldehyde solution in water (freshly prepared). After incubation (2h, room temperature) and centrifugation (10 min, 4000 g) the supernatant was discarded and the Sepharose treated with 10 ml 5 mM NaH₂PO₄; 35 mM NaCl; 250 mM ethanolamine; 0.05% SDS; pH 7.4 for 1 h at RT. The mixture was centrifuged at 4000 g, for 10 min and the supernatant discarded. The ethanolamine blocking reaction was repeated and finally the Sepharose material was collected by filtration and washed 4 x with 25 ml 5 mM NaH₂PO₄; 35 mM NaCl; 0.05% SDS ; pH 7.4.
The final Aβ(1 - 42) globulomer Sepharose material was added with 4.0 ml 5 mM NaH₂PO₄; 35 mM NaCl; 0.05% SDS; 0.02% NaN₃; pH 7.4 and stored at 4 °C in a refrigerator (0.5 g Sepharose/ ml) for further affinity chromatography.
- Affinity chromatography:
   0.8 g of Aβ(1 - 42) globulomer Sepharose material was collected by filtration and washed 4 times with 10 ml TBS buffer (25 mM Tris; 150 mM NaCl; pH 7.5). Rabbit serum 5598 was diluted (1:1) with TBS buffer. 40 ml of this sample was added to 0.8 g of Aβ(1 - 42) globulomer Sepharose and incubated at 8 °C over night. Aβ(1 - 42) globulomer-Sepharose was then filled into a column and washed with 25 ml TBS buffer. The elution was done with 0.58% acetic acid in 140 mM NaCl and the eluted UV-active fraction was collected. The eluted fraction (immunopurified Pab 5598 stock solution) was immediately adjusted to pH 7.5 by adding 2 M Tris buffer, pH 8.5, and stored at -80 °C.
- Preparation of antibody solution:
   The 5598 PAb stock solution was thawed and diluted 1:240 in coating buffer.
2. Blocking reagent :
Dissolve blocking reagent in 100 ml water to prepare the blocking stock solution and store aliquots of 10 ml at -20 °C.
Dilute 3 ml blocking stock solution with 27 ml water for each plate to block.
3. Aβ(1 - 42) globulomer standard dilution:
A - Add 1 µl of Aβ(1 - 42) globulomer standard stock solution to 1076 µl PBST + 0.5% BSA = 10 µg/ ml
B - Add 5µl of 10 µg/ ml Aβ(1 - 42) globulomer standard solution to 4995 µl PBST + 0.5% BSA = 10 ng/ ml

**Standard curve:**

| No | Stock | PBST + 0.5% BSA | Final conc. |
|---|---|---|---|
| 1 | 2 ml B | 0 ml | 10000 pg/ ml |
| 2 | 0.633 ml (1) | 1.367 ml | 3160 pg/ ml |
| 3 | 0.633 ml (2) | 1.367 ml | 1000 pg/ ml |
| 4 | 0.633 ml (3) | 1.367 ml | 316 pg/ ml |
| 5 | 0.633 ml (4) | 1.367 ml | 100 pg/ ml |
| 6 | 0.633 ml (5) | 1.367 ml | 31.6 pg/ ml |
| 7 | 0.633 ml (6) | 1.367 ml | 10 pg/ ml |
| 8 | 0 ml | 2 ml | 0.0 pg/ ml |

**Samples:**

| No | Stock | PBST + 0.5% BSA | dilution factor |
|---|---|---|---|
| 1 | 1 ml S | 0 ml | directly |
| 2 | 0.2 ml(1) | 0.8 ml | 1:5 |

4. Primary antibody 6B1:
Mouse monoclonal antibody 6B1 was obtained by standard procedures via immunisation with Aβ(1 - 42) globulomer. The antibody was purified from hybridoma using standard procedures.
The concentrated anti-Aβ mAb clone 6B1 was diluted in PBST + 0.5 % BSA-buffer. The dilution factor was 1/6000 = 0.2 µg/ ml. The antibody was used immediately.
5. Label Reagent:
Reconstitute anti-mouse-POD conjugate lyophilizate in 0.5 ml water. Add 500 µl glycerol and store aliquots of 100 µl at -20 °C for further use. Dilute the concentrated label reagent in PBST buffer. The dilution factor is 1/5000. Use immediately.
6. TMB solution:
Mix 20 ml 100 mM sodium acetate pH 4.9 with 200 µl of the TMB solution and 29.5 µl 3 % peroxide solution. Use immediately.
3.2.B.3. Sample Plate Setup (Note that all standards and samples are run in duplicate)

| | 1 | 2 | 3 | 4 | 5 | 6 | 7 | 8 | 9 | 10 | 11 | 12 |
|---|---|---|---|---|---|---|---|---|---|---|---|---|
| A | 10000 | 10000 | U1 | U1 | | | | | | | | |
| B | 3160 | 3160 | U2 | U2 | | | | | | | | |
| C | 1000 | 1000 | U3 | U3 | | | | | | | | |
| D | 316 | 316 | U4 | U4 | | | | | | | | |
| E | 100 | 100 | U5 | U5 | | | | | | | | |
| F | 31.6 | 31.6 | U6 | U6 | | | | | | | | |
| G | 10 | 10 | U7 | U7 | | | | | | | | |
| H | 0.0 | 0.0 | U8 | U8 | | | | | | | | |

| | | | | | | | | | | | | |
|---|---|---|---|---|---|---|---|---|---|---|---|---|
| U1-U8 = Unknown samples | | | | | | | | | | | | |

3.2.B.4. Procedure
1. Apply 100 µl anti-Aβ pAb rabbit 5598 solution per well and incubate overnight at 4 °C.
2. Discard the antibody solution and wash the wells with 250 µl PBST buffer for three times.
3. Add 300 µl block solution per well and incubate 2 h at room temperature.
4. Discard the block solution and wash the wells with 250 µl PBST buffer for three times.
5. After preparation of standards and samples, apply 100 µl per well of standards and samples to the plate. Incubate 2 h at room temperature and overnight at 4 °C.
6. Discard the standard/sample solution and wash the wells with 250 µl PBST buffer for three times.
7. Add 200 µl primary antibody solution per well and incubate 1.5 h at room temperature.
8. Discard the antibody solution and wash the wells with 250 µl PBST buffer for three times.
9. Add 200 µl label solution per well and incubate 1 h at room temperature.
10. Discard the label solution and wash the wells with 250 µl PBST buffer three times.
11. Add 100 µl of TMB solution to each well and incubate at room temperature (5 - 15 min).
12. Observe colour development and apply 50 µl of the Stop solution per well.
13. Read at 450 nm.
14. Calculate results from standard curve.
15. Evaluation:
   If extinctions from unknown samples are not in the linearity range of the calibration curve, repeat Elisa with appropriated sample dilution.

From these data it can be concluded that Aβ(1 - 42) globulomer epitopes exist in vivo at a very early stage of Alzheimer's disease, preceding all clinical manifestations. Therefore it is to be assumed that they may be valuable in early detection and risk assessment of AD and related conditions.

### Example 4: Generation and characterization of globular Aβ(1 - 42) oligomers

The initial step in the procedure of globular Aβ(1 - 42) oligomer generation (see Fig. 11 a) is a pretreatment of synthetic Aβ(1 - 42) peptide with HFIP in order to remove pre-existing structural inhomogeneities (Stine, W.B., Jr., Dahlgren, K.N., Krafft, G.A. & LaDu, M.J. In vitro characterization of conditions for amyloid-beta peptide oligomerization and fibrillogenesis. J Biol Chem 278, 11612 - 11622 (2003)). After resuspension in DMSO incubation in 0.2% SDS generates first an intermediate oligomeric Aβ form with 16/20 kDa (apparent mass of the two bands on SDS-PAGE). Upon further dilution and incubation this intermediate self-associates to the final 38/48 kDa oligomeric Aβ form, which was found to be of globular structure and therefore is referred to as 38/48 kDa globular Aβ(1 - 42) oligomer or briefly Aβ(1 - 42) globulomer in the following (see Fig. 11 b).

Separation of residual fibril prone monomers from the globulomer fraction from can be achieved by depletion via affinity chromatography using antibodies specific for fibril prone Aβ(1 - 42) monomer bound to immobilized Protein A, or via direct antibody depletion (immunoprecipitation or immunosorption) of fibril prone Aβ(1 - 42) monomer. These methods are described in more detail below.

Glutardialdehyde cross-linking of both the intermediate 16/20 kDa and the final 38/48 kDa Aβ(1 - 42) globulomer merged the two bands on SDS-PAGE into one, indicating a uniform Aβ(1 - 42) globulomer structure. The Aβ(1 - 42) peptide interactions forming the Aβ(1 - 42) globulomer structure are exclusively non-covalent. Thermal denaturation reverted the Aβ(1 - 42) globulomer but not the cross-linked Aβ(1 - 42) globulomer to monomeric Aβ(1 - 42). Nevertheless, the non-covalently linked Aβ(1 - 42) globulomer exhibited long-term stability at physiological temperature (37 °C). The Aβ(1 - 42) globulomer structure is maintained for up to 4 days in vitro without obvious disassembly or further polymerization to fibrils.

In contrast, a standard monomer preparation of Aβ(1 - 42) peptide (Bachem, Switzerland) dissolved in 0.1 % NH₄OH (Aβ(1 - 42) NH₄OH prep.) showed a high extent of aggregation after already 1 day of incubation (see Fig. 11c).

Aβ-forms were analyzed with respect to their molecular weight distribution using a Superose 12 HR10/30 column (Amersham Biosciences, Germany) and PBS (pH 7.4) as running-buffer. The column was calibrated with a set of standard proteins. Aβ-peptide mass was determined by SELDI-MS using a H4 protein chip (Ciphergen Biosystems, USA) and alpha-cyano - 4-hydroxy-cinnamic acid in 50% acetonitril / 0.5% trifluor acetic acid as matrix.

Under native conditions (PBS, pH 7.4) size exclusion chromatography of the Aβ(1 - 42) globulomer showed a single, although broad peak at ∼100 kDa (see Fig. 11d). Similar to SDS-PAGE the cross-linked Aβ(1 - 42) globulomer showed a reduced apparent molecular weight with a narrow peak at 60 kDa, suggesting that Aβ(1 - 42) globulomers exist only in a single globular-shaped form which condenses upon glutardialdehyde cross-linking.

In contrast, the Aβ(1 - 42) NH₄OH preparation was highly instable and fibrillization prone. Already about 5 min after resuspension prominent aggregation was detectable (11 kDa major peak, 74 kDa minor peak) and after 1 h of incubation pronounced fibril aggregation prevented Aβ from entering the chromatography column (data not shown). Proteolysis with promiscuous proteases truncated the Aβ(1 - 42) globulomer from the N-terminus onwards up to -amino acid 20, leaving the C-terminal part and the globular structure intact (see Fig. 11e). A SELDI-MS analysis of thermolysine cleavage products showed that cross-linking of Aβ(1 - 42) globulomer led to a loss of the Aβ(4 - 42) cleavage product, while Aβ(20 - 42) was maintained (see Fig. 11f). Hence, cross-linking affected only the N-terminus and Lys-16 but not Lys-28, which must therefore be hidden in the interior of the Aβ(1 - 42) globulomer.

In the following, suitable methods for clearing the Aβ(1 - 42) globulomersolution of residual fibril prone monomers are described. All of these require the following reagents in addition to those listed:
- Protein A Sepharose 4 Fast Flow Amersham Biosciences Cat.No.: 17-0974-01
- PBS buffer: 20 mM NaH₂PO₄; 140 mM NaCl; 0.05 % Pluronic F68; pH 7.4

4.1. Purification of Aβ(1 - 42) globulomers by depleting contaminations of Aβ(1 - 42) fibril prone monomers in Aβ(1 - 42) globulomer preparations via Protein A bound fibril prone Aβ(1 - 42) specific antibody affinity chromatography
   4.1.A. List of reagents:
      - Capture antibody anti-Aβ(1 - 42) rabbit pAb; biotinylated IgG solution in PBS with 50 % glycerol; conc.: 0.5 mg/ ml ; Signet Cat.No. 9135 ; store at -80 °C
      - Aβ(1 - 42) oligomer standard stock; solution in 5 mM NaH₂PO₄; 35 mM NaCl; pH 7.4; conc.: 10.77 mg/ ml; store at -20 °C
   4.1.B. Preparation of reagents:
      1. Capture antibody dilution: Dilute the concentrated anti-Aβ(1 - 42) pAb 1:5 in PBS to 0.1 mg/ ml.
      2. Capture antibody immobilization on Protein A Sepharose: Equilibrate 20 µl Protein A Sepharose with 200 µl PBS-buffer followed by centrifugation for 5 min at 3000 g and discard supernatant. Repeat procedure 4 times. Add 100 µl of the diluted anti-Aβ(1 - 42) pAb 1:5 in PBS to the Protein A Sepharose pellet and shake for 1 hour at room temperature. Centrifuge for 5 min at 3000 g. Discard supernatant and wash Protein A Sepharose three times with 200 µl PBS followed by centrifugation for 5 min at 3000 g each time. Discard supernatants.
      3. A_{1 - 42} oligomer standard dilution: Add 50 µl Aβ(1 - 42) oligomer standard stock solution to 450 µl PBS = 1.08 mg/ ml.
   4.1.C. Procedure:
      500 µl Aβ(1 - 42) of oligomer standard dilution are added to the 20 µl Protein A Sepharose immobilized anti-Aβ(1 - 42) pAb resin. Shake sample for 2 hours at room temperature and further 16 hours at 4 °C followed by centrifugation for 5 min at 3000 g.
      The Aβ fibril prone contaminants (fibrillomers) react with the affinity matrix and the supernatant contains the purified Aβ globulomer in a globulomer epitope purity >> 95 %.
      The purity of this sample is further improved by repeating the procedure twice which yielded globulomer epitope purity > 99 % in the supernatants.
4.2. Purification of Aβ globulomers by depleting contaminations of Aβ(1 - 42) fibril prone monomer in Aβ(1 - 42) crude preparations via direct depletion of fibril prone Aβ(1 - 42).
   4.2.A. List of reagents:
      - F96 Cert. Maxisorp NUNC-Immuno Plate Cat.No.: 439454
      - Capture antibody anti-Aβ(1 - 42) rabbit pAb; biotinylated IgG solution in PBS with 50 % glycerol; conc.: 0.5 mg/ ml; Signet Cat.No. 9135; store at -80 °C
      - Coating buffer: 100 mM sodium hydrogen carbonate; pH 9.6
      - Blocking Reagent for Elisa; Roche Diagnostics GmbH Cat.No.: 1112589
      - PBST buffer: 20 mM NaH₂PO₄; 140 mM NaCl ; 0.05 % Tween 20 ; pH 7.4
      - Aβ(1 - 42) oligomer Standard Stock ; solution in 5 mM NaH₂PO₄ ; 35 mM NaCl; pH 7.4; conc.: 10.77 mg/ ml ; store at -20 °C
   4.2.B. Preparation of reagents:
      1. Capture antibody: Dilute the concentrated anti-Aβ(1 - 42) pAb 1:100 in coating buffer.
      2. Blocking reagent: Dissolve blocking reagent in 100 ml water to prepare the blocking stock solution and store aliquots of 10 ml at -20 °C. Dilute 3 ml blocking stock solution with 27 ml water for each plate to block.
      3. Aβ(1 - 42) globulomer standard dilution: Add 1 µl Aβ(1 - 42) globulomer standard stock solution to 1076 µl PBST = 10 µg/ ml Aβ(1 - 42) globulomer standard solution.
   4.2.C. Procedure:
      Apply 100 µl anti-Aβ(1 - 42) mAb clone Signet 9135 solution per well and incubate overnight at 4 °C. Discard the antibody solution and wash the wells with 250 µl PBST buffer three times. Add 300 µl block solution per well and incubate 2 h at room temperature. Discard the block solution and wash the wells with 250 µl PBST buffer three times. After preparation of standard, apply 100 µl to each well of Aβ(1 - 42) globulomer standard solution to the plate. Incubate for 2 h at room temperature and overnight at 4 °C. Collect the Aβ(1 - 42) globulomer standard solution in a 15 ml Falcon tube which now has a purity >95 %.
         The globular epitope purity can be further improved be repeating the above immunoaffinity procedure in the microtiter plate twice. After this procedure the Aβ(1 - 42) globulomer solution is >> 99 % pure with respect to Aβ fibril prone type structures (monomer and fibrillomers).
4.3. Enrichment of Aβ globulomer epitopes by depleting contaminations of Aβ fibril prone monomer in Aβ(1 - 42) ADDL preparations via Protein A bound fibril prone Aβ(1 - 42) specific antibody affinity chromatography
   4.3.A. List of reagents:
      - Capture antibody anti-Aβ(1 - 42) rabbit pAb; biotinylated IgG solution in PBS with 50 % glycerol; conc.: 0.5 mg/ ml; Signet Cat.No. 9135; store at -80 °C
      - Aβ(1 - 42) ADDL standard stock; solution in F12-medium; conc.: 0.12 mg/ ml ; stored at -80 °C
   4.3.B. Preparation of reagents:
      1. Equilibration of Protein A-Sepharose: Equilibrate 20 µl Protein A Sepharose with 200 µl PBS buffer followed by 200 µl of 0.58 % acetic acid and further wash with 200 µl PBS buffer in a column. Place the equilibrated Protein A-Sepharose in a tube and add the same volume PBS-buffer to the matrix.
      2. Aβ(1 - 42) ADDL standard dilution: Add 42 µl Aβ(1 - 42) ADDL standard stock solution to 58 µl PBS = 0.05 mg/ml.
   4.3.C. Procedure:
      20 µl anti-Aβ(1 - 42) rabbit pAb; biotinylated IgG solution are added to the 100 µl Aβ(1 - 42) ADDL solution. Shake sample for 2 hours at room temperature. Add 10 µl equilibrated Protein A Sepharose suspension in PBS buffer to the sample and shake for 1 hour at room temperature followed by centrifugation for 5 min at 3000 g.
      The Aβ fibril prone contaminants (fibrillomers) react with the affinity matrix and the supernatant shows a globulomer epitope purity >> 55 %.
      The purity of this sample is further improved by repeating the procedure twice which yielded globulomer epitope purity > 95% in the supernatants.
4.4. Enrichment of Aβ globulomer epitopes by depleting contaminations of fibril prone Aβ in Aβ(1 - 42) ADDL preparations via Protein A bound fibril prone Aβ(1 - 42) specific antibody affinity chromatography
   4.4.A. List of reagents:
      - Capture antibody: anti-Aβ(1 - 42) mAb C-terminal clone BD1780; purified, lyophilized ; Biodesign Cat.No. Q67780M; store at -20 °C
      - Aβ(1 - 42)-ADDL's standard stock; solution in F12-medium; conc.: 0.12mg/ ml; store at -80 °C
   4.4.B. Preparation of reagents:
      1. Equilibration of Protein A-Sepharose: Equilibrate 20 µl Protein A Sepharose with 200 µl PBS-buffer followed by 200 µl of 0.58 % acetic acid and further wash with 200 µl PBS-buffer in a column. Place the equilibrated Protein A-Sepharose in a tube and add the same volume PBS-buffer to the matrix.
      2. Capture antibody reconstitution: Reconstitute 100 µg antibody with 1 ml water to 0.1 mg/ ml.
      3. Aβ(1 - 42) ADDL Standard dilution: Add 42 µl Aβ(1 - 42) ADDL's standard stock solution to 58 µl PBS = 0.05 mg/ ml.
   4.4.C. Procedure :
      100 µl anti-Aβ(1 - 42) rabbit pAb; biotinylated IgG solution are added to the 100 µl Aβ(1 - 42) ADDL solution. Shake sample for 2 hours at room temperature. Add 10 µl equilibrated Protein A Sepharose suspension in PBS-buffer to the sample and shake for 1 hour at room temperature followed by centrifugation for 5 min at 3000 g. The Aβ fibril prone contaminants (fibrillomers) react with the affinity matrix and the supernatant shows a globulomer epitope purity >> 50 %.
      The purity of this sample is further improved by repeating the procedure twice which yielded globulomer epitope purity > 90% in the supernatants.
4.5. Enrichment of Aβ globulomer epitopes by depleting contaminations of Aβ fibrilomers in Aβ(1 - 42) NH₄OH preparations via Protein A bound fibril prone Aβ(1 - 42) specific antibody affinity chromatography
   4.5.A. List of reagents:
      - Capture antibody anti-Aβ(1 - 42) rabbit pAb; biotinylated IgG solution in PBS with 50% glycerol; conc.: 0.5 mg ml ; Signet Cat.No. 9135 ; store at -80 °C
      - Aβ(1 - 42) NH₄OH standard stock; solution in 0.1 % NH₄OH; conc.: 2 mg/ ml; store at -80 °C
   4.5.B. Preparation of reagents:
      1. Equilibration of Protein A-Sepharose: Equilibrate 20 µl Protein A Sepharose with 200 µl PBS-buffer followed by 200µ1 of 0.58 % acetic acid and further wash with 200 µl PBS-buffer in a column. Place the equilibrated Protein A-Sepharose in a tube and add the same volume PBS-buffer to the matrix.
      2. Aβ(1 - 42) NH₄OH Standard dilution: Add 2.5 µl Aβ(1 - 42) ADDL's standard stock solution to 97.5 µl PBS = 0.05 mg/ ml.
   4.5.C. Procedure: As described under 4.3.C.

### Example 5: Generation of Aβ(20 - 42) globulomer starting from peptide Aβ(20 - 42)

### a) Preparation of stock globulomer solution

0.5 mg β-amyloid(20 - 42) protein (lyophilised, Anaspec Inc., Nr.: 408/452-5055/33908) were dissolved in 25 µl 1,1,1,3,3,3-hexafluoro-2-propanol and incubated for 1 hour, 37 °C an Eppendorff tube followed by 10 min centrifugation at 10'000 g. The supernatant is removed yielding a 20 mg/ ml (9 mM) Aβ(20 - 42) stock solution which could be stored at -80 °C.

### b) Preparation of final stable Aβ(20 - 42) globulomer

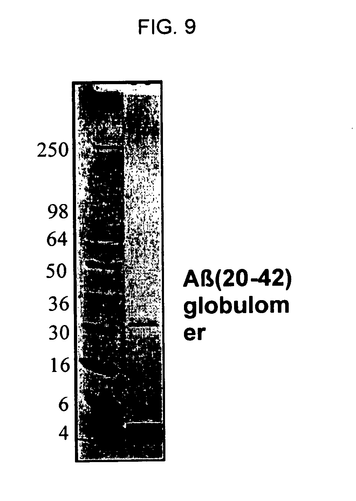

4 µl of Aβ(20 - 42) stock solution from Example 5a) were mixed with 196 µl of 2 mM sodium phosphate, 14 mM NaCl, pH 7.4 and 20 µl of a 1% sodium dodecyl sulfate (SDS) solution followed by 20 h incubation at 6 °C and finally centrifugation for 10 min at 10'000 g. The supernatant was collected as about 180 µM Aβ(20 - 42) globulomer preparation and could be stored at -20 °C. The sample was analyzed by subsequent SDS-PAGE (Fig 9) and the product showed up at 32 kDa. Further purification can be achieved by purification method described in Example 4.

### Example 6: Generation of Aβ(12 - 42) globulomer starting from peptide Aβ(12 - 42)

### a) Preparation of stock globulomer solution

Starting with 0.5 mg β-amyloid (12 - 42) protein (lyophilised, Anaspec Inc., Nr.: 408/452-5055/33907A), the procedure as described in Example 5a) yielded a 20 mg/ ml (6.2 mM) Aβ(12 - 42) stock solution which could be stored at -80 °C.

### b) Preparation of final stable Aβ(12 - 42) globulomer

4 µl of Aβ(12 - 42) stock solution from Example 6a) were processed as described in Example 5b) to yield about 120 µM Aβ(12 - 42) globulomer preparation which could then be stored at -20 °C.

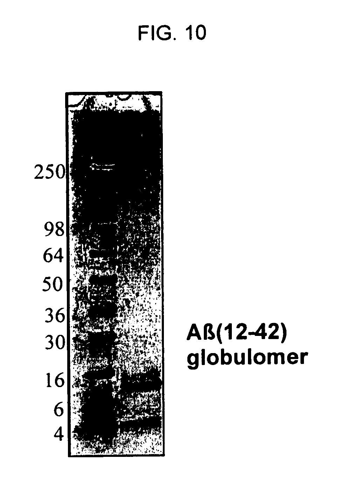

The sample was analyzed by subsequent SDS-PAGE (Fig 10) and the product showed up at about 12 kDa in almost quantitative yield. Ultrapure Aβ(12-42) globulomer might be obtained by further purification with the method described in Example 4.

### Example 7: Detection of anti-globulomer auto-antibodies in human body fluids by dot-blot of plasma or CSF

Treatment of plasma or serum samples:
a) Titers of the free fraction of auto-antibodies were directly measured from sera or plasma.
b) The titers of the total auto-antibodies were measured by pre-treatment of plasma/serum samples as follows: 100 µl plasma/serum was mixed with 10 ml 140 mM NaCl + 0.58% acetic acid and incubated for 20 min followed by concentration with a Centriprep YM30 (Amicon Inc.) to 1 ml.

Then the plasma/serum samples were assessed for anti-Aβ auto-antibodies. To this end, dilution series of the individual Aβ(1-42) forms ranging from 100 pmol/µl to 0.01 pmol/µl in PBS supplemented with 0.2 mg/ ml BSA were made. 1 µl of each sample was blotted onto a nitrocellulose membrane. For detection the corresponding mouse plasma samples were used (diluted 1:400). Immunostaining was done using alkaline phosphatase conjugated anti-mouse-IgG and the staining reagent NBT/BCIP.

### Aβ-standards for dot-blot:

### 1. Aβ(1-42) globulomer

The preparation of the Aβ(1-42) oligomers (termed Aβ(1-42) globulomer in the following) is described in WO2004/067561.

### 2. Aβ(1-42) monomer

3 mg human Aβ(1-42), (Bachem Inc.; cat. no. H-1368) were dissolved in 0.5 ml HFIP (6 mg/ml suspension) in an 1.7 ml Eppendorff tube and was shaken (Eppendorff Thermo mixer, 1400 rpm) for 1.5h at 37 °C until a clear solution was obtained. The sample was dried in a SpeedVac concentrator (1.5 h) and resuspended in 13.2 µl DMSO, shaken for 10 sec., followed by sonification (20 sec), and shaking (e.g. in Eppendorff Thermo mixer, 1400 rpm) for 10 min. 6 ml of 20 mM NaH₂PO₄; 140 mM NaCl; 0.1 % Pluronic F68; pH 7.4 were added and stirred for 1 h at room temperature. The sample was centrifuged for 20 min at 3000 g. The supernatant was discarded and the precipitate solved in 0.6 ml 20 mM NaH₂PO₄ ; 140 mM NaCl ; 1% Pluronic F68, pH 7.4. 3.4 ml of H₂O was added and stirred for 1 h at room temperature followed by 20 min centrifugation at 3000 g. Eight aliquots of each 0.5 ml of the supernatant were stored at -20° for further use.

### 3. Aβ(12-42) globulomer

The preparation of the Aβ(12-42) oligomers (termed Aβ(12-42) globulomer in the following) is described in WO2004/067561.

### 4. Aβ(12-42) monomer

5 mg/ ml solution (Anaspec Inc.) in 0.1 % NaOH was prepared and stored at -20 °C.

### 5. Aβ(17-42) globulomer

### Preparation of truncated Aβ(17-42) globulomer, starting from Aβ(1-42) oligomers, by cleavage with trypsin:

0.1 ml of Aβ(1-42) globulomer (conc. 12.5 mg/ ml, preparation of the Aβ(1-42)-globulomer is described in WO2004/067561), are mixed with 2,4 ml of buffer (20 mM NaH₂PO₄, 140 mM NaCl pH 7.4) and 125 µl of a 0.2 mg/ml Trypsin solution (Roche Inc.) in 20 mM NaH₂PO₄, 140 mM NaCl pH 7.4. The reaction mixture is stirred at RT for 20 h. Then 25 µl of a 100 mM diisopropylfluorophosphate solution in water are added and the mixture is furthermore adjusted to an SDS content of 0.03% with 80 µl of a 1 % strength SDS solution. The reaction mixture is concentrated to approx. 0.25 ml via a 10 kDa Centriprep tube. The concentrate is mixed with 0.625 ml of buffer (20 mM NaH₂PO₄, 140 mM NaCl, pH 7.4) and again concentrated to 0.25 ml.

### 6. Aβ(17-42) monomer

5 mg/ ml solution (Anaspec Inc.) in 0.1 % NaOH was prepared and stored at -20 °C.

### 7. Aβ(20-42) globulomer

The preparation of the Aβ(20-42) oligomers (termed Aβ(20-42) globulomer in the following) is described in WO2004/067561.

### 8. Aβ(20-42) monomer

5 mg/ ml solution (Anaspec Inc.) in 0.1 % NaOH was prepared and stored at -20 °C.

### 9. Aβ(1-40) monomer

1 mg human Aβ(1-40), (Bachem Inc, cat. no. H-1194) were suspended in 0.25 ml HFIP (4mg/ ml suspension) in an Eppendorff tube. The tube was shaken (e.g. in Eppendorff Thermo mixer, 1400 rpm) for 1.5h at 37 °C to get a clear solution and afterwards dried in a speed vac concentrator (1.5h). The sample was redissolved in 46µl DMSO (21.7mg/ ml solution = 5 mM), shaken for 10sec and subsequently sonicated for 20sec. After 10 min shaking (e.g. in Eppendorff Thermo mixer, 1400rpm) the sample is stored at -20 °C for further use.

### 10. Aβ(1-42) fibrils

1 mg human Aβ(1-42) (Bachem Inc. Catalog Nr.: H-1368) were dissolved in 500µl aqueous 0.1 % NH₄OH (Eppendorff tube) and the sample was stirred for 1 min at room temperature. 100µl of this freshly prepared Aβ(1-42) solution were neutralized with 300µl 20 mM NaH₂PO₄ ; 140 mM NaCl, pH 7.4. The pH was adjusted to pH 7.4 with 1% HCl. The sample was incubated for 24h at 37 °C and centrifuged (10 min at 10'000 g). The supernatant was discarded and the fibril pellet resuspended with 400µl 20 mM NaH₂PO₄; 140 mM NaCl, pH 7.4 by vortexing for 1 min.

### Materials dot blot:

| Aβ-standards: | |
|---|---|
| | Serial dilution of Aβ-antigens in 20 mM NaH₂PO₄, 140 mM NaCl, |
| | pH 7.4 + 0.2 mg/ ml BSA |
| | 1) 100 pmol/µl |
| | 2) 10 pmol/µl |
| | 3) 1 pmol/µl |
| | 4) 0.1 pmol/µl |
| | 5) 0.01 pmol/µl |
| | |
| Nitrocellulose: | |
| | Trans-Blot Transfer medium, Pure Nitrocellulose Membrane (0.45 |
| | µm); Fa. BIO-RAD |
| | |
| Anti-Mouse-AP: | |
| | AQ330 (Fa. Chemicon) |
| | |
| Detection reagent: | |
| | NBT/BCIP Tablets ( Roche) |
| | |
| Bovine Serum Albumin, (BSA): | |
| | A-7888 (SIGMA) |
| | |
| Blocking reagent: | |
| | 5 % low fat milk in TBS |
| | |
| Buffer solutions: | |
| | TBS |
| | 25 mM Tris / HCl - buffer pH 7.5 |
| | + 150 mM NaCl |
| | TTBS |
| | 25 mM Tris / HCl - buffer pH 7.5 |
| | + 150 mM NaCl |
| | + 0.05 % Tween 20 |
| | PBS +0.2 mg/ ml BSA |
| | 20 mM NaH₂PO₄ buffer pH 7.4 |
| | + 140 mM NaCl |
| | + 0.2 mg/ ml BSA |
| | |
| Antibody solution I: | |
| | Mouse plasma samples from an active immunization |
| | study with Aβ(20-42) globulomer (1:400 diluted in 20 ml 1 |
| | % low fat milk in TBS) |
| | |
| Antibody solution II: | |
| | 1:5000 dilution |
| | Anti-Mouse-AP in 1 % low fat milk in TBS |

### Dot blot procedure:

1) 1 µl each of the different Aβ-standards (in their 5 serial dilutions) were dotted onto the nitrocellulose membrane at a distance of approximately 1 cm from each other.
2) The Aβ-standards dots are allowed to dry on the nitrocellulose membrane on air for at least 10 min at room temperature (RT) (= dot blot)
3) Blocking: The dot blot is incubated with 30 ml 5% low fat milk in TBS for 1.5 h at RT.
4) Washing:
   The blocking solution is discarded and the dot blot incubated under shaking with 20 ml TTBS for 10 min at RT.
5) Antibody solution I:
   The washing buffer is discarded and the dot blot incubated with antibody solution I overnight at RT.
6) Washing:
   The antibody solution I is discarded and the dot blot incubated under shaking with 20 ml TTBS for 10 min at RT. The washing solution is discarded and the dot blot incubated under shaking with 20 ml TTBS for 10 min at RT. The washing solution is discarded and the dot blot incubated under shaking with 20 ml TBS for 10 min at RT.
7) Antibody solution II:
   The washing buffer is discarded and the dot blot incubated with antibody solution II for 1 h at RT
8) Washing:
   The antibody solution II is discarded and the dot blot incubated under shaking with 20 ml TTBS for 10 min at RT. The washing solution is discarded and the dot blot incubated under shaking with 20 ml TTBS for 10 min at RT. The washing solution is discarded and the dot blot incubated under shaking with 20 ml TBS for 10 min at RT.
9) Development:
   The washing solution is discarded. 1 tablet NBT/BCIP is dissolved in 20 ml H₂O and the dot blot is incubated for 5 min with this solution. The development is stopped by intensive washing with H₂O.
10) Densitometer analysis:
   Quantitative evaluation was done using a densitometric analysis (BioRad densitometer and software package Quantity one (BioRad)) of the intensity; for the evaluation in Fig. 24 the 100 pMol refractive density values were taken as readout.

The results are shown in Fig. 24 a and b.

It is concluded that the plasma of a human Alzheimer disease patient does contain auto-antibodies which are reactive to Aβ(12 - 42) globulomer and Aβ(20 - 42) globulomer, and that the CSF of a human Alzheimer disease patient does contain auto-antibodies which are reactive to Aβ(12 - 42) globulomer and Aβ(20 - 42) globulomer.

These results demonstrate the feasibility of testing for the existence of globulomers, which in themselves are difficult to detect because of their high affinity to their targets, in body fluids by assessing the existence of antibodies against globulomers. They further corroborate the status of globulomers as patho-physiological entities.

### Example 8: Active immunization

### Description:

Active immunization of APP transgenic mice carrying and overexpressing the human APP with the London mutation (APP/Lo; Moechars et al., 1999) under control of a Thy1 promotor reversed a memory deficit in the novel object recognition task. In APP/Lo mice immunized with Aβ(1 - 42) globulomer or Aβ(1 - 42) globulomer, but not in mice immunized with the Aβ(1 - 42) monomer the inventors found an increased investigation index for a new object compared to a known object which they first encountered 3 hours before (Fig. 21). The curiosity of the mice measured as time of investigation of the object during the first encounter was not changed between groups. Therefore, the active immunization with Aβ(1 - 42) globulomer and Aβ(20 - 42) globulomer selectively improved the short term memory for objects.

### Methods:

### - Active immunization of APP transgenic mice:

APP [V717I] C57Bl x FVB female mice (n = 36; 6 weeks old) were used for this study (Moechars et al., 1999). All mice were housed and bred in the animal housing facilities of the Catholic University Leuven, Campus Gasthuisberg, in accordance with the Belgian ethical law on housing, breeding and handling laboratory animals.

They were genotyped by polymerase chain reaction (PCR) at the age of 3 weeks and were double checked by a second PCR at the onset of the study. All mice were blind randomised, age-matched and allocated rando mly to a treatment. Animals were re-caged by treatment group one day before the onset of the study in order to allow them to familiarize to the new cage context. They had free access to pre-filtered and sterile (UV-lamp) water and standard mouse chow (Muracon-G, Trouw Nutrition, Gent). The food was stored under dry and cool conditions in a well-ventilated storage room. The amount of water and food was checked daily, supplied when necessary and standard refreshed twice a week. Mice were housed under a reversed day-night rhythm: 14 hours light / 10 hours darkness, light starting at 7 p.m., in standard metal cages type RVS T2 (area of 540 cm²) equipped with solid floors and layer of bedding litter.

Mice were treated intraperitoneally with one of the following solutions (200 µL per injection; n = 7-9 per group): phosphate-buffered saline (PBS; pH 7.4), 100 µg Aβ(1 -42) (Bachem, H1368) in 0.25% NH₄OH 100 µg Aβ(1 - 42) globulomer, or 30 µg Aβ(1 - 42) globulomer. The first injection contained 50 % (v/v) complete Freund's Adjuvants (Difco Laboratories, Detroit, Michigan, USA, ref n° 263810) while the boost injections contained 50 % (v/v) incomplete Freund's Adjuvant (Difco Laboratories, Detroit, Michigan, USA, ref n° 263910). Administration of compounds was performed every 3 weeks during 3 months (at week 0/3/6/9/12).

### - Novel object recognition task in immunized mice:

After the last injection all mice underwent a novel objection recognition task. The protocol used followed the method as described by Dewachter I. et al., Journal of Neuroscience, 2002, 22(9):3445 - 3453. Mice were familiarized for one hour to a Plexiglas open-field box (52 x 52 x 40 cm) with black vertical walls and a translucent floor, di mly illuminated by a lamp placed underneath the box. The next day the animals were placed in the same box and submitted to a 10 minutes acquisition trial. During this trial mice were placed individually in the open field in the presence of object A (blue ball or red cube, similar sized of ± 4 cm), and the duration (time_{AA}) and the frequency (Freq_{AA}) exploring object A (when the animals snout was directed towards the object at a distance of < 1 cm and the mice were actively sniffing in the direction of the object) was recorded by a computerized system (Ethovision, Noldus information Technology, Wageningen, the Netherlands). During a 10 minutes retention trial (second trial) performed 3 hours later, a novel object (object B, red cube or blue ball) was placed together with the familiar object (object A) into the open field. (Freq _{A} and Freq _{B} and Time_{A} and Time_{B}, respectively).

The recognition index (RI), defined as the ratio of the duration in which the novel object was explored over the duration in which both objects were explored [Time _{B}/ (Time _{A} + Time _{B}) x 100], was used to measure non-spatial memory. The duration and frequency object A was explored during the acquisition trial (Time_{AA} and Freq_{AA}) was used to measure curiosity.

### - Results:

During the active immunization process, the gain of body weight among the differentially treated groups of mice was the same, indicating no side effects of the immunization procedure. During the first encounter of the object all groups showed the same curiosity, i.e. no statistically significant differences in Time_{AA} and Freq_{AA}. During the second encounter, the mice immunized with Aβ(1 - 42) globulomer and Aβ(20 - 42) globulomer but not those with Aβ(1 - 42) monomer or vehicle investigated the unknown subject significantly longer, i.e. RI more than 50% (=chance level because of no memory; P < 0.01; Student's t-test). Moreover, the RI of mice immunized with Aβ(1 - 42) globulomer and Aβ(20 - 42) globulomer was significantly higher than the RI of mice treated with Aβ(1 - 42) monomer or vehicle (P < 0.01; ANOVA followed by post-hoc Holm-Sidak t-test).

### Example 9: Passive immunization

### Description:

Passive immunization of APP transgenic mice carrying and overexpressing the human APP with the London mutation (APP/Lo; Moechars et al., 1999) under control of a Thy1 promotor reversed a memory deficit in the novel object recognition task. In APP/Lo mice immunized with the mouse monoclonal antibodies 6G1 and 8F5 compared to PBS-treated mice, the inventors found an increased investigation index for a new object compared to a known object which they first encountered 3 hours before (Fig. 22). The curiosity of the mice measured as time of investigation of the object during the first encounter was not changed between groups. Therefore, the passive immunization with the mouse monoclonal antibodies 6G1 and 8F5 selectively improved the short term memory for objects.

### Methods:

Mice and housing conditions were identical to the protocol used in Example 8. Mice were intraperitoneally injected with 250 µg of the monoclonal antibodies 6B1 and 8F5 in 250 µL phosphate-buffered saline (PBS) or with PBS alone (n = 8 for all groups) which were raised against Aβ globulomer once a week for two weeks (2 injections in total). One day after the last injection an object recognition task as described in Example 8 was performed.

### Results:

During the first encounter of the object all groups showed the same curiosity, i.e. no statistically significant differences in Freq_{AA}. During the second encounter, the mice immunized with the mouse monoclonal antibodies 6G1 and 8F5 but not those injected with PBS only investigated the unknown subject significantly longer, i.e. RI more than 50% (=chance level because of no memory; P at least < 0.05; Student's t-test).

### Example 10: Induction of Aβ(1 - 42) globulomer formation by fatty acids

A variety of fatty acids were tested if they are capable to replace SDS as inducer of in vitro Aβ(1 - 42) globulomer formation.

Results are shown in Fig. 14. As can be seen, lauric acid, oleic acid, and arachidonic acid also induced the characteristic 38/48 kDa Aβ(1 - 42) globulomer, whereas eicosapentanoic acid created a partly shifted oligomer, and linoleic acid, docosahexanoic acid and docosatetranoic acid could not generate the characteristic 38/48 kDa Aβ double band within the detection limit.

These data suggests that in vivo globulomer formation is promoted not only by the as yet uncharacterized factors leading to erroneous cleavage of APP and thus overproduction of Aβ but also by a direct catalytic influence of lipids, presumably by providing anionic surfaces, in form of micelles or vesicles, which induce conformational changes in Aβ and/or serve as scaffolds where globulomer formation may occur. This is of particular interest as it may indicate a role of globulomers in the dementing effects of certain lipid metabolism disturbances as well as potentially beneficial effects of drugs influencing cerebral lipid metabolism or composition on AD and related conditions.

### Example 11: In vivo binding of Aβ(1 - 42) globulomers

Intracranial injections in rats were performed to test the feasibility of in vivo administration of Aβ(1 - 42) globulomer. For electrophysiological and histological studies, male Sprague-Dawley rats were obtained from Janvier (France) at an age of about 8 weeks. All animals were maintained under standard conditions (12 h day/night cycle, 22 °C, 50% humidity) with free access to food and tap water and allowed to recover for at least one week after arrival in our animal colony.

The rats (260 - 430 g) were anesthetized with pentobarbital (60 mg/kg i.p.; Narcoren) and fixed in a stereotaxic apparatus according to Paxinos and Watson³⁶. For intraventricular application, 18 rats received a stereotaxic microinjection of 0.8 nmol of the Aβ(1 - 42) globulomer into the right ventricle using the following coordinates (in mm to Bregma): caudal 1.0, lateral 1.5 and ventral 3.9. A total volume of 4.3 µL was infused by a 10 µL Hamilton syringe attached to a motorized micropump (WPI, Germany) during 5 min with the cannula being left in place for additional 2 min. For intracortical injection, 8 rats received stereotaxic microinjections of Aβ(1 - 42) globulomer (0.15 nmol in 1 µL) using the following coordinates (in mm to Bregma): caudal 2.0, lateral 2.0 and ventral 2.0. The infusion was applied via a 10 µL Hamilton syringe during 5 min with the cannula being left in place for additional 5 min.

Animals were allowed to recover and subsequently sacrificed 1, 4 and 7 days later to study the penetration of the Aβ(1 - 42) globulomer from the ventricle / injection sites into the surrounding tissue by immunohistochemistry and biochemical dot blot analysis. Therefore, rats were deeply anesthetized with Narcoren. In order to analyze if Aβ(1 - 42) globulmers are cleared from cerebrospinal fluid (CSF), in some of the rats several µL of CSF was withdrawn after exposing the atlanto-occipital membrane and punctuating the cisterna magna with a 1 ml syringe and a 0.3 mm cannula. Then, the rats were transcardially perfused with 10 mM PBS (pH 7.2) for 5 min, followed by 4% paraformaldehyde (PFA) in PBS. The brains were removed, postfixed overnight in 4% PFA in PBS containing 30% sucrose and transferred into 30% sucrose in PBS for cryoprotection.

For staining of Aβ(1 - 42) globulomer 40 µm sections were cut on a freezing microtome and incubated free-floating in 5% donkey serum (Serotec) in TBST for 20 min. Then, the sections were transferred into the monoclonal primary antibody 8F5, which is specific for Aβ(1 - 42) globulomers, at a dilution of 1:1000 in TBST for 24 h. A Cy3-labeled donkey anti-mouse antibody (Jackson Laboratories) was used for fluorescence labelling for 1 h. Between the steps and at the end, sections were thoroughly washed in TBST three times for 5 min, after which they were mounted onto glass slides, air-dried and cover-slipped with Vectashield hardest mounting medium containing DAPI (Vector Laboratories) to visualize cell nuclei. All incubations were performed at room temperature. Some sections were also stained with 0.05% thioflavine S (Sigma, Germany) in 50% ethanol for 10 min, followed by two rinses of 100% ethanol for 5 min before embedding in Vectashield. Analysis of labelling was performed under a confocal fluorescence microscope (Zeiss LSM 510 Meta, Germany).

Injections into the third ventricle led to rapid and persistent binding of the Aβ globulomers to cells of the ventricle walls (see Fig. 16(II)a) while they completely disappeared from the cerebrospinal fluid (CSF; data not shown). The injected Aβ(1 - 42) globulomers were not masked by other proteins in CSF since application of different concentrations into rat CSF in vitro led to a dose-dependent recovery. The bound Aβ globulomers stayed in the ependyme without detectable diffusion into underlying brain tissue and without obvious cell toxicity since cell density along the ventricle wall appeared unchanged. Poor tissue penetration was also shown by intracerebral injections in which Aβ globulomers staining was restricted close to the injection site and did not decrease for several days (see Fig. 16(II)b-c). Biochemical analysis confirmed that the vast majority of injected Aβ(1 - 42) globulomer is still present in the hippocampus for at least 7 days (data not shown). In contrast, injected Aβ(1 - 42) monomers stained a larger area and labelling decreased after 7 days (see Fig. 16(II)c) suggesting better tissue penetration than Aβ(1 - 42) globulomer.

### Example 12: Specific binding of Aβ(1 - 42) globulomers to hippocampal neurons

The inventors tested the physiological relevance of Aβ(1 - 42) globulomers by studying their interaction with primary hippocampal neurons.

Hippocampal cells from rat brain (QBM Cell Science, Canada) were cultured in 24-well plates on poly-L-lysine coated coverslips (BD Biosciences, Germany) in Neurobasal medium with B27 supplement (Invitrogen, Germany) at 37 °C, 5% CO₂. To some cultures mitosis inhibitors (35 µg/ ml uridine, 15 µg/ ml 5-fluoro-2'-deoxyuridine) were added at DIV6 to prevent an overgrowing with glia. Unless otherwise stated hippocampal cells were used at DIV13-14. When Aβ-forms were added, 750 µL fresh 37 °C warm medium containing the Aβ-forms were exchanged of the total 1 ml. Incubation took place for 15 min at 37 °C, 5% CO₂. Aβ-monomer (0.5 mg/ ml) stock solution was in HFIP and Aβ(1 - 42) globulomer in 35 mM NaCl, 5 mM NaH₂PO₄, pH 7.4.

Cells were rinsed three times with 1 ml medium and fixed with 3.7% buffered formaldehdye (Accustain, Sigma, Germany). Unspecific binding sites were blocked by incubation with 10% normal goat serum in PBS (pH 7.4) for 90 min at room temperature to which 0.1% Triton X-100 was added in the case of intracellular counterstaining. Primary antibodies (anti Aβ 6E10 (Signet Laboratories, USA), 1:2000; anti GFAP (Dako Cytomation, Germany), 1:2500; anti MAP2 (Chemicon International, Germany), 1:1000) and secondary antibodies (Alexa 488, 1:500; Alexa 635, 1:500; Molecular Probes, Germany) were subsequently incubated for 2 h at room temperature in 10% normal goat serum in PBS (pH 7.4). After each antibody incubation cells were washed three times with PBS and finally mounted in ProLong antifade reagent (Molecular Probes, Germany).

The Aβ(1 - 42) globulomers exhibited a strong binding to hippocampal neurons at a concentration of 17 nM (equivalent to 200 nM monomer concentration) in contrast to controls without Aβ(1 - 42) or with 200 nM monomeric Aβ(1 - 42) (see Fig. 15a). Monomeric structure of Aβ(1 - 42) was ensured by HFIP stock solution. A possibly negative effect of the HFIP on the ability of hippocampal neurons to bind Aβ(1 - 42) was ruled out by incubation of an equivalent volume HFIP together with Aβ(1 - 42) globulomers without difference to Aβ(1 - 42) globulomers alone. Hippocampal neurons bound Aβ(1 - 42) globulomer as low as 2 nM (data not shown). The binding of Aβ(1 - 42) globulomer to neurites was highly punctuated, suggesting a specific binding to synaptic terminals or dendritic spines (see Fig. 15b, inset). A strong dependence of Aβ(1 - 42) globulomer binding on the age of cultured neurons was observed. At DIV8 almost no hippocampal binding (∼1 %) was found, whereas at DIV11 and DIV15 Aβ(1 - 42) globulomer binding was pronounced (∼90%) (see Fig. 15b). The high specificity of Aβ(1 - 42) globulomer binding was further substantiated by confined binding to neurons while glia in this hippocampal preparation did not bind Aβ(1 - 42) globulomer (see Fig. 15c). Furthermore, a variety of neuronal cell-lines were negative for Aβ(1 - 42) globulomer binding (data not shown).

### Example 13: Complete inhibition of long-term potentiation by Aβ(1 - 42) globulomers

In order to determine the effect of Aβ(1 - 42) globulomers on synaptic function the inventors recorded extracellular potentials from the CA1 region of brain slices perfused with or without Aβ(1 - 42) globulomers.

Slices were prepared from male Sprague-Dawley rats (7-8 weeks old). Isolated hemispheres were trimmed and then cut using a vibratome. Slices (400 µM) were directly transferred to an interface recording chamber and continuously perfused at 33°C with oxygenated artificial CSF (aCSF: 118 mM NaCl, 1.24 mM KH₂PO₄, 10 mM glucose, 2 mM MgSO₄, 2.5 mM CaCl₂, 5 mM KCI, 25.6 mM NaHCO₃) at a flow rate of 1.8 ml/min. Freshly prepared slices were allowed to equilibrate for at least one hour before recording. Field potentials were obtained from stratum radiatum of CA1 using glass microelectrodes (0.8-1.2 MΩ) filled with aCSF. The Schaffer collateral was stimulated by a bipolar tungsten electrode (0.5 MΩ, Science Products GmbH, Germany). Constant voltage biphasic pulses (0.1 ms/phase; range 1-5 V) were applied once per minute at an intensity yielding 30% of the fEPSP maximum. LTP was induced by 2 trains of 100 Hz (1 sec/train with 10 sec intertrain intervall) using the same intensity and pulse length. In the Aβ-group, 42 nM Aβ(1 - 42) globulomer was perfused during the entire experiment, starting 80-120 min before tetanization. The input/output relation was determined before baseline recording (64-94 min after Aβ(1 - 42) globulomer wash-in). Signals were digitized by a Power CED 1401 and analyzed using the software Signal 2.14 (Cambridge Electronic Design Ltd., Cambridge). For statistical comparison data were analyzed by 2-way repeated measures ANOVA using SAS.

Field EPSPs obtained from the CA1 dendritic layer of Aβ(1 - 42) globulomer-treated slices appeared normal in size and shape (see Fig. 17b), indicating that excitatory synaptic transmission was unaffected. The field EPSPs did also not reveal lack of synaptic inhibition (i.e. multiple population spikes), suggesting that GABA-ergic transmission was intact. Also, the comparison of the averaged input/output relation of Aβ(1 - 42) globulomer-treated versus untreated slices (see Fig. 17c) indicated that basic synaptic functioning was not impaired by Aβ(1 - 42) globulomers. However, when examining high frequency stimulation-induced LTP, Aβ(1 - 42) globulomer-treated (42 nM) slices revealed a complete block (see Fig. 17a). The potentiation decayed to baseline level within 30 min after tetanic stimulation. Although short-term potentiation could be readily induced in the Aβ(1 - 42) globulomer group, the potentiation was significantly impaired as early as 5 min after tetanization (p<0.05). In contrast, control slices revealed stable LTP for at least one hour, with at least 30% of potentiation throughout the entire recording time. In some experiments the Aβ(1 - 42) globulomer concentration at the outflow of the recording chamber was determined, and 50-70% of the Aβ(1 - 42) globulomers could be detected, indicating that a substantial amount (albeit not all) of the Aβ(1 - 42) globulomers had reached the slices during the experiments. Thus, less than 42 nM Aβ(1 - 42) globulomers were sufficient to specifically inhibit LTP, while leaving basic synaptic transmission unaffected.

### Example 14: Antibodies to Aβ(1 - 42)

The anti Aβ(20-42) globulomer polyclonal antibody 5598 was generated from immunized rabbits and affinity purified against immobilized Aβ(1 - 42) globulomer. The methods used are well documented in the art. Briefly, Aβ(1 - 42) synthetic peptide (Cat. No. H-1368, Bachem, Switzerland) was suspended in 111,333-hexafluoro-2-propanol (HFIP) at - 6 mg/ ml and incubated for complete solubilization under shaking at 37 °C for 1.5 h. HFIP was removed by evaporation in a SpeedVac and Aβ(1 - 42) resuspended at a concentration of 5 mM in dimethyl sulfoxide (DMSO) and 20 sec sonicated. The HFIP pretreated Aβ(1 - 42) was diluted in phosphate buffered solution (PBS) (20 mM NaH₂PO₄, 140 mM NaCl, pH 7.4) to 400 µM and 1/10 volume 2% SDS (in H₂O) added (final concentration of 0.2% sodium dodecyl sulphate (SDS)). An incubation for 6 h at 37 °C resulted in the 16/20 kDa Aβ(1 - 42) globulomer intermediate. The 38/48 kDa Aβ(1 - 42) globulomer was generated by a further dilution with three volumes H₂O and incubation for 18 h at 37 °C. After centrifugation at 3,000 ×g for 20 min the sample was concentrated by ultrafiltration (30 kDa cut-off), dialyzed against 5 mM NaH₂PO₄, 35 mM NaCl, pH 7.4, centrifuged at 10,000 xg for 10 min and the supernatant containing the 38/48 kDa Aβ(1 - 42) globulomer withdrawn. For fatty acid induced 38/48 kDa Aβ(1 - 42) globulomer the SDS was replaced by the addition of 1/10 volume of 0.5% fatty acid. For cross-linking the 38/48 kDa Aβ(1 - 42) globulomer was treated before concentration and dialysis with 1 mM glutardialdehyde for 2 h at RT followed by ethanolamine (5 mM) treatment for 30 min at room temperature (RT). Limited proteolysis of the 38/48 kDa Aβ(1 - 42) globulomer was performed using 1/50 (mg/mg) of the respective protease and incubation for 20 h at RT.

Immunization of mice resulted in high titers (1:10,000-100,000) yielding potent Aβ(1 - 42) globulomer-specific (8F5) and non-specific monoclonal antibodies (6G1). In contrast to the non-specific 6G1 and the reference antibody 6E10, the specific antibody 8F5 detected Aβ(1 - 42) globulomers only by native PAGE-Western blot but not by SDS-PAGE Western blot analysis (data not shown) indicating binding to a more complex detergent-dissociable inter-subunit epitope in the core Aβ(1 - 42) globulomer structure (see Fig. 12a). Dot blot analysis against various Aβ(1 - 42) and Aβ(1 - 40) standard preparations showed significant differences in recognition of Aβ(1 - 42) globulomer versus non-globulomer Aβ-forms (standard Aβ(1 - 40/42) monomer preparation, aggregated Aβ(1 - 42)) for specific 8F5 and 5598 but not for isoform non-specific antibodies 6G1 and 6E10 (see Fig. 12b). The globulomer specificity of 8F5 and 5598 but not of 6G1 and 6E10 was confirmed by quantifying Aβ(1 - 42) globulomer, Aβ(1 - 42) monomer, Aβ(1 - 40) and sAPPα binding in sandwich ELISAs (see Fig. 12c). However, it is assumed that the selectivity of antibodies was underestimated due to minor cross contaminations of our synthetic standard preparations with Aβ(1 - 42) globulomer epitopes since our specific antibodies did not detect Aβ(1 - 42) globulomer epitopes at a level of as low as <0.5 % compared to Aβ monomer in CSF samples (see data in Fig. 13). The degree of cross contaminations in synthetic Aβ preparations was calculated from ELISA value ratios (see Fig. 12d). In the Aβ(1 - 42) NH₄OH preparation and Aβ oligomers prepared according to Lambert et al. (Lambert, M.P. et al. Diffusible, non-fibrillar ligands derived from Abeta1 - 42 are potent central nervous system neurotoxins. Proc. Natl. Acad. Sci U. S. A 95, 6448 - 6453 (1998)) ∼5% of Aβ(1 - 42) globulomer epitopes and 95% Aβ(1 - 42) monomer epitopes were measured, whereas the Aβ(1 - 42) globulomer preparation of the invention was of 95% purity with only 5% cross contamination signal for monomeric Aβ(1 - 42). In conditioned medium (CM) from CHO cells expressing APP with V717F mutation (according to Walsh et al. (Walsh, D.M. et al. Naturally secreted oligomers of amyloid beta protein potently inhibit hippocampal long-term potentiation in vivo. Nature 416, 535 - 539 (2002)) the Aβ(1 - 42) globulomer- epitope level was below detection limit.

### Example 15: Endogenous amyloid β(1- 38), (1-40) and (1-42) levels in AD brain tissue extracts after immunoprecipitation with globulomer selective anti-Aβ mAb 8F5

154 mg human AD brain (sample from Brain-net, Munich) were suspended at 0 °C with 1.39 ml extraction buffer. The sample was homogenized in a glass potter by 20 strikes with the pistill. The homogenized tissue was centrifuged at 100'000 g for 1 hour. The supernatant was taken as the human AD brain extract.

### Material

Basic buffer:
   50 mM Tris, 150 mM NaCl, 2 mM EDTA, 0.01 % Tergitol NP-40, 0.1% SDS pH 7.6 (adjusted with 10 M HCl)
extraktion buffer =
   100 ml buffer
   + 1 tablet Complete protease inhibitor cocktail (Fa.Roche Nr.: 1697498) solved in 1 ml H₂O
   + 200 µl 250 mM PMSF (solved in Methanol),
   prepared directly before use.

### Immobilization of anti-Aβ mAbs to CNBr-activated Sepharose 4B:

### mAb 8F5:

0.4 g CNBr-activated Sepharose 4B (Amersham Inc No.: 17-0430-01) were added to10 ml aqueous 1 mM HCl and incubated for 30 min at room temperature. The CNBr-activated Sepharose 4B was washed three times with10 ml 1 mM HCl und finally twice with 10 ml 100 mM NaHCO₃; 500 mM NaCl; pH 8.3. For each of the immobilized antibodies 100µl CNBr-activated Sepharose 4B Matrix were added to 950 µl 0.5 mg/ ml anti-Aβ mAb 8F5 solution in 100 mM NaHCO₃; 500 mM NaCl; pH 8.3. After 2 h shaking at room temperature samples were centrifuged for 5 min at 10'000 g. Then 500 µl 100 mM ethanolamine ; 100 mM NaHCO₃; 500 mM NaCl; pH 8.3, buffer was added to the beads and samples were shaken for 1 h at room temperature.

The anti-Aβ mAb 8F5-Sepharose sample was centrifuged for 5 min at 10'000 g and washed 5 times with 500 µl 20 mM NaH₂PO₄; 140 mM NaCl; pH 7.4. Before freezing for storage samples were stabilized by adding sodium azide to 0.02% final concentration.

### Immunoprecipitation:

### mAb 8F5-Sepharose:

150 µl of the human AD brain extract were diluted with 150 µl 20 mM NaH₂PO₄ 140 mM NaCl; 0.05% Tween 20; pH 7.4. These samples were added to 2 µl anti-Aβ mAb 8F5-Sepharose Matrix and stirred for 1.5 h at room temperature. The samples were centrifuged for 5 min at 10'000 g. The supernatants were discarded and the anti-Aβ mAb 8F5-Sepharose was washed twice with 50 µl PBS, stirred for 1 min and centrifuged (5 min at 10'000 g). The supernatants were discarded and the Sepharose beads were suspended in 50 µl 2 mM NaH₂PO₄; 14 mM NaCl; pH 7.4; followed by 1 min stirring at room temperature and 5 min centrifugation at 10'000 g. In a next step the anti-Aβ mAb-Sepharose beads were treated with 10 µl 50% CH₃CN; 0.2% TFA in Water. After 10 min shaking at room temperature samples were centrifuged 5 min at 10'000 g. The supernatants were collected.

Determination of Aβ(1-xx) with Seldi-MS:

2 µl of the anti-Aβ mAb 8F5-Sepharose IP-eluate were dotted on a spot of a H4 Protein Chip Array configuration A-H Fa.Ciphergen part number C553-0028 . The chip was dried on air. After that 2 µl of 3.33 mg/ ml CHCA in 50% CH₃CN ; 0.5% TFA in water were dotted on the spot. The chip was dried again by air and analyzed in a SELDI mass spec Ciphergen, Inc
conditions :
intensity : 200
sensitivity : 6
mass range : 800-10'000 Da
calibration : All-in-1 Peptide Standard Mix of 7 Fa.Ciphergen part number C100-0005.

## Claims

1. A non-diffusible globular Aβ(X - 38 .. 43) oligomer or a derivative thereof, wherein X is selected from the group consisting of the numbers 1 .. 24.

2. The oligomer or derivative of claim 1, wherein the oligomer is a non-diffusible globular Aβ(X - 42) oligomer and X is as defined in claim 1.

3. The oligomer or derivative of claim 1 or 2, wherein X is 12 or 20.

4. The oligomer or derivative of any one of claims 1 to 3, wherein the oligomer is obtainable by a process which comprises exposing monomeric Aβ(X - 38 .. 43) peptide to a detergent.

5. The oligomer or derivative of any one of claims 1 to 4, which is soluble.

6. The oligomer or derivative of any one of claims 1 to 5, which is non-fibillar.

7. The oligomer or derivative of any one of claims 1 to 6, which is 99 %, 99,9 %, or 99,99 % pure.

8. Vaccine comprising non-diffusible globular Aβ(X - 38 .. 43) oligomer or a derivative thereof as defined in any one of claims 1 to 7.

9. Vaccine of claim 8, which is capable of inducing an antibody-mediated, non-inflammatory response against the oligomer or derivative.

10. Antibody having specificity for non-diffusible globular Aβ(X - 38 .. 43) oligomer or a derivative thereof as defined in any one of claims 1 to 7.

11. The antibody of claim 10, wherein the antibody binds to an epitope located between the amino acid positions of from about 20 to about 30 of the oligomer or derivative.

12. The non-diffusible globular Aβ(X - 38 .. 43) oligomer or a derivative thereof as defined in any one of claims 1 to 7 for use in treating or preventing an amyloidosis.

13. The non-diffusible globular Aβ(X - 38 .. 43) oligomer of claim 12, wherein the amyloidosis is Alzheimer's disease.

14. The antibody as defined in claim 10 or 11 for use in treating or preventing an amyloidosis.

15. The antibody of claim 14, wherein the amyloidosis is Alzheimer's disease.
